# EUROPEAN PATENT APPLICATION

(11) **EP 0 805 205 A1**
(43) Date of publication of application: **05.11.1997**
(21) Application number: 96201224.1
(22) Date of filing: 02.05.1996
(51) Int. Cl.: C12N 15/52, C12N 15/74, C02F 3/34, A23L 1/015, A23L 2/84

(54) **Nitrate reduction system of Staphylococcus carnosus**

(71) Applicant: SOCIETE DES PRODUITS NESTLE S.A., 1800 Vevey (CH)
(72) Inventor: Fast, Beate, 72076 Tuebingen (DE); Gaier, Walter, 1801 Le Mont Pelerin (CH); Götz, Friedrich, 72076 Tuebingen (DE); Lindgren, Per-Eric, 75322 Uppsala (SE); Neubauer, Heike, 72072 Tuebingen (DE); Pantel, Iris, 72810 Gomaringen (DE)

(57) **Abstract**

The invention comprises (1) a recombinant protein involved in nitrate and nitrite reduction in *Staphylococcus carnosus* (DSM 10563), selected from the nitrate reductase NarGHJ having three subunits, the nitrate reductase biogenesis protein NarI, the nitrate transport protein NarT, the nitrate reductase molybdenum cofactor biosynthesis proteins MoeB, MoaB, MoaC, MoeA, MoaD, MobA, MoaA, MobB, MoaE, the nitrite reductase protein NirBD having two subunits consisting, the nitrite reductase sirohem cofacteur biosynthesis protein CysG, and functional derivatives of these proteins; (2) genes encoding these recombinant proteins; (3) the use of recombinant protein or recombinant cells according to the invention to reduce nitrate and nitrite in a nitrate polluted material.

## Description

The present invention relates to proteins and genes involved in the reduction of nitrate and nitrite, the transformed organisms comprising these genes and proteins, and a method for reducing nitrate in water, vegetables and other nitrate polluted materials.

### Background of the invention

Nitrate in food originates from two main sources. The raw materials naturally contain nitrate, e.g. vegetables may contain 1000 ppm nitrate or more (values greater than 10'000 ppm nitrate in lettuce have been reported). In fact, vegetables are responsible for about 80% of a human's daily intake of nitrate. The principal source of nitrate in vegetables and drinking water is through the excessive use of nitrate containing fertilisers. The other source is nitrate which is used as a food additive, e.g. it can be added to hard cheeses in order to avoid late blowing which is caused by the germination of thermophilic bacterial endospores. Nitrite is added to some meat products which undergoes disproportion to nitrate and nitric oxide. Nitric oxide leads to the desired red colour, microbiological safety (e.g. inhibition of spore germination) and the specific curing aroma. Nitrate is reduced to nitrite by suitable starter cultures.

Nitrate itself has little primary toxicity. However, severe health problems can arise from the transformation to nitrite in the human body during digestion. Therefore research on the reduction of nitrate (and nitrite) in vegetables is pursued (Corré and Breimer, PUDOG Literature Survey No. 39, Wageningen, Center for Agriculture Publishing documentation).

Many denitrifying organisms have been described, but few are food grade organisms. In carrots, complete nitrate reduction with aid of *L. plantarum* has been reported. The natural Gram-negative flora of the carrots first reduces the nitrate and then *L. plantarum* reduces the nitrite to nitrous oxide (Andersson, R., Int. J. Microbiol., 2, 219-225, 1985).

*Staphylococcus carnosus* is of great importance as a component of starter cultures used in meat product manufacturing. This organism efficiently reduces nitrate to nitrite, which is then available for the development of the typical red color of cured meat and meat products.

Recently immobilized cells of *S. carnosus* were tested for the reduction of nitrate in carrot juice (J. Reiß, Deutsche Lebensmittel-Rundschau 11, p. 352-353, 1992). However, the rates of nitrate reduction were relatively low.

### Summary of the invention

The present invention provides new proteins and genes involved in the reduction of nitrate and nitrite allowing the application of particular microorganisms more adapted to nitrate and nitrite reduction in water, vegetables and other nitrate and/or nitrite polluted materials.

Accordingly, the invention provides a recombinant protein involved in nitrate and nitrite reduction in *Staphylococcus carnosus*, selected from the nitrate reductase NarGHJ having three subunits consisting of respectively amino acid sequences SEQ ID NO:4, NO:6 and NO:7, the nitrate reductase biogenesis protein NarI consisting of amino acid sequence SEQ ID NO:8, the nitrate transport protein NarT consisting of amino acid sequence SEQ ID NO: 10, the nitrate reductase molybdenum cofactor biosynthesis proteins MoeB, MoaB, MoaC, MoeA, MoaD, MobA, MoaA, MobB, MoaE consisting of respectively amino acid sequences SEQ ID NO:12, NO:13, NO:14, NO:15, NO:16, NO:17, NO:18, NO:19 and NO:20, the nitrite reductase protein NirBD having two subunits consisting of respectively amino acid sequences SEQ ID NO:2 and NO:3, the nitrite reductase sirohem cofacteur biosynthesis protein CysG consisting of amino acid sequence SEQ ID NO:5, and functional derivatives of these proteins.

The present invention relates to a DNA encoding a protein according to the present invention.

The invention also relates to a recombinant cell comprising the DNA molecule according to the invention, the said cell being capable of expressing the nitrate reductase protein NarGHJ, the molybdenum cofactor biosynthesis proteins MoeB, MoaB, MoaC, MoeA, MobB, MoaE, MoaD, MobA, MoaA, the nitrite reductase protein NirBD, Nar I, NarT, CysG, and/or functional derivatives of these proteins.

The invention also provides a method for producing recombinant proteins according to the invention comprising, providing recombinant cells according to the invention in a suitable growth medium under conditions which allow the cells to express the said recombinant protein(s), and optionally isolating the said recombinant protein(s) in the form of a concentrate.

The invention further provides the application of recombinant proteins or cells according to the invention to reduce nitrate in a nitrate polluted material.

In a further aspect of the invention a method is provided for reducing nitrate to a non-toxic compound in a nitrate polluted material, comprising treating the said material with NarGHJ or functional derivatives, treating said material with NirBD, and recovering the nitrate and nitrite free material.

### Short description of the drawings

Figure 1: Figure 1A presents a restriction map of the transposon Tn*917* (approximately 5.2 kb), and figure 1B presents a restriction map of the 6.0-kb *Eco*RI-*Sal*I fragment of *S. carnosus* Tn*917* insertion mutant nrI.

Figure 2: Map of the organization of the *S. carnosus* genes necessary for nitrate and nitrite reduction. The Tn*917* insertion sites of the nitrate reductase negative mutant nrI and of the nitrite reductase negative mutant nir1 are marked.

Figure 3: Map of the *S. carnosus* locus containing genes most likely involved in molybdenum cofactor biosynthesis. The Tn*917* insertion in mutant nrIV which was identified in *moeB* results in the complete loss of nitrate reductase activity.

Figure 4: Effect of oxygen on nitrite reduction in resting cells of *S. carnosus*. At zero time. nitrite was added, and the reaction mixture was incubated in the presence (open circles) or absence (closed circles) of oxygen.

Figure 5: Effect of nitrate on nitrite reduction in resting cells of *S. carnosus*. Nitrite was added at zero time, and the production of nitrite (open circles) and ammonia (closed circles) during anaerobic incubation at 37°C was monitored.

After 26 min., nitrate (400 µM) was added as indicated by the arrow and incubation was continued.

Figure 6: Time-dependent formation of nitrite by *S. carnosus* and *L. plantarum* in a nitrate containing material.

### Detailed description of the invention

For purpose of this disclosure and claims, the terms 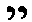 *narGHJ* 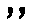 *,* 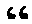 *narI* 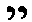 *,* 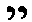 *moeB* 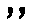 *,* 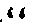 *moaB* 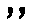 *,* 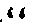 *moaC* 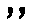 *,* 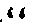 *moeA* 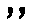 *,* 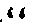 *mobB* 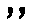 *,* 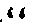 *moaE* 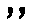 *,* 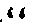 *moaD* 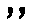 *,* 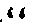 *mobA* 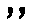 *,* 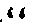 *moaA* 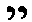 *,* 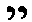 *narT* 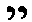 *,* 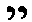 *nirBD* 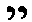 *and* 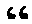 *cysG* 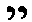 refer to the genes encoding the respectively related proteins NarGHJ, NarI, MoeB, MoaB, MoaC, MoeA, MobB, MoaE, MoaD, MobA, MoaA, NarT, NirB, NirD and CysG.

Within the context of the present invention the expression 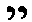 functional derivative" may comprise all DNA or amino acid sequences which differ by substitution, deletion, addition of some nucleotides or amino acids but which keep their original activities or functions. In this field, two DNA sequences may be considered as derivative if they encode the same or a derivative protein due to the degeneracy of the genetic code or to the cross species variation.

A DNA molecule may be generally considered as a derivative to another DNA molecule or protein, if its sequence is at least 20% identical to the another DNA molecule, preferably at least 40%, in particular 60%. In the present disclosure, the identity is determined by the ratio between the number of bases of a derivative sequence which are identical to those of *narGHJ, nirBD, cysG, moeB, moaB, moaC, moeA, mobB, moaE, moaD, mobA, moaA or narT*, and the total number of bases of the said dÈrivative sequence.

A protein may be generally considered as a derivative to another protein, if its sequence is at least 70% identical to the protein, preferably at least 80%, in particular 95%. In the present disclosure, the identity is determined by the ratio between the number of amino acids of a derivative sequence which are identical to those of NarGHJI, MoeB, MoaB, MoaC, MoeA, MobB, MoaE, MoaD, MobA, MoaA, NirBD, CysG or NarT, and the total number of or amino acids of the said derivative sequence.

The DNA molecule according to the present invention may be obtained in substantially purified form by using the method described within the following examples from any strain of *S. carnosus*, more particularly from the strain *S. carnosus* TM300 (Scheifer *et al*., Int. J. Syst. Bacteriol. 32, 153-156, 1982; Shaw *et al*., J. Bacteriol, 164, 782-796 1985) which was deposited at the Deutsche Sammlung Von Mikroorganismen und Zellkulturen, Mascheroder Weg 1b, 38124 Braunschweig, Germany, on March 8, 1996, where it received the deposit number DSM 10563.

Alternatively, the DNA molecule may be recovered also from other genera or species of bacteria by use of DNA probes derived from a DNA sequence of the invention in a stringent hybridization assay.

In a further aspect, the DNA molecule may also be synthesized from sequences given in the sequence listing below, multiplied *in vitro* for instance by using the polymerase chain reaction or multiplied *in vivo* for instance in bacteria of the species *Escherichia coli, Lactococcus lactis*, *Streptococcus thermophilus* or *Staphylococcus carnosus*.

The DNA molecule according to the invention comprises at least an encoding part of at least 20 base pairs (bp) of a DNA sequence selected from the group comprising the DNA sequence SEQ ID NO: 1 in particular from nucleotide 181-2583 (*nirB*), 2589-2900 (*nirD*), 2894-3844 (*CysG*), 4140-7811(*narG*), 7804-9378 (*narH*), 9374-9946 (*narJ*) and 9942-10622 (*narI*), the DNA sequence SEQ ID NO:9 in particular from nucleotide 538-1701 (*narT*), the DNA sequence SEQ ID NO: 11 in particular from nucleotide 117-1115 (*moeB*), 1150-1659 (*moaB*), 2396-3652 (*moeA*), 3652-4131 (*mobB*), 4131-4580 (*moaE*), 4583-4813 (*moaD*), 4821-5408 (*mobA*), 5470-6489 (*moaA*) and complementary nucleotide 2331-1849 (*moaC*).

The DNA molecule according to the invention may also comprise a sequence which is a derivative (see definition) to the above sequences.

The DNA molecule according to the invention can be present in a vector, such as a replicative plasmid or an integrative circular or linearized non replicative plasmid, for example.

The DNA molecule can also comprise, operably linked to the said DNA, regulatory sequences native to the organism from which derives the nucleotide sequence encoding NarGHJ, NarI, NirBD, CysG, MoeB, MoaB, MoaC, MoeA, MobB, MoaE, MoaD, MobA, MoaA and NarT or functional. The said native regulatory sequences can be a promoter, a terminator, a Shine-Dalgarno sequence, an enhancer, or a sequence encoding a leader peptide, for example, that regulate expression of *narGHJ, nirBD, cysG, moeB, moaB, moaC, moeA, mobB, moaE, moaD, mobA, moaA* and *narT* or similar genes.

In another embodiment, regulatory sequences can be native sequences that regulate a different gene in the said organism of origin or that regulate a different gene in a foreign organism, for example. A regulatory sequence other than the native regulatory sequence will generally be selected for its high efficiency. It is also possible to select as regulatory sequence a promoter on the basis of other desirable characteristics, for example thermo inducibility, or a sequence encoding a peptide signal which will permit excretion of the protein.

If heterologous expression is preferred, meaning that the genes of the invention are expressed in another organism than the original host (strain, variety, species, genus, family, order, class or division) the regulatory sequence is preferably derived from an organism similar or equal to the expression host. For example, if the DNA molecule of the invention is derived from *S. carnosus* and the expression host is a yeast cell, then the regulatory sequence will be derived from a yeast cell. The promoter suitable for constitutive expression, preferably in a fungal host, may be a promoter from the following genes: glycerolaldhehyde-3-phosphate dehydrogenase, phospho-glycerate kinase, triose phosphate isomerase and acetamidase, for example. Promoter suitable for inducible expression, preferably in a fungal host, may be a promoter from the following genes: endoxylanase IIA, glucoamylase A, cellobiosehydrolase, amylase, invertase, alcohol dehydrogenase and amyloglucosidase. The selection of a desirable regulatory sequence operably linked to a sequence of the invention and capable of directing the expression of the said nucleotide sequence is considered to be obvious to one skilled in the art.

The DNA molecule according to the invention may also comprise a selection marker to discriminate host cells into which the recombinant DNA material has been introduced from cells that do not comprise the said recombinant material. It may also comprise at least one suitable replication origin. Suitable transformation methods and suitable expression vectors provided with a suitable transcription promoter, suitable transcription termination signals and suitable marker genes for selecting transformed cells are already known in the literature for many organisms including different bacteria, fungal and plant species.

Overexpression of proteins of the invention may be achieved by incorporation of the DNA molecule of the present invention in an expression host, the said DNA molecule comprising one or more regulatory sequences which serve to increase expression levels of the protein(s) of the invention. The overexpression can be further achieved by introducing a multicopy of the DNA molecule of the invention, for example.

The invention encompasses also a recombinant cell comprising the DNA molecule of the invention, the said cell being able to expressing at least one of the genes encoding NarGHJ, NirBD, CysG, MoeB, MoaB, MoaC, MoeA, MobB, MoaE, MoaD, MobA, MoaA, NarI or NarT or functional derivatives. These cells may be derived from the group of fungal cells in particular of the genus *Aspergillus*, yeast cells in particular of the genera *Saccharomyces, Kluyveromyces, Hansenula* and *Pichia*, bacterial cells in particular Gram-positive bacteria of the genera *Bacillus*, *Lactobacillus*, *Streptococcus and Staphylococcus*, and plant cells, in particular of the vegetable group, for example.

Recombinant cells may comprise the DNA molecule of the invention stably integrated into the chromosome or on a replicative plasmid. Preferably, the DNA molecule of the invention is integrated into the chromosome by using the process described in EP564966, i.e.,
(1) transforming a host strain microorganism with a donor plasmid which does not replicate in the host strain, wherein the donor plasmid comprises a vector backbone and a sequence comprising a promoterless foreign gene *(narGHJ, nirBD, cysG, moeB, moaB, moaC, moeA, mobB, moaE, moaD, mobA, moaA*, *narI* and *narT* or similar genes) operably integrated into at least a part of an operon of the host strain, maintaining the frame and the function of the genomic operon of the host strain;
(2) identifying cointegrate transformants in which the complete donor plasmid is integrated into the genomic operon of the host strain;
and (3) selecting an integrant transformant from the cointegrate transformants, wherein the genome of the selected integrant transformant does not include the vector backbone of the donor plasmid but does include the foreign gene, which is operably integrated into the conserved genomic operon and which is stably maintained and expressed due to selective pressure on the correct functioning of the essential cistron upon growth in a standard medium.

Progeny of an expression host comprising a DNA molecule according to the invention is also included in the present invention. Accordingly, a preferred embodiment of the invention is directed to a cell comprising a recombinant DNA molecule of the invention in any of the embodiments described above, wherein the said cell is able to integrate the nitrate and/or nitrite reductase enzymes or functional derivatives into the cell wall or the cell membrane or secrete the enzymes into the periplasmic space or the culture medium. The secreting route to be followed by recombinant proteins according to the invention will depend on the selected host cell and the composition of the recombinant DNA according to the invention. Most preferably, however, the protein will be secreted into the culture medium. To this end, the cell according to the invention can comprise recombinant DNA further comprising operably linked DNA encoding foreign leader sequences (pre or prepro), for example.

The invention is also directed to a process for producing recombinant protein(s) according to the invention comprising, providing recombinant cells according to the invention in a suitable growth medium under conditions that the cells express the said recombinant protein(s), and optionally isolating the said recombinant protein(s) in the form of a concentrate. The selection of the appropriate medium may be based on the choice of expression host and/or based on the regulatory requirements of the DNA recombinant material. Such media are well-known to those skilled in the art.

After fermentation, the cells can be removed from the fermentation broth by centrifugation or filtration. Depending on whether the host cells have secreted the recombinant protein(s) of the invention into the medium or whether the said protein(s) are still connected to the host cells in some way either in the cytoplasm, in the periplasmic space or attached to or in the membrane or cell wall, the cells can undergo further treatment to obtain the recombinant protein(s). In the latter case, where the recombinant protein(s) are still connected to the cells, recovery may be accomplished by rupturing the cells for example by high pressure, sonication, enzymatic digestion or simply by cell autolysis followed by subsequent isolation of the desired product. The protein(s) can be separated from the cell mass by various methods, such as ultrafiltration, and then subsequently precipitated with an organic solvent. The isolated protein(s) may be further purified by conventional methods such as precipitation and/or chromatography.

The invention is also directed to the use of recombinant proteins according to the invention for reduction of nitrate in a nitrate polluted material. To this end, a nitrate polluted source may be treated with the nitrate and/or nitrite reductase enzymes (NarGHJ and NirBD) or functional derivatives, for example. Attention should be paid for the presence of suitable nitrate reductase molybdenum cofactor and/or nitrite reductase sirohem cofactor.

The invention is also directed to the use of recombinant cells according to the invention for reduction of nitrate in a nitrate polluted material. To this end, a nitrate polluted source may be treated with cells expressing the NarGHJ, NarI and NarT proteins, and/or NirBD, or functional derivatives, for example. Attention should also be paid for the presence of a molybdenum cofactor which could be naturally expressed in the host cell, or alternatively biosynthesed by the recombinant *moeB, moaB, moaC, moeA, mobB, moaE, moaD, mobA, moaA* genes. The same remark may be applied for a sirohem cofactor which could be naturally expressed in the host cell, or alternatively biosynthesed by the recombinant *CysG* gene.

In particular, the nitrate reductase activity of recombinant protein(s) of the invention may be used for various purposes, especially in drinking water and in vegetables where the content of nitrate is often above acceptable levels. A critical product group is baby food containing vegetables because levels around 30 mg nitrate per kg body mass have been reported to cause methaemoglobinaemia in babies (Corré and Breimer, 1979). For the reduction of nitrate in vegetables, nitrite as end product is not acceptable since nitrite is more toxic than nitrate, but the accumulation of gaseous products such as nitrogen (N₂) or nitrogenous oxides (e.g. N₂O) is generally accepted as non-toxic in foods.

A preferred embodiment of the invention is thus directed towards a method for reducing nitrate to a non-toxic compound such as N₂O or N₂ in a nitrate polluted material comprising, treating the said material with NarGHJ proteins or functional derivatives according to the invention, and in the meantime or thereafter, treating the said material with denitrifying cells such as cells expressing naturally nitrite-, nitric oxide-, and/or nitrous oxide reductase activities.

Alternatively, the said material can be treated either with the nitrite reductase enzyme derived from *S.carnosus* (NirBD) or functional derivatives or with cells expressing *nir*B and *nir*D or functional derivatives according to the invention. However, endproduct of this process is ammonium. Finally, the nitrate and nitrite free material can be recovered.

In particular, the material may be treated with cells expressing NarGHJ, NarI, MoeB, MoaB, MoaC, MoeA, MobB, MoaE, MoaD, MobA, MoaA, and NarT proteins or functional derivatives according to the invention. Likewise, the material may also be treated with cells expressing NirBD and CysG proteins or functional derivatives according to the invention.

In another hand, the material may also be treated with recombinant denitrifying cells, for example. Preferably, the nitrate polluted material is treated with naturally denitrifying cells synthesizing the recombinant NarGHJ, NarI and NarT proteins or functional derivatives according to the invention in any of the embodiments described above. A food grade denitrifying microorganism, as described below, can thus be transformed with a DNA molecule according to the present invention.

The cells expressing the NarGHJ, NirBD, CysG, MoeB, MoaB, MoaC, MoeA, MobB, MoaE, MoaD, MobA, MoaA, NarI and NarT proteins are understood in the context of the present method to be cells naturally possessing these activities, such as *Staphylococcus carnosus*, but also recombinant cells according to the invention in any of the embodiments described above. The cells able to reduce a nitrite polluted material can be chosen in the group comprising the genus *Achromobacter, Alcaligenes, Bacillus, Chromobacterium, Corynebacterium, Halobacterium, Hyphomycrobium, Micrococcus, Moraxella, Nitrosomonas, Propionibacterium Pseudomonas, Spirillum, Staphylococcus, Thiobacillus, Xanthomonas* and *Lactobacillus*, for example. Food grade microorganisms are however preferred, such as *Lactobacillus sake* such as strain L110 of Lacto-Labo, Texel, France*, Lactobacillus farciminis, Lactobacillus brevis* such as strain DSM20057 (Deutsche Sammlung von Mikroorganismen, DE)*, Lactobacillus plantarum* such as strain DSM2601 or DSM20246*, Lactobacillus pentosus* such as strain DSM20314, *Lactobacillus lactis* such as strain NCDO280 (National Collection of Dairy Organisms, UK), and *Lactobacillus suebicus*, for example.

In particular, cells or enzymes used to transform nitrate to a non-toxic compound are immobilised. The immobilisation of biocatalysts, such as enzymes or cells, is industrially regarded, for a skilled person, as a standard technique. Many methods for immobilisation can be used, such as adsorption on inert supports, entrapment within crosslinked gels, encapsulation within micro-capsules, intermolecular crosslinking of enzymes, and covalent binding to supports via functional groups of the protein which do not take part in the catalytic process, for example. The subject matter of EP676145 relating to methods of enzyme immobilisation is incorporated by reference into the present disclosure.

Immobilised enzymes or cells according to the invention represent certain advantages. They allow a good separation of the biocatalyst and the material by filtration, sedimentation or centrifugation. They allow also reduction of emulsification of the material and an increased stability of the biocatalyst, for example.

The immobilised enzymes or cells according to the present invention can be used in a batch system, in which a nitrate polluted material is denitrated and denitrified in a reactor of the stirred tank type with immobilised enzymes or cells. The system may be automated and operates in a semi-continuous manner with the following three stages. In the first instance, the tank is filled with the nitrate polluted material, denitrated and denitrified with mechanical stirring, after which the tank is emptied. The retention of the enzyme in the reactor may take place by means of a filter disposed in the lower portion of the tank, for example.

The immobilised enzymes or cells according to the present invention can also be packed into a column for use in a continous process. The column can comprise a fluidized bed of immobilised enzymes or cells. The flow of nitrate polluted material, circulating from the bottom to the top, then places the particles in suspension.

The present invention is further illustrated hereafter, and not limited, by a supplemental description which refers examples of characterisation of DNA molecules, cells, proteins and use according to the invention, in which all parts, ratios, and percentages are expressed on a weight basis unless otherwise stated, with reference to the accompanying drawings. These examples are prefaced by the description of culture conditions and of methods to accumulate nitrite, to determine the concentration of nitrite and ammonia, and to analyse the activity of the nitrate and nitrite reductase.

Restriction enzymes, T4 DNA ligase and alkaline phosphatase were purchased from New England Biolabs (Beverly, Mass., USA), Boehringer Biochemicals (Mannheim, FRG), and Pharmacia Biotechnology (Freiburg, FRG) and were used according to the suppliers recommendations. Other methods involving DNA techniques were essentially performed as described in the Book of Sambrook *et al*. (Sambrook *et al*., Molecular Cloning, A Laboratory Manual, Cold Spring Harbor Laboratory Press, U.S.A., 1989).
1. Culture conditions and nitrite accumulation: Cells of *S. carnosus* TM300 are grown at 37°C in modified basic medium (BM) (10g of casein hydrolysate per liter, 5g of yeast extract per liter, 1g of glucose per liter, 13g of K 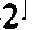 HPO 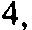 , and 2g of NaCl per liter; pH 7.4; Götz and Schumacher, FEMS Microbiol. Lett., 40, 285-288, 1987). Aerobic cultures are incubated on a rotary shaker at 130 rpm. Anaerobic cultures are incubated in screw-cap bottles with stirring (100 rpm). The medium is supplemented with Oxyrase (20 ml/liter of medium; Oxyrase Inc. Mansfield, Ohio), an enzyme system that creates anoxic conditions in broth media at a pH range between 6.8 and 9.0. Additionally, the medium is overlaid with light mineral oil (Sigma) during anaerobic incubation. For measuring nitrite accumulation in the culture medium of *S. carnosus* wild-type and the Tn*917* insertion mutants, modified BM containing various concentrations of sodium nitrate, is inoculated with an aerobically grown overnight culture. After incubation up to 15h, aerobically or anaerobically, the culture is centrifuged, and the nitrite concentration in the culture supernatant is determined.
2. Nitrite determination: nitrite concentration is determined by use of the colorimetric assay of Nicholas and Nason (Methods Enzymol., 3, 981-984, 1957) which was modified by Showe and DeMoss (J. Bacteriol., 95, 1305-1313, 1968).
3. Nitrate reductase activity: nitrate reductase activity is determined by using a modified method of Lowe and Evans (Biochim. Biophys. Acta, 85, 377-389, 1964). A reaction mixture is used (3ml total vol.), consisting of potassium phosphate buffer (90mM, pH 7.2), benzyl viologen (1mM) as artificial electron donor, sodium nitrate (10mM), and the reducing agent dithionite (4.7mM). The addition of dithionite in excess facilitates the handling under laboratory conditions without an anoxic chamber. The mixture is stirred at 37°C and the reaction is started by the addition of washed cells (washed twice with 90mM K-phosphate buffer, pH 7.2). Cell densities vary between 0.2 and 1.0 (final OD 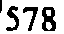 ), depending on the expected nitrate reductase activities of the wild-type and the various mutants. Samples are taken at various time intervals and nitrate reduction is stopped by vigorously vortexing the mixture in order to oxidize all dithionite and benzyl viologen.
4. Ammonia determination: the concentraion of ammonia was determined, using the NADH-dependent conversion of 2-oxoglutarate to L-glutamate catalyzed by glutamate dehydrogenase (Boehrnger Mannheim, Federal Republic of Germany).
5. Characterization of the nitrate reductase of *S. carnosus*
   A) Wild-type: during growth in the presence of nitrate, cells of *S. carnosus* reduce nitrate to nitrite, which subsequently accumulates in the growth medium. The rate of nitrite accumulation is eight- to tenfold higher under anoxic than under oxic conditions. The presence of nitrate under anoxic conditions results in a higher growth yield: the final OD₅₇₈ of 1.1 to 1.2 under anoxic conditions increases up to 2.6 to 2.7 when nitrate (25 mM) is present.
   B) Tn*917* insertion mutants: in order to identify genes involved in nitrate reduction in *S. carnosus* TM300, a transposon mutagenesis in *S. carnosus* wild-type is performed using the temperature-sensitive plasmid pTV1ts containing the transposon Tn*917* (Youngman *et al*., Proc. Natl. Acad. Sci., 80, 2305-2309, 1983; Youngman *et al*., In: Regulation of procaryotic development. Eds: Smith, I., Slepecky, R.A., and Setlow, P., ASM, Washington, 65-69, 1989; Shaw and Clewell, J. Bacteriol., 164, 782-796, 1985). *Tn917* encodes genes necessary for transposition and mediates erythromycin resistance.

To this end*, S. carnosus* is transformed by protoplast transformation method with pTV1ts (Götz et al., FEMS Microbiol. Lett., 40, 285-288, 1987). *S. carnosus* harboring pTV1ts is grown in BM medium containing chloramphenicol (Cm, 20 µg/ml) and erythromycin (Em, 20µg/ml) at 30°C to an OD 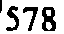 of 1 (reached after 5-7 h growth). The bacterial culture is then diluted 200-fold in BM supplemented with Em (4µg/ml) and incubated 14h at 42°C. After dilution, the cells are spread on BM-agar plates containing 4 µg/ml Em and incubated at 42°C for 2 days. The frequency of Tn*917* insertion mutants is 5x10 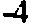 . Approximately 98% of the Em-resistant mutants are also Cm-sensitive, indicating the loss of the plasmid pTV1ts. Altogether, 2800 Tn*917* insertion mutants are isolated.

The obtained Tn*917* insertion mutants were screened for nitrate reduction different from wild-type, measured as nitrite accumulation in the growth medium. Nine mutants, *S. carnosus* nrl-nrlX, affected in nitrite accumulation, were isolated. A characteristic phenotype of most of the mutants is their increased sensitivity to nitrate. This effect is more pronounced during anaerobic than during aerobic growth.

The mutants were further characterized with respect to nitrite accumulation during aerobic and anaerobic growth and nitrate reductase activity determined in resting cells precultivated anaerobically in the presence of nitrate (10mM). The results led to the following grouping of the mutants (Tab. 1):
* Class A, to which Tn*917* insertion mutants nrI, II, IV, and IX belong, shows none or only slight nitrite accumulation and no nitrate reductase activity.
* Class B, to which nrIII, VI, and VII belong, has no detectable accumulation of nitrite under physiological growth conditions (up to 10mM nitrate added), but are similar to wild-type when higher nitrate concentrations are added.
* Class C, in which mutant nrVIII is the sole member, has the same level of nitrate reductase activity as wild-type, but does not accumulate nitrite.

**Table 1**

| Grouping of Tn*917* insertion mutants into classes according to nitrite accumulation and nitrate reductase activity | | |
|---|---|---|
| Class of mutants | Phenotype | Tn*917* insertion mutant |
| A | - No nitrite accumulation | nrI, nrII, nrIV, nrV, nrIX |
| | - No nitrate reductase activity | |
| B | - No nitrite accumulation up to 10 mM nitrate added | nrIII, nrVI, nrVII |
| | - At 100 and 1000mM nitrate added, wild-type levels of nitrite accumulation | |
| | - Wild-type levels of nitrate reductase activity | |
| C | - No nitrite accumulation | nrVIII |
| | - wild-type levels of nitrate reductase activity | |

Southern-blotting: mutants nrI to nrIX are further characterized by Southern blotting to determine whether Tn*917* is inserted at different sites in the mutants nrI - nrIX.

Chromosomal DNA from the mutants is digested with *Eco*RI or *Pst*I. DNA fragments are separated on an agarose gel. The appropriate fragments are cut out, the DNA is eluted using Biotrap BT1000 (Schleicher and Schuell, Dassel, DE, and probed with a 1.9-kb *Bgl*II fragment derived from Tn*917* and labeled using the DIG DNA labeling kit (Boehringer, Mannham, DE). For the transfer of DNA to nylon filters, Hybond-N 0.45 Micron (Amersham, Amersham, Great Britain) is used. Southern blotting is performed using a vacuum-blot apparatus (vacuGene, Pharmacia LKB, Bromma, Sweden) as recommended by the suppliers. DNA hybridization is performed according to the protocol supplied with the DIG-labelling kit.

*Eco*RI and *Pst*I do not cleave within the transposon. Table 2 shows the sizes of the various restriction fragments obtained after hybridization, which indicate that Tn*917* is inserted in the same fragment in nrIII, VI, and VII, although they differ slightly in nitrite accumulation.

**Table 2**

| Characterization of *Staphylococcus carnosus* Tn*917* insertion mutants by Southern blotting. | | |
|---|---|---|
| Tn*917* insertion mutant | Fragment size (kb) when cleaved with | |
| | *Eco*RI | *Pst*I |
| nrI | 9.0 | 8.5 |
| nrII | 8.5 | 15 |
| nrIII | 9.0 | 11 |
| nrIV | 8.5 | 8.0 |
| nrV | 6.0 | 14 |
| nrVI | 9.0 | 11 |
| nrVII | 9.0 | 11 |
| nrVIII | 5.4 | 9.2 |
| nrIX | 7.0 | 6.6 |

### Tn917 insertion mutant nrI

In contrast to *S. carnosus* wild-type, mutant nrI shows neither nitrite accumulation nor nitrate reductase activity. In the case of *S. carnosus* wild-type, nitrate leads to an advantage during anaerobic growth, which was not observed in the case of mutant nrI. These data indicate that the transposon insertion in mutant nrI causes a defect in components essential for the energy gaining reduction of nitrate.

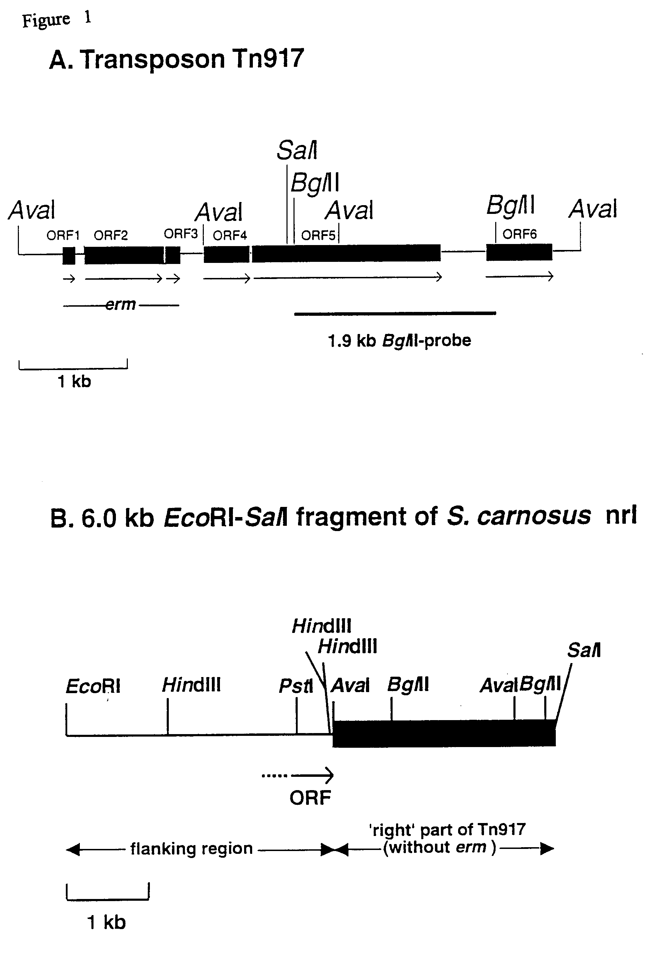

The cleavage of the transposon into two parts using the *Sal*I-site in Tn*917* allows the cloning of the right part of the transposon (Fig. 1A) together with its flanking region (3.3kb). An enriched gene library of mutant nrI was thus made by digesting chromosomal DNA with *Eco*RI and *Sal*I. Fragments around 6kb were isolated and ligated into the multiple cloning site of the shuttle vector pRB473 (Brückner, Gene, 122, 187-192, 1992) and transformed into *E. coli* DH5 (Hanahan, J. Mol. Biol., 166, 557-580, 1983).

The gene libary was screened for a fragment containing the first half of Tn*917* (without erythromycin resistance) and its flanking region (Fig. 1B). The positive clone was identified by restriction analysis, Southern blotting using a 1.9 kb *Bgl*II-fragment specific for the transposon Tn*917* as probe and by nucleotide sequencing. A part of the chromosomal DNA which was hit by the transposon insertion in mutant nrI was used as probe in order to isolate the appropriate intact gene from wild-type. Altogether, three overlapping fragments were cloned from *S. carnosus* gene libaries:
(a) an 8.6 kb *Nco*I-fragment ligated into pACYC184 (Chang and Cohen, J. Bacteriol., 134, 1141-1156, 1978) and transformed into *E. coli* DH5 and,
(b) a 2.9 kb *Eco*RI-*Pst*I-fragment ligated into pUC18 (Yanisch-Perron *et al*., Gene, 33, 103-109, 1985) and transformed into *E. coli* SURE™ (Strategene) and,
(c) a 5.3 kb *Nco*I-fragment ligated into pACYC184 and transformed into *E. coli* DH5.

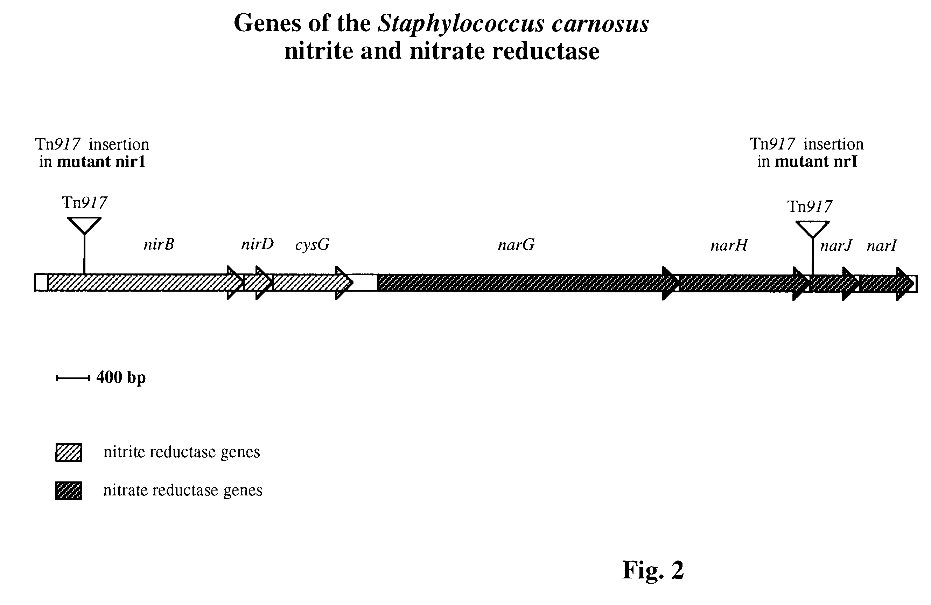

*S. carnosus* wild-type nitrate reductase was sequenced by using pUC18 and pBluescriptII KS+ (Stratagene) as vectors and *E. coli* SURE™ as host strain for subcloning. The nitrate reductase is encoded by an operon comprising 4 genes called *nar*G, *-H, -J* and *-I* (Fig. 2), said genes having each a nucleotide sequence listed in the sequence listing below (SEQ ID NO:1: *narG* = nucleotide 4140-7811; *narH* = nucleotide 7804-9378; *narJ* = nucleotide 9374-9946; *narI* = nucleotide 9942-10622). The deduced amino acid sequence of each subunit is also given in the sequence listing below (NarG: SEQ ID NO: 4; NarH: SEQ ID NO:6; NarJ: SEQ ID NO:7; NarI: SEQ ID NO:8).

For mutant nrI, the transposon insertion site could be identified. The transposon hit the nitrate reductase operon (*narGHJ*I) of *S. carnosus* in the beginning of the *narJ* gene (Fig. 2).

The amino-acid sequences of NarG,H, J and I of *S. carnosus* reveal identities to the proteins encoded by the nitrate reductase operon *nar*GHJI of *Escherichia coli* (Tab. 3a) (Blasco *et al*., Mol. Gen. Genet., 218, 249-256, 1989). Thus, it can be deduced from the comparison that the native nitrate reductase of *S. carnosus* is in fact composed of the three subunits NarG, NarH and NarJ. On the other hand, NarI is not part of the native enzyme, but is required for the biogenesis of the active enzyme complex.

**Table 3a**

| *Staphylococcus carnosus* proteins | Identity | number of aa overlapping | to |
|---|---|---|---|
| NarG (1224 aa¹) | 57.8% | 1221 | NarG (1228 aa) of *Bacillus subtilis* |
| | 49.2% | 1237 | NarZ (1245 aa) of *E. coli* |
| | 48.1% | 1238 | NarG (1238 aa) of *E. coli* |
| NarH (525aa) | 67.4% | 484 | NarH (487 aa) of *B. subtilis* |
| | 59.6% | 492 | NarY (514 aa) of *E. coli* |
| | 57.2% | 493 | NarH (512 aa) of *E. coli* |
| NarJ (191aa) | 41.0% | 83 | NarJ (184 aa) of *B. subtilis* |
| | 27.7% | 112 | NarJ (236 aa) of *E. coli* |
| | 24.5% | 106 | NarW (231 aa) of *E. coli* |
| NarI (227 aa) | 49.1% | 222 | NarI (223 aa) of *B. subtilis* |
| | 35.1% | 225 | NarV (226 aa) of *E. coli* |
| | 34.7% | 222 | NarI (225 aa) of *E. coli* |

| | | | |
|---|---|---|---|
| ¹⁾ amino acid | | | |

### Tn917 insertion mutant nrIII

Mutant nrIII does not accumulate nitrite when grown in modified BM supplemented with up to 10mM nitrate under anoxic conditions, but displayes wild-type levels of benzyl viologen-linked nitrate reductase activity. Cultivated under oxic conditions, mutant nrIII accumulates five-fold less nitrite than the wild-type. Its ability to reduce nitrite is indistinguishable from that of the wild-type.

Chromosomal DNA from nrIII was cleaved with various restriction endonucleases and probed with a Tn*917* specific 1.9kb *Bgl*II-fragment. A hybridizing 10.1kb *Pst*I-fragment, comprising Tn*917* and the flanking DNA regions of nrIII, was cloned in pT181mcs (Augustin et al., Eur. J. Biochem., 204, 1149-1154, 1992). *S. carnosus* wild-type was transformed with the resultant plasmid, pTnrIII-1. A 3.3kb *Ava*I-fragment of pTnrIII-1 covering one of the DNA regions adjacent to Tn*917*, served as a probe for hybridization of *Pst*I-cleaved wild-type DNA. A hybridizing 4.9kb *Pst*I-fragment was subcloned into the shuttle vector pRB473 and transformed to nrIII. The new plasmid was named pRBnitIII-4. pRBnitIII-4 was shown to complement the defect in nrIII. The detailed restriction analysis of the 4.9kb *Pst*I-fragment, together with the determination of the insertion site of Tn*917* represented the basis for the creation of subclones. All subclones were able to complement nrIII. The 2kb *Eco*RI-*Hpa*I insert of the smallest subclone, pRBnitIII-44, was expected to harbor the gene affected in nrIII and was sequenced. The nucleotide sequence is shown in the list of sequences below (*narT*: SEQ ID NO:9). An open reading frame was found. Analysis of the deduced amino acid sequence (NarT: SEQ ID NO:10) revealed that this gene, designated *nar*T, encodes a highly hydrophobic 41,869Da transmembrane protein of 388 amino acids showing identities to transport proteins (Tab. 3b), such as NarK of *E. coli* which has been assigned the role of an nitrite exporter (Rowe *et al*., Mol. Microbiol., 12, 579-586, 1994), and NasA of *B. subtilis* (Ogawa *et al*.*,* J. Bacteriol., 177, 1409-1413, 1995) which has been suggested to be involved in nitrate uptake.

**Table 3b**

| *Staphylococcus carnosus* proteins | Identity | aa overlapping | to |
|---|---|---|---|
| NarT (388 aa¹⁾) | 53.2% | 374 | NarK (395 aa) of *B. subtilis* |
| | 28.7% | 310 | NasA (421 aa) of *B. subtilis* |
| | 42.5% | 275 | NarK (463 aa) of *E. coli* |

| | | | |
|---|---|---|---|
| ¹⁾ amino acid | | | |

The postulated gene product, NarT, was overexpressed *in vivo* using the T7-system (Tabor and Richardson, Proc. Natl. Acad. Sci. USA 82, 1074-1078, 1985) and detected in SDS-PAGE. The results of the physiological studies and the similarity search strongly suggest that NarT represents a transport protein required for nitrate uptake under anoxic conditions in *S. carnosus*.

### Tn917 insertion mutant nrIV

Similar to mutant nrI, mutant nrIV shows neither nitrite accumulation during growth, nor nitrate reductase activity, which indicates that gene(s) essential for nitrate reduction are hit by the transposon insertion.

A similar strategy as described for Tn*917* insertion mutant nrIII led to the cloning of a 8.5kb *Pst*I-fragment comprising Tn*917* and flanking DNA region in the vector pT181mcs in *S. carnosus* wild-type. In order to isolate the corresponding intact DNA region, chromosomal DNA from *S. carnosus* wild-type was digested with various restriction endonucleases and probed with DNA flanking Tn*917* in mutant nrIV. A hybridizing 8.2kb EcoRV-fragment was cloned in pBluescriptII KS+ in *E. coli* SURE™. This fragment was analyzed by restriction analysis and was sequenzed using pUC18 and pBluescriptII KS+ as vectors and *E. coli* SURE™ as host strain for subcloning.

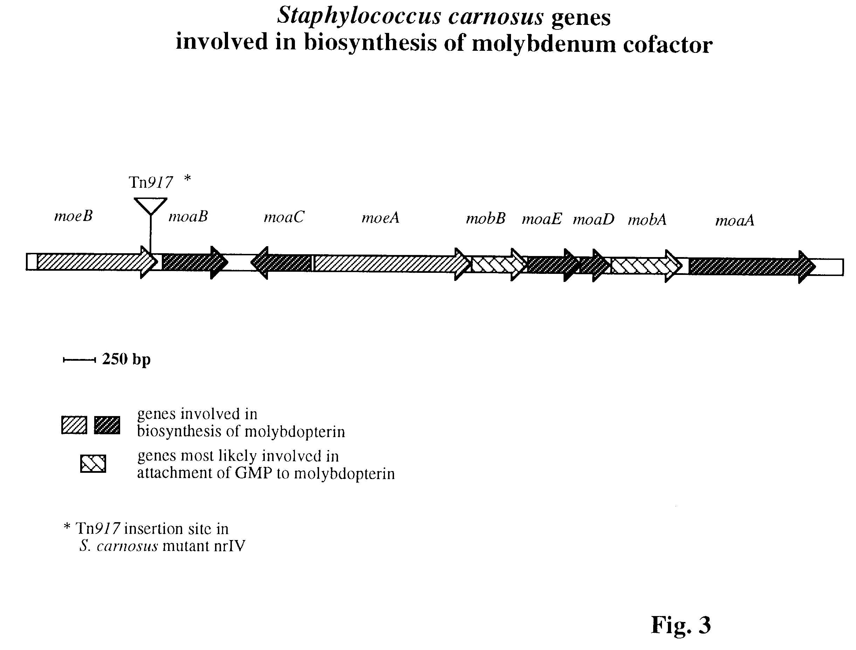

For mutant nrIV, the transposon insertion site was identified in a gene similar to *moe*B in *E. coli* (Fig. 3) which is involved in the biosynthesis of the for nitrate reduction essential molybdenum cofactor. Altogether, nine open reading frames were found (see SEQ ID NO: 11 in particular from nucleotide 117-1115 = *moeB*, 1150-1659 = *moaB*, 2396-3652 = *moeA*, 3652-4131 = *mobB*, 4131-4580 = *moaE*, 4583-4813 *moaD*, 4821-5408 = *mobA*, 5470-6489 = *moaA* and complementary nucleotide 2331-1849 = *moaC*). All of the open reading frames reveal identities to different proteins essential for the molybdenum cofactor biosynthesis (Tab. 3c; MoeB, MoaB, MoaC, MoeA, MoaD, MobA, MoaA, MobB, MoaE consisting of respectively amino acid sequences SEQ ID NO:12, NO:13, NO:14, NO:15, NO:16, NO:17, NO:18, NO:19 and NO:20)

In *E. coli* the products of at least five different loci - *moa, mob, mod, moe* and *mog and mol*R - are required for active expression of molybdoenzymes (Rivers *et al.*, Mol. Microbiol., 8, 1071-1081, 1993). Whereas the products of the *moa* and *moe* loci (MoaA, MoaB, MoaC, MoaD, MoaE, and MoeA, MoeB) are necessary for the synthesis of molybdopterin (Johnson and Rajagopalan, J. Bacteriol., 169, 110-116 and 117-125, 1987; Pitterle and Rajagopalan, J. Bacteriol., 171, 3373-3378, 1989), the proteins of the *mob* locus are required for a late step in molybdenum cofactor biosynthesis involving the addition of GMP to molybdopterin to produce molybdopterin guanin dinucleotide (MGD) (Johnson *et al*., Proc. Natl. Acad. Sci USA, 87, 3190-3194, 1990; Boxer *et al*., J. Bacteriol., 169, 4678-4685, 1987; Santini *et al.*, J. Gen. Microbiol., 135, 3467-3475, 1989; Giordano *et al*., Mol. Microbiol., 4, 645-650, 1990; Santini *et al*., J. Bacteriol., 174, 7934-7940, 1992). In *S. carnosus*, genes having the same functions to those of these three loci are clustered in one locus (Fig. 2).

**Table 3c**

| *Staphylococcus carnosus* proteins | Identity | aa¹ overlapping | to |
|---|---|---|---|
| MoeB (333 aa) | 30.7% | 241 | MoeB (249 aa) of *E. coli* |
| | 32.0% | 203 | MoeB (243 aa) *Haemophilus influenzae* |
| MoaB (170 aa) | 43.8% | 146 | MoaB (169 aa) of *E. coli* |
| | 33.0% | 115 | Mog (197 aa) of *H. influenzae* |
| | 31.3% | 115 | Mog (195 aa) of *E. coli* |
| MoaC (161 aa) | 49.1% | 161 | MoaC (160 aa) of *H. influenzae* |
| | 49.1% | 159 | MoaC *E. coli* (159 aa) |
| MoeA (419 aa) | 28.7% | 401 | MoeA (404 aa) of*H. influenzae* |
| | 28.2% | 383 | MoeA (411 aa) of *E. coli* |
| MobB (160 aa) | 28.2% | 170 | MobB (174 aa) of *E. coli* |
| | 29.5% | 149 | MobB (178 aa) of *H. influenzae* |
| MoaE (150 aa) | 36.0% | 150 | MoaE (150 aa) of *H. influenzae* |
| | 38.7% | 124 | MoaE (149 aa) of *E. coli* |
| MoaD (77 aa) | 30.9% | 81 | MoaD (81 aa) of *E. coli* |
| MobA (196 aa) | 21.8% | 197 | MobA (194 aa) of *E. coli* |
| MoaA (340 aa) | 49.3% | 339 | NarA (341 aa) of *B. subtilis* |
| | 33.4% | 320 | MoaA (329 aa) of *E. coli* |
| | 32.5% | 317 | MoaA (337 aa) of *H. influenzae* |

| | | | |
|---|---|---|---|
| ¹⁾ amino acid | | | |

### 4) Characterization of the nitrite reductase of S. carnosus

### A) Wild-type

Nitrite reduction during growth: when 2mM nitrite was added to aerobically growing cells of *S. carnosus*, no nitrite reduction was detected. The nitrite concentration remained constant until the cells were in the stationary phase. In contrast, nitrite was entirely reduced under anoxic conditions. As observed with nitrate, a higher growth yield was obtained when nitrite was present during anaerobic growth. Despite increasing concentrations of nitrite in the growth medium (1 to 15mM), the cells were not able to reduce more than 3 mM during 22h of anaerobic growth. The amount of nitrite reduced and the cell yield actually decreased slightly with higher nitrite concentrations. When 15mM nitrite was added, only 1mM nitrite was reduced during 22h of anaerobic growth.

Growth stimulation in the presence of nitrite was less distinct than that with nitrate (OD₅₇₈ increased only to 1.5 - 1.6 with nitrite, compared to 2.6 with nitrate). Considering that nitrate reduction is much faster than nitrite reduction (up to 25mM of nitrate can be reduced during anaerobic growth), the energy yield per time is higher with nitrate, although the molar energy yield from nitrate or nitrite might be similar.

Induction and end product of nitrite reduction in resting cells: resting cells are prepared as follows. Cells are grown anaerobically with 1mM of nitrite, harvested in the late-exponential growth phase, washed twice in potassium phosphate buffer (pH 7.2), and finally resuspended to an OD₅₇₈ of 2.0 in 40ml of the same buffer supplemented with 25mM glucose as electron donor.

Resting cells of *S. carnosus* are incubated anaerobically in nitrite-containing buffer (100mM potassium phosphate buffer, pH7.2; 200µM nitrite) with glucose as electron donor. For anaerobic incubation, Oxyrase and mineral oil are added. Nitrite reduction and ammonia production are monitored. Anaerobically grown cells reduced nitrite and converted it entirely into ammonia. After all nitrite was reduced, the ammonia concentration remained constant. Nitrite reduction was enhanced 4.5- and 5.5-fold when cells were precultured in the presence of nitrite (2mM) and nitrate (20mM), respectively. The rates of nitrite reduction were not affected by the presence of ammonia (18mM).

Consistent with the results from the growth experiments, no nitrite reduction was observed when the cells were precultured aerobically, regardless of the presence of nitrite in the growth medium.

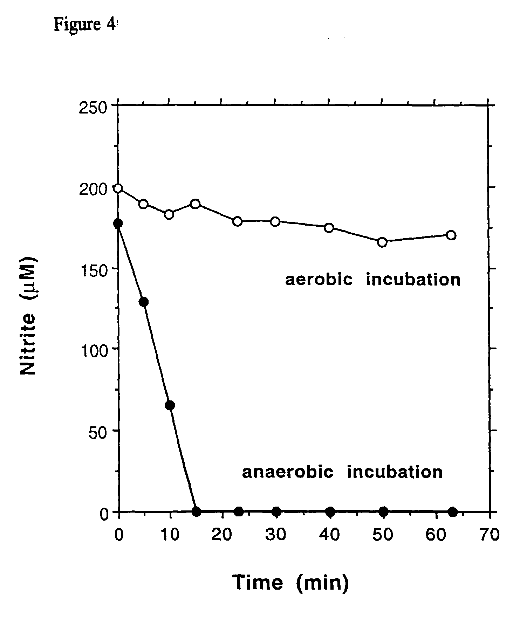

Under oxic conditions, that is to say when cultures are incubated with rapid stirring, the nitrite reductase activity of the anaerobically grown resting cells is strongly inhibited (Fig. 4). These results suggest that the activity and the synthesis of nitrite reductase is effectively inhibited and repressed by oxygen and induced by anaerobiosis and nitrite and nitrate.
Figure 4 illustrates the effect of oxygen on nitrite reduction in resting cells of wild-type *S. carnosus* TM300.
B) Tn*917* insertion mutant nir1: for identification of genes involved in nitrite reduction in *S. carnosus* TM300, Tn*917* insertion mutants were screened for their ability to reduce nitrite during anaerobic growth. One nitrite reductase negativ mutant, nir1, was selected. A similar strategy as described for Tn*917* insertion mutant nrI led to the identification of the transposon insertion site in a gene called *nirB* (SEQ ID NO:1, nucleotide 181-2583; Fig. 2). The deduced amino acid sequence of the protein NirB (SEQ ID NO:2) encoded by *nirB* reveals 52% identity to the *B. subtilis* large subunit of the nitrite reductase (Tab. 3d). *NirB* is the first structure gene of the nitrite reductase of *S. carnosus*, followed by two other genes called *nirD* and *cysG* (SEQ ID NO: 1: *nir D* = nucleotide 2589-2900; *cysG*: nucleotide 2894-3844; Fig. 2). Homology comparisons indicate that *nirD* encodes the NirD protein (SEQ ID NO:3) which represents the small subunit of the active enzyme. *CysG* encodes the CysG protein (SEQ ID NO:5) which is necessary for biosynthesis of the essential siroheme cofactor. This operon is located proximal upstream of the nitrate reductase operon *NarGHJ*I described above (Fig. 2).

**Table 3d**

| *Staphylococcus carnosus* proteins | Identity | aa¹⁾ overlapping | to |
|---|---|---|---|
| NirB (801 aa¹⁾) | 52.0% | 796 | NasD (805 aa) of *B. subtilis* |
| | 41.9% | 589 | NasB (770 aa) of *B. subtilis* |
| | 34.9% | 816 | NirB (847 aa) of *E. coli* |
| NirD (104 aa) | 49.5% | 101 | NasE (106 aa) of *B.subtilis* |
| | 30.0% | 100 | NirD (102 aa) of *E. coli* |
| CysG (296 aa) | 41.2% | 238 | SUMT (238 aa) of *Bacillus megaterium* |
| | 37.0% | 243 | CysG (457 aa) of *E. coli* |

### 5.) Treatment of nitrate polluted material (Biotransformation)

Principally, nitrate in water, vegetables and other nitrate polluted materials can be reduced by the nitrate and nitrite reductase of *S. carnosus* (A) or by a combination of the nitrate reductase of *S. carnosus* with the nitrite reductase of a denitrifying organism, such as *L. plantarum* (B).

### (A) Nitrate- and nitrite reduction by S. carnosus

In buffer: the time course of nitrite and ammonia formation after addition of nitrate was determined in resting cells (precultured anaerobically and harvested in the mid-exponential phase of growth) that were incubated anaerobically in glucose-containing buffer (100mM potassium phosphate buffer, pH 7.2). In the beginning, only nitrite was formed, and no ammonia was detected. Ammonia production started after all the nitrate was reduced. Nitrite was subsequently converted entirely into ammonia. These results indicate that the presence of nitrate inhibits nitrite reduction.

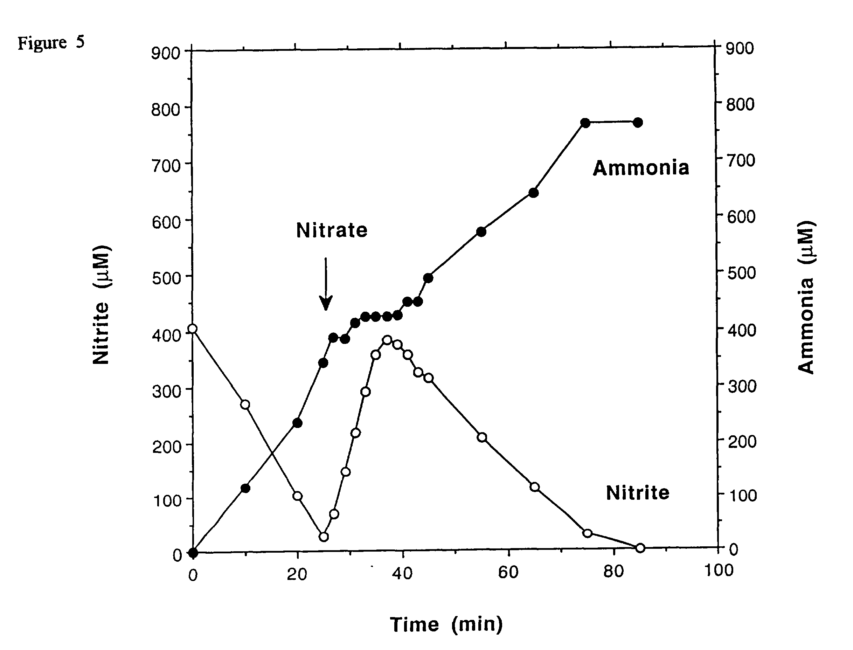

These results were confirmed in another experiment. Cells were grown anaerobically with 1mM nitrite and harvested in the late-exponential growth phase. After washing (twice in potassium phosphate buffer, pH 7.2), the cells were resuspended to an OD₅₇₈ of 2.0 in 50ml of the same buffer supplemented with 25mM glucose, Oxyrase, and mineral oil. Nitrite was added at zero time, and the production of nitrite and ammonia during anaerobic incubation at 37°C was monitored. After 26min., nitrate (400µM) was added and incubation was continued. The results are presented in Figure 5. Nitrate reduction started immediately and was monitored by a rapid increase of the nitrite concentration in the buffer. Ammonia production ceased after nitrate was added and restarted only after nitrate was completely reduced.

### (B) Nitrate reduction by S. carnosus combined with L. plantarum

In buffer: *Staphylococcus carnosus* strain Duploferment 66 (Müller, DE), called STC2, was used to reduce nitrate to nitrite and combined with *Lactobacillus plantarum* LP63 (NESTLE collection, Centre de recherche Nestle, Vers-Chez-Les-Blanc, Suisse) for further nitrite reduction.

For the preparation of resting cells, *L. plantarum* and *S. carnosus* were cultivated anaerobically overnight at 30°C in MRS broth (De Man *et al*., J. Appl. Bacteriol., 23, 130-135, 1960) and BHI broth (2.75% brain-, heart-extract and peptones; 0.2% D-glucose, 0.5% sodium chloride, 0.25% di-sodium hydrogenphosphate), respectively. The optical density (OD) of the cell suspension in 50mM phosphate buffer (pH5.5) is adjusted to 10 (at 600 nm), corresponding to about 9.5x10⁸ Colony Forming Unit (CFU) per ml.

Fermentation is carried out in 200mM phosphate buffer (pH5.5) containing 200mM glycerol (H-donor), 0.1% haematin and 50mM NaNO₃. The fermentation medium is continuously flushed with nitrogen to maintain an anaerobic environment. Initial fermentation conditions are given in Table 4.

**Table 4**

| | Assay 1 | Assay 2 | Assay 3 |
|---|---|---|---|
| Volume (ml) | 32.5 | 32.5 | 550 |
| Anaerobicity | nitrogen flush | nitrogen flush | stirred |

| Inoculation cell suspension | | | |
|---|---|---|---|
| *S.carnosus* STC2 | 3% | 3% | 1% |
| *L. plantarum* LP63 | | 15% | 15% |
| NaNO₃ (mM) | 50 | 50 | 50 |
| pH / temperature °C | 5.5 / 40 | 5.5 / 40 | 5.5 / 40 |

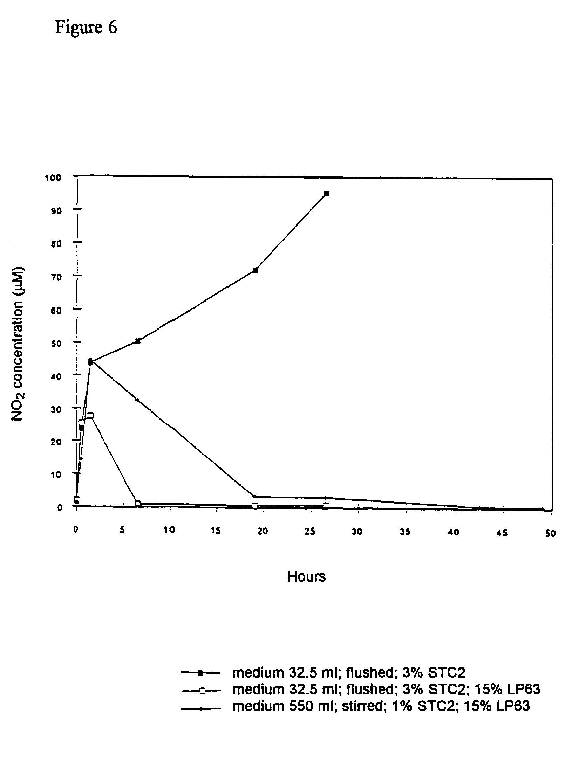

Nitrite concentration is determined by HPLC (column PRP-X100 Guard) using a mobile phase of 60% methane sulphonylchloride/40% acetonitrile, and a flow rate of 1.5ml/min. The concentration of nitrite in the medium in relation to the fermentation time is shown in figure 6.

*S. carnosus* reduces nitrate completely but does not reduce accumulated nitrite under these conditions. After 10h, the nitrite concentration exceeds 50mM which is probably caused by evaporation of water.

In the fermentations carried out with 15% of *L. plantarum*, a clear reduction of the initially accumulated nitrite is observed. The fermentation under anaerobic conditions is preferred.

### In carrot juice using immobilised cells

Cell suspensions of *S.carnosus* and *L. plantarum* are mixed (1:5), and entrapped in k-carrageenan gelbeads. The gelbeads are prepared by dissolving 13g k-carrageenan in 450ml bidistilled water at 60°C, sterilising the solution at 121°C during 5 min, adjusting the diameter of the beads from 1.5 to 2.5mm at 40°C. These gelbeads are added to 40 ml of the cell suspension at a temperature of 0°C. The immobilised cells are stable in 50mM phosphate buffer at 4°C for several weeks.

For the preparation of carrot juice, carrots are washed, trimmed, blanched (100°C for 5 min), and processed conventionally to carrot juice. 500 ml of carrot juice are inoculated with 200 g gelbeads, flushed with nitrogen to maintain an anaerobic environment, and incubated at 30°C for 50min. At different time points, the nitrite concentration in the carrot juice is determined. Since natural components of the juice interfere with the HPLC method, nitrite determination is carried out with the diazo-coupling method of Junger *et al*. (Fleischwirtsch., 61, 791-792, 1982).

Results show that the carrot juice contains a similar nitrite concentration in relation to the time fermentation as those obtained for assay 2 of the cofermentation in buffer.

Several fermentations are subsequently carried out with the same gelbeads without any significant changes in the nitrate and nitrite reduction.

## Claims

1. A recombinant protein involved in nitrate and nitrite reduction in *Staphylococcus carnosus*, selected from the nitrate reductase NarGHJ having three subunits consisting of respectively amino acid sequences SEQ ID NO:4, NO:6 and NO:7, the nitrate reductase biogenesis protein NarI consisting of amino acid sequence SEQ ID NO:8, the nitrate transport protein NarT consisting of amino acid sequence SEQ ID NO:10, the nitrate reductase molybdenum cofactor biosynthesis proteins MoeB, MoaB, MoaC, MoeA, MoaD, MobA, MoaA, MobB, MoaE consisting of respectively amino acid sequences SEQ ID NO:12, NO:13, NO:14, NO:15, NO:16, NO:17, NO:18, NO:19 and NO:20, the nitrite reductase protein NirBD having two subunits consisting of respectively amino acid sequences SEQ ID NO:2 and NO:3, the nitrite reductase sirohem cofacteur biosynthesis protein CysG consisting of amino acid sequence SEQ ID NO:5, and functional derivatives of these proteins.

2. A DNA molecule encoding a recombinant protein according to claim 1.

3. A DNA molecule according to claim 2 comprising at least an encoding part of at least 20 base pairs (bp) of a DNA sequence selected from the group comprising the DNA sequence SEQ ID NO:1 in particular from nucleotide 181-2583 (*nirB*), 2589-2900 (*nirD*), 2894-3844 (*CysG*), 4140-781 1(*narG*), 7804-9378 (*narH*), 9374-9946 (*narJ*) and 9942-10622 (*narI*), the DNA sequence SEQ ID NO:9 in particular from nucleotide 538-1701 (*narT*), the DNA sequence SEQ ID NO:11 in particular from nucleotide 117-1115 (*moeB*), 1150-1659 (*moaB*), 2396-3652 (*moeA*), 3652-4131 (*mobB*), 4131-4580 (*moaE*), 4583-4813 (*moaD*), 4821-5408 (*mobA*), 5470-6489 (*moaA*) and complementary nucleotide 2331-1849 (*moaC*).

4. A DNA molecule according to claim 2, wherein the nucleotide sequence originates from *Staphylococcus carnosus*.

5. A DNA molecule according to claim 2, wherein the nucleotide sequence is present in a vector.

6. A DNA molecule according to claim 5, wherein the nucleotide sequence is operably linked to at least one regulatory sequence able to direct the expression of the said nucleotide sequence.

7. A DNA molecule according to claim 5, wherein the regulatory sequence is derived from another organism than the one from which the nucleotide sequence is derived.

8. A recombinant cell expressing at least a recombinant protein according to claim 1.

9. A recombinant cell according to claim 8, the said cells being able to overexpress the recombinant protein.

10. A method for producing a recombinant protein according to claim 9, comprising cultivating recombinant cells according to claims 8 and 9 in a suitable growth medium under conditions that the cells express the said recombinant protein, and optionally isolating the said recombinant protein in the form of a concentrate.

11. Use of a recombinant protein according to claim 1 or recombinant cells according to claim 8 to reduce nitrate in a nitrate polluted material.

12. A method to reduce nitrate to a non-toxic compound in a nitrate polluted material comprising, treating the said material with NarGHJ proteins or functional derivatives according to claim 1, treating said material with NirBD proteins or functional derivatives according to claim 1, and recovering the nitrate and nitrite free material.

13. A method according to claim 12, in which the said nitrate polluted material is derived from vegetables.

14. A method to reduce nitrate according to claim 12, in which the material is treated with cells expressing NarGHJ, NarI, MoeB, MoaB, MoaC, MoeA, MobB, MoaE, MoaD, MobA, MoaA and NarT proteins or functional derivatives according to claim 6.

15. A method to reduce nitrite according to claim 12, in which the material is treated with cells expressing NirBD and CysG proteins or functional derivatives according to claim 1.

16. A method to reduce nitrite according to claim 14, in which the material is treated with naturally denitrifying cells instead of cells expressing NirBD and CysG proteins.

17. A method to reduce nitrite, in which said material is treated with denitrifying cells according to claim 16 expressing the recombinant proteins NarGHJ, MoeB, MoaB, MoaC, MoeA, MobB, MoaE, MoaD, MobA, MoaA, NarI and NarT proteins or functional derivatives according to claim 1.

18. A method according to any claims 12 to 17, in which said cells or enzymes are immobilised.


SEQUENCE LISTING

(1) GENERAL INFORMATION:
(i) APPLICANT:
(A) NAME: SOCIETE DES PRODUITS NESTLE
(B) STREET: AVENUE NESTLE 55
(C) CITY: VEVEY
(E) COUNTRY: SWITZERLAND
(F) POSTAL CODE (ZIP): CH-1500
(G) TELEPHONE: (41) 21 924 4760
(H) TELEFAX: (41) 21 924 2880
(ii) TITLE OF INVENTION:
Nitrate reduction system of Staphylococcus carnosus
(iii) NUMBER OF SEQUENCES: 20
(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:
(i) SEQUENCE CHARACTERISTICS:
(A) LENGTH: 10670 base pairs
(B) TYPE: nucleic acid
(C) STRANDEDNESS: double
(D) TOPOLOGY: linear
(ii) MOLECULE TYPE: DNA (genomic)
(ix) FEATURE:
(A) NAME/KEY: -35_signal
(B) LOCATION:93..98
(ix) FEATURE:
(A) NAME/KEY: -10_signal
(B) LOCATION:116..122
(ix) FEATURE:
(A) NAME/KEY: RBS
(B) LOCATION:167..171
(ix) FEATURE:
(A) NAME/KEY: CDS
(B) LOCATION:181..2583
(D) OTHER INFORMATION:/function= "nirB gene"
(ix) FEATURE:
(A) NAME/KEY: CDS
(B) LOCATION:2589..2900
(D) OTHER INFORMATION:/function= "nirD gene"
(ix) FEATURE:
(A) NAME/KEY: misc_feature
(B) LOCATION:2894..3844
(D) OTHER INFORMATION:/function= "cysG gene"
(ix) FEATURE:
(A) NAME/KEY: terminator
(B) LOCATION:3853..3878
(ix) FEATURE:
(A) NAME/KEY: -35_signal
(B) LOCATION:4037..4042
(ix) FEATURE:
(A) NAME/KEY: -10_signal
(B) LOCATION:4057..4062

(ix) FEATURE:
(A) NAME/KEY: RBS
(B) LOCATION:4128..4134
(ix) FEATURE:
(A) NAME/KEY: CDS

(B) LOCATION:4140..7811
(D) OTHER INFORMATION:/function= "narG gene"
(ix) FEATURE:
(A) NAME/KEY: CDS
(B) LOCATION:7804..9378
(D) OTHER INFORMATION:/function= "narH gene"
(ix) FEATURE:
(A) NAME/KEY: CDS
(B) LOCATION:9374..9946
(D) OTHER INFORMATION:/function= "narJ gene"
(ix) FEATURE:
(A) NAME/KEY: CDS
(B) LOCATION:9942..10622
(D) OTHER INFORMATION:/function= "narI gene"
(xi) SEQUENCE DESCRIPTION: SEQ ID NO: 1:

(xi) SEQUENCE DESCRIPTION: SEQ ID NO: 1:

CAGCAATTAG TAAACCATAC GGGTGATAAA TCACAATCAG ATTTTTTCAA TATGATAGAA 60

TTTGATTTTG AAGATTTAGC CATCAGTGTG CGTTCAAACG GCGGGGATTA TAAAAAAGTA 120

AAACAAGTTT CAAAAGCGAT AGAAAAGTAT TTACAGGAAG AATATGGGAG GGAATAACGG 180

ATG TCT AAA CAA CGA TTA GTT ATG ATC GGA AAT GGG ATG GCC GGA GTA 228
Met Ser Lys Gln Arg Leu Val Met Ile Gly Asn Gly Met Ala Gly Val
1               5                   10                  15

AGA ACA ATT GAG GAA ATT ATA GAT CGA GAT CCT GAT AAA TTC GAT ATT 276
Arg Thr Ile Glu Glu Ile Ile Asp Arg Asp Pro Asp Lys Phe Asp Ile
            20                  25                  30

ACA ATT ATT GGG AAA GAA CCA TAT CCG AAC TAT AAC CGT ATC ATG TTA 324
Thr Ile Ile Gly Lys Glu Pro Tyr Pro Asn Tyr Asn Arg Ile Met Leu
        35                  40                  45

TCT AAT ATT TTA CAG AAA AAG ATG ACA GTT GAA GAA ACG ATA ATG AAT 372
Ser Asn Ile Leu Gln Lys Lys Met Thr Val Glu Glu Thr Ile Met Asn
    50                  55                  60

CCT TAT TCA TGG TAT GAA GAA AAT GGT ATT GCA TTA ATT ACT GGA GAA 420
Pro Tyr Ser Trp Tyr Glu Glu Asn Gly Ile Ala Leu Ile Thr Gly Glu
65                  70                  75                  80

AAA GCA ACA CGT GTT AAT CGT GAA AAA CAT ATA GTA GAG ACT GAA AAA 468
Lys Ala Thr Arg Val Asn Arg Glu Lys His Ile Val Glu Thr Glu Lys
                85                  90                  95

GGT CGC GAG ATT CCT TAT GAT ATC TGT ATT TTC GCA ACA GGT TCT AAA 516
Gly Arg Glu Ile Pro Tyr Asp Ile Cys Ile Phe Ala Thr Gly Ser Lys
            100                 105                 110

GCA TTT GTA TTA CCA ATT CCA GGA CAT GAG TTA CCA AGT GTA ATC GGT 564
Ala Phe Val Leu Pro Ile Pro Gly His Glu Leu Pro Ser Val Ile Gly
        115                 120                 125

TGG CGT ACA ATC GAA GAT ACT GAA CGT ATG ATT GAG ATT GCA CAA GAT 612
Trp Arg Thr Ile Glu Asp Thr Glu Arg Met Ile Glu Ile Ala Gln Asp
    130                 135                 140

AAA AAA GAA GCC ACT GTT ATC GGC GGT GGT TTG CTT GGT CTT GAA TGT 660
Lys Lys Glu Ala Thr Val Ile Gly Gly Gly Leu Leu Gly Leu Glu Cys
145                 150                 155                 160

GCT CGA GGA TTA TTG GAT CAA GGT ATG AAT GTC ACA GTT GTA CAT CTT 708
Ala Arg Gly Leu Leu Asp Gln Gly Met Asn Val Thr Val Val His Leu
                165                 170                 175

GCT GAA TGG CTG ATG GAA ATG CAA TTA GAC CGT CAA GCA GGA GAT ATG 756
Ala Glu Trp Leu Met Glu Met Gln Leu Asp Arg Gln Ala Gly Asp Met
            180                 185                 190

CTG CGT GAA GAT TTA GAA AAG CAA GGC ATG AAA TTT GAA CTT GGT GCT 804
Leu Arg Glu Asp Leu Glu Lys Gln Gly Met Lys Phe Glu Leu Gly Ala
        195                 200                 205

AAT ACA AAA GAA ATT ATC GGA AAC CAA GAT GTA GAA GCG ATT CGT TTG 852
Asn Thr Lys Glu Ile Ile Gly Asn Gln Asp Val Glu Ala Ile Arg Leu
    210                 215                 220

GCG GAT GGC CGC GAC CTG CCT GCT GAT TTA GTT GTT ATG GCA GTA GGT 900
Ala Asp Gly Arg Asp Leu Pro Ala Asp Leu Val Val Met Ala Val Gly
225                 230                 235                 240

ATC AAA CCA TTT ACA CAA GAA GCA ATT GAT TCT GGC ATC GAT GTT GAA 948
Ile Lys Pro Phe Thr Gln Glu Ala Ile Asp Ser Gly Ile Asp Val Glu
                245                 250                 255

CGC GGT ATT GTC GTA GAT GAT TTT ATG CGT ACA AGT GAT CCT GAA ATT 996
Arg Gly Ile Val Val Asp Asp Phe Met Arg Thr Ser Asp Pro Glu Ile
            260                 265                 270

TAT GCA GTT GGT GAA TGT GCA CAA CAT AAC TGC AAA GTT TAT GGT TTA 1044
Tyr Ala Val Gly Glu Cys Ala Gln His Asn Cys Lys Val Tyr Gly Leu
        275                 280                 285

GTT GCG CCT TTA TAT GAA CAA GGT AAA GTA TTA GCT GAT CAT TTA ACA 1092
Val Ala Pro Leu Tyr Glu Gln Gly Lys Val Leu Ala Asp His Leu Thr
    290                 295                 300

GGT CGT CCT ACT GAA GGT TAT CAA GGT TCT ACA ACA TTT ACT TCT TTG 1140
Gly Arg Pro Thr Glu Gly Tyr Gln Gly Ser Thr Thr Phe Thr Ser Leu
305                 310                 315                 320

AAA GTG TCT GGT TGT GAT TTA TAT AGC GCA GGT CAA ATT CAA GAA GAT 1188
Lys Val Ser Gly Cys Asp Leu Tyr Ser Ala Gly Gln Ile Gln Glu Asp
                325                 330                 335

GAT GAA GTC GAA GGT GTA GAA ATT TAT AAC AGT CAT GAT CAT GTC TAC 1236
Asp Glu Val Glu Gly Val Glu Ile Tyr Asn Ser His Asp His Val Tyr
            340                 345                 350

AAA AAA ATC TTC TTA AAA GAA GAA AGA GTT GTA GGG GCT GTC TTA TAT 1284
Lys Lys Ile Phe Leu Lys Glu Glu Arg Val Val Gly Ala Val Leu Tyr
        355                 360                 365

GGT GAT ACA GAT GAC GGC ACA CGT TTC TTC AAT ATG ATG AAA AAG CGT 1332
Gly Asp Thr Asp Asp Gly Thr Arg Phe Phe Asn Met Met Lys Lys Arg
    370                 375                 380

GAA CCG ATT GAA GAC TAT ACA CTT GTC TCT CTT TTA CAA AAA GTA GGA 1380
Glu Pro Ile Glu Asp Tyr Thr Leu Val Ser Leu Leu Gln Lys Val Gly
385                 390                 395                 400

GAA GAA GCG GTA TCT ACA GTT GCT GAT ATG GAT GAC AGC GAG ACA ATT 1428
Glu Glu Ala Val Ser Thr Val Ala Asp Met Asp Asp Ser Glu Thr Ile
                405                 410                 415

TGT GGT TGT AAC GGT GTA TCA AAA GGG AAA ATT GTC AGT GCG ATT GTT 1476
Cys Gly Cys Asn Gly Val Ser Lys Gly Lys Ile Val Ser Ala Ile Val
            420                 425                 430

GAA GAT GGT TTA ACT TCA GTG GCT GAA GTG ACT AAA GCA ACA AAA GCA 1524
Glu Asp Gly Leu Thr Ser Val Ala Glu Val Thr Lys Ala Thr Lys Ala
        435                 440                 445

GGT AAT TCT TGC GGT AAG TGT AAA CCG CAA ATC GGT GAA TTA TTA GAA 1572
Gly Asn Ser Cys Gly Lys Cys Lys Pro Gln Ile Gly Glu Leu Leu Glu
    450                 455                 460

CAT ACA TTA GGC GGT GCT TTT GTA GCA TCT AAG CCA GCT GGT ATT TGC 1620
His Thr Leu Gly Gly Ala Phe Val Ala Ser Lys Pro Ala Gly Ile Cys
465                 470                 475                 480

GAT TGT ACA GAA TTA ACA CGT GAT CAA ATC GTA ACG CAA ATT CGT GCC 1668
Asp Cys Thr Glu Leu Thr Arg Asp Gln Ile Val Thr Gln Ile Arg Ala
                485                 490                 495

AAA GGT TTA AAA ACA TCG AAA GAA GTA CGT CAT GTT CTA GAC TGG AAA 1716
Lys Gly Leu Lys Thr Ser Lys Glu Val Arg His Val Leu Asp Trp Lys
            500                 505                 510

AAT AAA GGC GGT TGT CCG AAG TGT CGT CCA GCT ATC AAC TAT TAT TTG 1764
Asn Lys Gly Gly Cys Pro Lys Cys Arg Pro Ala Ile Asn Tyr Tyr Leu
        515                 520                 525

AAT ATG GTT TAT CCT CAT GAA CAT GAA GAT GAA AAA GCA TCA CGT TTT 1812
Asn Met Val Tyr Pro His Glu His Glu Asp Glu Lys Ala Ser Arg Phe
    530                 535                 540

GCA AAT GAA CGA TAC CAT GCC AAT ATT CAA AAT GAT GGA ACA TTC TCT 1860
Ala Asn Glu Arg Tyr His Ala Asn Ile Gln Asn Asp Gly Thr Phe Ser
545                 550                 555                 560

GTT ATC CCA CAA ATG CGC GGT GGT GTC ACA AAT CCT GAT CAA CTT ATA 1908
Val Ile Pro Gln Met Arg Gly Gly Val Thr Asn Pro Asp Gln Leu Ile
                565                 570                 575

CGT TTA GGT GAA GTT GCG AAG AAG TAT GAT GTG CCA TTG GTA AAA GTA 1956
Arg Leu Gly Glu Val Ala Lys Lys Tyr Asp Val Pro Leu Val Lys Val
            580                 585                 590

ACA GGT TCC CAA CGT ATC GGT TTA TAT GGT ATT AGC AAA GAA GAC TTG 2004
Thr Gly Ser Gln Arg Ile Gly Leu Tyr Gly Ile Ser Lys Glu Asp Leu
        595                 600                 605

CCG AAG GTT TGG AAA GAT TTA GGT ATG CGT TCA GCC TCT GCA TAT GCT 2052
Pro Lys Val Trp Lys Asp Leu Gly Met Arg Ser Ala Ser Ala Tyr Ala
    610                 615                 620

AAG AAA ACA CGT TCT GTT AAA AGT TGT GTC GGT AAA GAA TTC TGT CGT 2100
Lys Lys Thr Arg Ser Val Lys Ser Cys Val Gly Lys Glu Phe Cys Arg
625                 630                 635                 640

TTC GGA ACA CAA TAT ACA ACA CGT CTT GGT ATT CGC TTA GAA GAA ACG 2148
Phe Gly Thr Gln Tyr Thr Thr Arg Leu Gly Ile Arg Leu Glu Glu Thr
                645                 650                 655

TTT GAA TAT ATC GAT ACG CCG CAT AAA TTT AAA ATG GGT GTT TCT GGT 2196
Phe Glu Tyr Ile Asp Thr Pro His Lys Phe Lys Met Gly Val Ser Gly
            660                 665                 670

TGT CCG CGT AGC TGT GTT GAA TCT GGT GTG AAA GAC TTC GGT GTA ATC 2244
Cys Pro Arg Ser Cys Val Glu Ser Gly Val Lys Asp Phe Gly Val Ile
        675                 680                 685

GGA GTA GAA AAT GGT TTC CAA ATT TAT ATT GGC GGT AAC GGC GGT ACT 2292
Gly Val Glu Asn Gly Phe Gln Ile Tyr Ile Gly Gly Asn Gly Gly Thr
    690                 695                 700

GAA GTA ACG AAA GGT CAA TTC TTA ACT ACA GTA GAA ACT GAA GAA GAC 2340
Glu Val Thr Lys Gly Gln Phe Leu Thr Thr Val Glu Thr Glu Glu Asp
705                 710                 715                 720

GTG ATT CAA TTA TGC GGT GCT TAT ATG CAA TAT TAC CGT GAA ACT GGT 2388
Val Ile Gln Leu Cys Gly Ala Tyr Met Gln Tyr Tyr Arg Glu Thr Gly
                725                 730                 735

ATA TAT GCG GAA CGT ACT GCA CCT TGG TTA GAA CGT ATG GGC TTT GAA 2436
Ile Tyr Ala Glu Arg Thr Ala Pro Trp Leu Glu Arg Met Gly Phe Glu
            740                 745                 750

AAT GTA AAA GAA TAT GTT TTG GAT CCT GAA CGT CAA AAA GCA TTA TAT 2484
Asn Val Lys Glu Tyr Val Leu Asp Pro Glu Arg Gln Lys Ala Leu Tyr
        755                 760                 765

GAT CGT ATC ATG GAT GCC AAA CGT GCT GTG GCA GAA GAA CCA TGG TCA 2532
Asp Arg Ile Met Asp Ala Lys Arg Ala Val Ala Glu Glu Pro Trp Ser
    770                 775                 780

GAA ATT GTT AAT GAT CAA GCA GAT CAA AAA ATC TTT AAA GTA GAG AAG 2580
Glu Ile Val Asn Asp Gln Ala Asp Gln Lys Ile Phe Lys Val Glu Lys
785                 790                 795                 800

GTG TAAGC ATG CGA GCA ATA GAA AAA GTA AGA GTA GCA GCA TTA GAG 2627
Val Met Arg Ala Ile Glu Lys Val Arg Val Ala Ala Leu Glu
1 5 10

GAT TTA ACA CCT TTA ATT GGT ACA AAA GTT CTC GTA CGC GAT AAA GAA 2675
Asp Leu Thr Pro Leu Ile Gly Thr Lys Val Leu Val Arg Asp Lys Glu
15 20 25

ATT GGA TTG TTT TTA ACA GAA GAC GGT AGA ATC TTT GCG GTT AAT AAT 2723
Ile Gly Leu Phe Leu Thr Glu Asp Gly Arg Ile Phe Ala Val Asn Asn
30 35 40 45

GTT TGT CCG CAC AAA CAA GGT CCT TTA TCA GAA GGA ACT GTA AGT GAA 2771
Val Cys Pro His Lys Gln Gly Pro Leu Ser Glu Gly Thr Val Ser Glu
50 55 60

CAT TCA GTA TTT TGT CCA CTT CAC GAT CAA CAA ATT GAT TTA GAA ACA 2819
His Ser Val Phe Cys Pro Leu His Asp Gln Gln Ile Asp Leu Glu Thr
65 70 75

GGC CTT GTT CAA GAA CCG GAT ACG GGT TGC GTT GAA ACA TAT GAA GTC 2867
Gly Leu Val Gln Glu Pro Asp Thr Gly Cys Val Glu Thr Tyr Glu Val
80 85 90

GAA ATC ATC GAT GGA GAT GTA TAT TTA TGT CTG TAAGTCCAAC CGGCCATGTT 2920
Glu Ile Ile Asp Gly Asp Val Tyr Leu Cys Leu
95 100

TATCTTATCG GAGCGGGTCC AGGAAACCCG AACTTATTAA CACGTAAGGC TGAACATCTT 2980

ATTCGAACAG CGGATATCAT TTTGTATGAC CGCTTAGTTA ATCCATTTAT CTTACAACTT 3040

GCACCACCTG AAATAGAGAT TATTGATGTT GGCAAAAAGC CCTATAGCAG ACACATTCAA 3100

CAAAGTGAAA TTAATGAAGT ATTATTAGAT GCTGCTTACA AACATCGAAA TGTTGTCCGT 3160

TTAAAAGGTG GAGACCCTGC AATTTTCGGC AGAGTTCATG AAGAAGTAGA TATTTTAAAA 3220

GAACACAACA TTCCTTGTGA AATTGTACCA GGCATTACGA CAGCCAGTGC AGCAGCTGCA 3280

ACGCTCGGTG TCGGACTGAC TTGCCGTAAT GTCTCAACAA GTGTGACATT GACGACAGGC 3340

AGTTTTAAAG GATCTGAAGA ACAAGAGATT GATATTGCTT CTTTAATTGA TGGCGGCACA 3400

TTAGCCATTT ATATGGGAAT CAAACGTTTG CATACGATAA TTACTCAAAT CTATAAGCAA 3460

GTGGGTAAAG ATTATCCGAT TTGTGCAGTG TTTGATGTAT CTTATGCGAC ACAAAAAGTT 3520

ATCACTGGCC ACCTTTCTGA TATTGAGAAA AAAGTACAAG CCAATGCACC AGAAGATGCA 3580

CCTGGTATAT TGCTTATTGG TGATGCATTA GAGTATGCGG ATGAAAATAC ACATATTCAG 3640

TTGGAAGAAA CATCAAAATA TTTCGGTAAA GAATGTATCG TAACCGGACC AAGAGAAGCA 3700

GCAATTGAAA AAGCATTTGA AATATATGAT GCAGGCGGCT GGTGTATGAT AGATCCGAAC 3760

AGCGATGACC ATTATCATCA AAGTCAATTA GATTTGATTT ATCGTTATAT GCAAGTGAAT 3820

GACAATACAA TCATTGTAAA AGCATAGTAA GTATAAAACG GGCTGAGGCC CGTTTTTTTA 3880

TGCGTATTTT TATACAAGTT GTGCCTAAGT ATATTGCTGG TCGGTAATGA CAGAAGAATC 3940

CCAAACAATA TACTGTTAAA TCTATGTATA TCTTTTTAAC ACCAAGTGCA ATAAAAGTAG 4000

GGAATACCCC TAGGCATATA GGAGAATCCC TAATACCTGA AACACTCATC AAAATATACA 4060

ATTAAATTAA GTCGAAAAAG CAGTCACTGA TAATTGAACA GCTAAGTGTG CAACGTAATT 4120

TGATGAGAGG ATGTTATAT ATG GCG AAA TTT GGA ATG AAC TTT TTT AAG CCA 4172
Met Ala Lys Phe Gly Met Asn Phe Phe Lys Pro
1 5 10

ACT GAA AAA TTT AAC GGC AAT TGG TCG GTT TTA ACA GAT AAA AGC AGA 4220
Thr Glu Lys Phe Asn Gly Asn Trp Ser Val Leu Thr Asp Lys Ser Arg
15 20 25

GAA TGG GAG AAA ATG TAT CGT GAA CGT TGG AGT CAC GAT AAA GTC GTA 4268
Glu Trp Glu Lys Met Tyr Arg Glu Arg Trp Ser His Asp Lys Val Val
30 35 40

CGT ACA ACG CAT GGC GTG AAC TGT ACA GGT TCA TGT TCT TGG AAA GTA 4316
Arg Thr Thr His Gly Val Asn Cys Thr Gly Ser Cys Ser Trp Lys Val
45 50 55

TTT GTA AAA AAT GGG GTT ATC ACT TGG GAG AAC CAA CAA ACA GAT TAT 4364
Phe Val Lys Asn Gly Val Ile Thr Trp Glu Asn Gln Gln Thr Asp Tyr
60 65 70 75

CCA AGC TGC GGA CCG GAT ATG CCT GAG TTT GAA CCG CGC GGA TGT CCA 4412
Pro Ser Cys Gly Pro Asp Met Pro Glu Phe Glu Pro Arg Gly Cys Pro
80 85 90

CGT GGG GCG TCA TTT TCT TGG TAT GAA TAT AGT CCG CTT CGC GTA CGT 4460
Arg Gly Ala Ser Phe Ser Trp Tyr Glu Tyr Ser Pro Leu Arg Val Arg
95 100 105

TAC CCT TAT ATG CGC GGC AAG TTA TGG GAA CTT TGG ACC ACA GCA CTA     4508
Tyr Pro Tyr Met Arg Gly Lys Leu Trp Glu Leu Trp Thr Thr Ala Leu
        110                 115                 120

GAA GAA CAT GAA GGA AAC AAC ATT GCT GCA TGG GCT TCA ATC GTA GAA     4556
Glu Glu His Glu Gly Asn Asn Ile Ala Ala Trp Ala Ser Ile Val Glu
    125                 130                 135

GAT CCT GAA AAG GCA AAG ATT TAT AAA AAT GCA CGC GGT AAA GGC GGA     4604
Asp Pro Glu Lys Ala Lys Ile Tyr Lys Asn Ala Arg Gly Lys Gly Gly
140                 145                 150                 155

CAC ATG CGT GTG TCT TGG AAA GAT GTA TTG CAG CTG ATT GCA GCA CAA     4652
His Met Arg Val Ser Trp Lys Asp Val Leu Gln Leu Ile Ala Ala Gln
                160                 165                 170

TTA TTA TAC ACA ATT AAA AAA GAC GGT CCT GAC CGT ATC GCT GGT TTT     4700
Leu Leu Tyr Thr Ile Lys Lys Asp Gly Pro Asp Arg Ile Ala Gly Phe
            175                 180                 185

ACA CCT ATC CCA GCA ATG TCT ATG TTG AGT TAT GCA TCA GGT GCA CGA     4748
Thr Pro Ile Pro Ala Met Ser Met Leu Ser Tyr Ala Ser Gly Ala Arg
        190                 195                 200

TTC ATC AAT TTA ATG GGT GGA GAA ATG TTA AGT TTC TAT GAT TGG TAT     4796
Phe Ile Asn Leu Met Gly Gly Glu Met Leu Ser Phe Tyr Asp Trp Tyr
    205                 210                 215

GCA GAT TTA CCA CCA GCT TCA CCG CAA ATT TGG GGT GAA CAA ACA GAC     4844
Ala Asp Leu Pro Pro Ala Ser Pro Gln Ile Trp Gly Glu Gln Thr Asp
220                 225                 230                 235

GTT CCT GAA TCT AGT GAC TGG TAT AAC GCT AAC TAC ATT ATT ATG TGG     4892
Val Pro Glu Ser Ser Asp Trp Tyr Asn Ala Asn Tyr Ile Ile Met Trp
                240                 245                 250

GGC TCT AAC GTA CCA TTA ACA CGT ACA CCA GAT GCG CAC TTT ATG ACT     4940
Gly Ser Asn Val Pro Leu Thr Arg Thr Pro Asp Ala His Phe Met Thr
            255                 260                 265

GAA GTA CGT TAT AAA GGA ACA AAA GTA GTT TCT GTC GCA CCA GAT TAT     4988
Glu Val Arg Tyr Lys Gly Thr Lys Val Val Ser Val Ala Pro Asp Tyr
        270                 275                 280

GCT GAA AAC GTA AAA TTT GCA GAT AAC TGG TTA GCG CCG AAC CCT GGT     5036
Ala Glu Asn Val Lys Phe Ala Asp Asn Trp Leu Ala Pro Asn Pro Gly
    285                 290                 295

ACA GAT GCT GCG GTA GCA CAA GCG ATG ACA CAC GTT ATC TTA CAA AAA     5084
Thr Asp Ala Ala Val Ala Gln Ala Met Thr His Val Ile Leu Gln Lys
300                 305                 310                 315

TAT TAT GTA GAT GAA CCT TCA GAA ATG TTT GTC AAC TAT GCG AAA CAA     5132
Tyr Tyr Val Asp Glu Pro Ser Glu Met Phe Val Asn Tyr Ala Lys Gln
                320                 325                 330

TAT ACA GAT ATG CCA TTC TTA TTA ATG TTT GAT GAG CAT GAA GGC GGC     5180
Tyr Thr Asp Met Pro Phe Leu Leu Met Phe Asp Glu His Glu Gly Gly
            335                 340                 345

TAT AAA GCA GGA CGC TTT TTA CGT GCA AGC GAT TTA GGT ATG GAA TCT     5228
Tyr Lys Ala Gly Arg Phe Leu Arg Ala Ser Asp Leu Gly Met Glu Ser
        350                 355                 360

GAA AAT AAT GAA TGG AAA CCA GTT ATT GTT GAT AAC GAA TCC GGT GAA 5276
Glu Asn Asn Glu Trp Lys Pro Val Ile Val Asp Asn Glu Ser Gly Glu
365 370 375

ATC ATT GTT CCG AAC GGT ACT ATG GGA CAA CGT TGG GAA GAA GGC AAA 5324
Ile Ile Val Pro Asn Gly Thr Met Gly Gln Arg Trp Glu Glu Gly Lys
380 385 390 395

CAA TGG AAC TTG AAA TTA GAA AAT GAA GAT GGT TCT AAA GTA GAT CCT 5372
Gln Trp Asn Leu Lys Leu Glu Asn Glu Asp Gly Ser Lys Val Asp Pro
400 405 410

GCG TTG TCA GTT GCT GAA GGT GAA CAT GAC ATC GAT ATC ATG AAA TTC 5420
Ala Leu Ser Val Ala Glu Gly Glu His Asp Ile Asp Ile Met Lys Phe
415 420 425

CCT TAT TTC GAT AAT GCA GGT AAT GGT ATT TTC GAA CGT CCG ATT GCA 5468
Pro Tyr Phe Asp Asn Ala Gly Asn Gly Ile Phe Glu Arg Pro Ile Ala
430 435 440

TCT CGA GAA GTG ACA TTA GCA GAC GGT AAG AAA GTC AAA GTA GCA ACA 5516
Ser Arg Glu Val Thr Leu Ala Asp Gly Lys Lys Val Lys Val Ala Thr
445 450 455

GTT TAT GAC TTA ATG ACA AGC CAA TAT GGT ATC CGT CGT TTC AAC CAT 5564
Val Tyr Asp Leu Met Thr Ser Gln Tyr Gly Ile Arg Arg Phe Asn His
460 465 470 475

GAA CTT GAA GCT AAA GGT TTC GAT GAT GCT GAA ACA CCA TAT ACA CCA 5612
Glu Leu Glu Ala Lys Gly Phe Asp Asp Ala Glu Thr Pro Tyr Thr Pro
480 485 490

GCA TGG CAA GAA GCA GTA GCA GGT GTG AAG CAA GAT GTG ATT ACA CAA 5660
Ala Trp Gln Glu Ala Val Ala Gly Val Lys Gln Asp Val Ile Thr Gln
495 500 505

GTA GCA ACA GAA TTT GCA CAA AAT GCA ATC GAT ACA GGC GGT CGC TCA 5708
Val Ala Thr Glu Phe Ala Gln Asn Ala Ile Asp Thr Gly Gly Arg Ser
510 515 520

ATG ATT ATC ATG GGT GCC GGT ATT AAC CAC TGG TCC AAC TCA GAT ACG 5756
Met Ile Ile Met Gly Ala Gly Ile Asn His Trp Ser Asn Ser Asp Thr
525 530 535

ATT TAT CGT GCT GTC TTA AAC TTA GTT ATG CTT TGC GGC TGC CAA GGT 5804
Ile Tyr Arg Ala Val Leu Asn Leu Val Met Leu Cys Gly Cys Gln Gly
540 545 550 555

GTT AAC GGC GGA GGC TGG GCG CAC TAT GTA GGA CAA GAA AAA TGT CGT 5852
Val Asn Gly Gly Gly Trp Ala His Tyr Val Gly Gln Glu Lys Cys Arg
560 565 570

CCG ATT GAA GGA TGG AGC ACG ATT GCG TTC GCT AAA GAC TGG CAA GGT 5900
Pro Ile Glu Gly Trp Ser Thr Ile Ala Phe Ala Lys Asp Trp Gln Gly
575 580 585

CCG CCG CGT CTG CAA AAT GGT ACA AGC TGG TTC TAT TTC ACT ACG GAT 5948
Pro Pro Arg Leu Gln Asn Gly Thr Ser Trp Phe Tyr Phe Thr Thr Asp
590 595 600

CAA TGG AAA TAT GAA GAA TCA GGT GTT GAT AAA TTA GCT TCA CCG CTT 5996
Gln Trp Lys Tyr Glu Glu Ser Gly Val Asp Lys Leu Ala Ser Pro Leu
605 610 615

GCA GGT GAA CTT AAA CAT AAA CAC CCT GCA GAT TAT AAC GTT ACT GCA 6044
Ala Gly Glu Leu Lys His Lys His Pro Ala Asp Tyr Asn Val Thr Ala
620 625 630 635

GCA CGC TTA GGT TGG CTG CCA TCT TAT CCG CAA TTT GAT ACA AAT AGT 6092
Ala Arg Leu Gly Trp Leu Pro Ser Tyr Pro Gln Phe Asp Thr Asn Ser
640 645 650

TTG AGA TTT GGA GAA GAT GCG AAA GAA GCG GGA GAA TTC ACA AAT GAA 6140
Leu Arg Phe Gly Glu Asp Ala Lys Glu Ala Gly Glu Phe Thr Asn Glu
655 660 665

GAA GTA TTG AAA CGT GCC GTA GAA TCT GTA AAA AGT CGT GAA ACT AAA 6188
Glu Val Leu Lys Arg Ala Val Glu Ser Val Lys Ser Arg Glu Thr Lys
670 675 680

TTT GCG GTT GAA GAT CCA GAT TTA CGT ACG AAC CAT CCG AAA TCA CTA 6236
Phe Ala Val Glu Asp Pro Asp Leu Arg Thr Asn His Pro Lys Ser Leu
685 690 695

TTC ATT TGG CGT TCT AAC TTG TTA TCA AGT TCT GCT AAA GGT CAA GAG 6284
Phe Ile Trp Arg Ser Asn Leu Leu Ser Ser Ser Ala Lys Gly Gln Glu
700 705 710 715

TAT TTC ATG AAA CAT ATG TTA GGT ACA AGT TCT GGA TTA TTA GCT GAA 6332
Tyr Phe Met Lys His Met Leu Gly Thr Ser Ser Gly Leu Leu Ala Glu
720 725 730

CCG AAT GAA GAA GAC AAG CCA GAA GAA ATG ATT TGG CGT GAT GAT GTA 6380
Pro Asn Glu Glu Asp Lys Pro Glu Glu Met Ile Trp Arg Asp Asp Val
735 740 745

CAA GGT AAA TTA GAT TTA ATT GTT GCA CTA GAT TTC CGA ATG ACT TCA 6428
Gln Gly Lys Leu Asp Leu Ile Val Ala Leu Asp Phe Arg Met Thr Ser
750 755 760

ACG CCG CTT AAA GCA GAT ATC GTT TTA CCT GCT GCG ACA TGG TAT GAA 6476
Thr Pro Leu Lys Ala Asp Ile Val Leu Pro Ala Ala Thr Trp Tyr Glu
765 770 775

AAA CAT GAC TTA TCA TCA ACA GAC ATG CAT CCA TTC GTA CAT CCA TTC 6524
Lys His Asp Leu Ser Ser Thr Asp Met His Pro Phe Val His Pro Phe
780 785 790 795

AAT CCA GCA ATT GAT CCA TTA TGG GAG TCT CGT TCT GAC TGG GAT ATT 6572
Asn Pro Ala Ile Asp Pro Leu Trp Glu Ser Arg Ser Asp Trp Asp Ile
800 805 810

TTC AAA TCA TTG AGT CAG ACA GTA TCT GAA ATG GCA AAA GTT TAT ATG 6620
Phe Lys Ser Leu Ser Gln Thr Val Ser Glu Met Ala Lys Val Tyr Met
815 820 825

CCA GGT ACT TAC AAA GAT GTG GTG ACA TCA CCA TTA GCA CAT GAT TCA 6668
Pro Gly Thr Tyr Lys Asp Val Val Thr Ser Pro Leu Ala His Asp Ser
830 835 840

AAA CAA GAA ATT GCC ACA CCT TAC GGC AAA GTG AAA GAC TGG ACA AAA 6716
Lys Gln Glu Ile Ala Thr Pro Tyr Gly Lys Val Lys Asp Trp Thr Lys
845 850 855

GGT GAA GTT GAA GCG ATT CCT GGA AAA ACA ATG CCG GGC TTT GCA GTA 6764
Gly Glu Val Glu Ala Ile Pro Gly Lys Thr Met Pro Gly Phe Ala Val
860 865 870 875

GTA GAT CGT GAT TAT ACC CTT ATT TAT GAC AAA TAT GTC ACT GCT GGA 6812
Val Asp Arg Asp Tyr Thr Leu Ile Tyr Asp Lys Tyr Val Thr Ala Gly
880 885 890

CCT TTA TTA GAA ACA GCA AAA TTA GGT GCA CAT GGT GTC AGC TTC CCA 6860
Pro Leu Leu Glu Thr Ala Lys Leu Gly Ala His Gly Val Ser Phe Pro
895 900 905

GTT AAA AAA CAA TAT GAA GAA GCA AAA GAT ATT GTA GGT ACT TGG AGT 6908
Val Lys Lys Gln Tyr Glu Glu Ala Lys Asp Ile Val Gly Thr Trp Ser
910 915 920

GAC GGC ACA ATT AAA GAT GGT CGT CCA AGA ATT GAT ACT GCA AGA CGT 6956
Asp Gly Thr Ile Lys Asp Gly Arg Pro Arg Ile Asp Thr Ala Arg Arg
925 930 935

GCA GCA GAT GTT GTT TTA AAT ATT TCT TCA GCA ACA AAC GGT GAG TTA 7004
Ala Ala Asp Val Val Leu Asn Ile Ser Ser Ala Thr Asn Gly Glu Leu
940 945 950 955

TCT CAA GCG TCT TAT AAA GAT TTA GAA GAA AAA ACT GGA ATG CCT TTA 7052
Ser Gln Ala Ser Tyr Lys Asp Leu Glu Glu Lys Thr Gly Met Pro Leu
960 965 970

ACA GAT ATT TCG AGT GAC CGA AGT GCT GAA AAA ATT ACG TTT ATG AAT 7100
Thr Asp Ile Ser Ser Asp Arg Ser Ala Glu Lys Ile Thr Phe Met Asn
975 980 985

ATA ACA TCT CAA CCA CGG GAG GTT ATT CCG ACA GCA GTA TTC TCA GGT 7148
Ile Thr Ser Gln Pro Arg Glu Val Ile Pro Thr Ala Val Phe Ser Gly
990 995 1000

TCA AAC AAA GGG AAC AAA CGT TAT TCA CCA TTT ACA ACA AAT ATT GAA 7196
Ser Asn Lys Gly Asn Lys Arg Tyr Ser Pro Phe Thr Thr Asn Ile Glu
1005 1010 1015

CGT CTC GTA CCA TTC CGT ACA TTA ACA GGA CGT CAA AGT TAT TAT TTA 7244
Arg Leu Val Pro Phe Arg Thr Leu Thr Gly Arg Gln Ser Tyr Tyr Leu
1020 1025 1030 1035

GAT CAT GAA ATC TTC CAG CAA TTC GGT GAA AGC TTG CCG GTA TAC AAA 7292
Asp His Glu Ile Phe Gln Gln Phe Gly Glu Ser Leu Pro Val Tyr Lys
1040 1045 1050

CCA ACT TTA CCG CCA ATG GTA TTT GGT ACA AAA GAT AAA CAA GTT ATC 7340
Pro Thr Leu Pro Pro Met Val Phe Gly Thr Lys Asp Lys Gln Val Ile
1055 1060 1065

GGC GGA CAA GAC AAT TTA GTT TTA AGA TAT TTA ACA CCG CAT GGT AAA 7388
Gly Gly Gln Asp Asn Leu Val Leu Arg Tyr Leu Thr Pro His Gly Lys
1070 1075 1080

TGG AAT ATC CAC TCT ACT TAC CAA GAT AAC CAG CAC ATG TTG ACA CTA 7436
Trp Asn Ile His Ser Thr Tyr Gln Asp Asn Gln His Met Leu Thr Leu
1085 1090 1095

TTC CGT GGT GGA CCA ACA GTA TGG ATT GCT AAA GAA GAT GCA GAA GCA 7484
Phe Arg Gly Gly Pro Thr Val Trp Ile Ala Lys Glu Asp Ala Glu Ala
1100 1105 1110 1115

CAC GAT ATT CAC GAT AAT GAT TGG TTA GAA GTT TAT AAC CGT AAC GGG 7532
His Asp Ile His Asp Asn Asp Trp Leu Glu Val Tyr Asn Arg Asn Gly
1120 1125 1130

GTT GTA ACA GCG AGA GCC GTT ACT TCA CAC AGA ATG CCT AAA GGG ACA 7580
Val Val Thr Ala Arg Ala Val Thr Ser His Arg Met Pro Lys Gly Thr
1135 1140 1145

ATG TTT ATG TAT CAC GCA CAA GAT AAA CAC ATT CAA ACA CCA GGA TCT 7628
Met Phe Met Tyr His Ala Gln Asp Lys His Ile Gln Thr Pro Gly Ser
1150 1155 1160

GAA ATT ACA GGA TTA CGT GGC GGT TCA CAT AAC GCG CCA ACT AGA ATC 7676
Glu Ile Thr Gly Leu Arg Gly Gly Ser His Asn Ala Pro Thr Arg Ile
1165 1170 1175

CAT TTA AAA CCG ACA CAA TTA GTC GGA GGG TAT GCG CAA ATC AGT TAC 7724
His Leu Lys Pro Thr Gln Leu Val Gly Gly Tyr Ala Gln Ile Ser Tyr
1180 1185 1190 1195

AGC TTC AAC TAT TAC GGT CCA ATT GGA AAC CAA AGA GAT GTT TAT GTA 7772
Ser Phe Asn Tyr Tyr Gly Pro Ile Gly Asn Gln Arg Asp Val Tyr Val
1200 1205 1210

GCA GTG AGA AAG ATG AAG GAG GTA GAT TGG CTT GAA GAT TAAAGCACAA 7821
Ala Val Arg Lys Met Lys Glu Val Asp Trp Leu Glu Asp
1215 1220

GTAGCCATGG TATTGAACTT GGATAAGTGT ATCGGATGCC ATACATGCAG TGTGACATGT 7881

AAAAATACTT GGACAAACCG TCCAGGTGCA GAATATATTT GGTTCAATAA TGTAGAAACA 7941

AAACCAGGTG TCGGTTATCC GAAACGTTGG GAAGACCAAG AACACTATAA AGGTGGATGG 8001

ACATTAAATA AAAAAGGAAA ATTAGATTTG AAAGCAGGCA GCAGATGGTC AAAAATTGCT 8061

TTCGGTAAGA TTTTCTATAA TCCAGATATG CCGGTCATCA AAGACTATTA TGAACCATGG 8121

ACTTATAACT ATGAACATTT AACAACAGCT AAAGCCAGCA AACATTCTCC TGTCGCAACT 8181

GCACATTCAG TTATTTCTGG CGATAAAATT GATTTGCACT GGGGACCTAA CTGGGAAGAT 8241

GATTTAGCAG GCGGTCATGT TACAGGACCT GAAGATCCTA ACATTCAACG TATTGAAGAA 8301

AAAATTCAAT TTGATTTTGA CCAAACATTT ATGATGTATT TACCTCGTTT ATGCGAACAT 8361

TGCTTAAATC CTAGTTGTGT AGCATCTTGT CCTTCTGGTG CAATGTATAA ACGTGATGAA 8421

GACGGTATCG TCTTAGTAGA CCAAGATGCT TGTCGCGGGT GGCGTTATTG TATGACAGGT 8481

TGCCCTTATA AAAAAGTGTA CTTCAACTGG AAAACAAATA AAGCTGAAAA ATGTACATTC 8541

TGTTTCCCAA GAGTTGAAGC AGGGATGCCG ACAGTTTGTT CTGAAACTTG TACAGGACGT 8601

ATGCGTTATT TAGGTGTTTT ATTATATGAT GCTGATCGTG TACATGAAGT TGCTTCTTGT 8661

GAAAATGAAC AGGATTTATA CGAAAAACAA TGTGAATTGT TCTTGAATCC TTATGATGAA 8721

GATGTGATTG AACAAGCGAA AAAAGATGGC ATCACAATGG ATTGGATTGA AGCAGCACAA 8781

AACTCTCCAG TCTACAAATT AGCAATTGAA TACAAACTTG CATTCCCATT GCACCCTGAA 8841

TATCGTACTT TGCCAATGGT TTGGTATTGC CCGCCATTAA GTCCGATTAT GAACTACTTT 8901

GAAGGTAAAG ATTCTATTAA AAATCCTGAT GCAATCTTCC CTGCCATTGA AGAAATGCGT 8961

TTGCCGATTG AATACCTTGC CAGCTTATTG ACTGCAGGTG ATACAAAATC TGTGAAAGAA 9021

GCATTACAAC GCATGGCGAT GATGAGAAGT TATATGCGTG CACAAGTTAC AGGTAAGGAA 9081

TTTGATACAG ACCGTTTAGC ACGTCTTGGT TTAACTGAGC GTCAAACAAA AGATATGTAT 9141

CGCTTATTAG CAATTGCTAA GTACGAAGAT CGTTTCGTGA TTCCGACTTC TCATAAAGAA 9201

GAATATATGG ACACTTATGC AGCACAAGGC AGTCAAGGTT ATACATTCGG TGATCAAATG 9261

AATGGAACTG GCAGCGGAAG CGGTTGTGAA GGCTGTGGTG CACCAGTTGC TATGAAACCG 9321

GGACAAACTG GACAAGAAGC TTACAACGAA AGCTTTTATG GAGGGATTTT CCGTGATTAA 9381

CTTAGATAAC TTTTATAAAT ATCAAGAATC AATCGGTTAT TTCGCGCAAC AATTGAACTT 9441

TCCAGAAAAA TTAACATTCC ATCCTAAAAC TTTTAATGAT ATTTTTGATA CAAACCACCC 9501

AGCATACGCT GAGGTGGTAG CGTATCGTGA TCAAATTCAA AGTTATACAT TAACAGAAAT 9561

CAAACAACTT TATACAGATA CTTTTGATTT TAATAAACAA GCACCGCTTT ATATGACTTA 9621

CAATAAATTT GATACACAAA AAGAACGTGG TCAAATGTTG GCTAAATTAA AAGTACTTTA 9681

TGAAATGTTC GGATTAGAGA TGGTAGATAA TGAATTATCA GATTACCTAC CTTTGATGCT 9741

TGAATTTTTA TATGCAGCAC AATGGCAAAA TGATGATCGT GCAGAAGGCA ATATCGAATT 9801

CATCATTATG GTTATTGAAG ATGGTTCTTA TGCGATGCTC CAACAACTTA GAGAGATAGA 9861

TAGTCCGTAT GCCCCTTTAA TTAAGGGTTT AAGAGAGACT TTAAAATTAT GCTTACAACC 9921

GAGCGAAGAG GTGAGCCATC ATGCTTAATC AGTTTTTATG GGTGATTTAT CCATACTTGT 9981

GTGTTGCTAT TTTTATCATT GGCCATATTG CACGTTATCG ATATGATCAA TTTTCATGGA 10041

CAGCCAAATC AAGTGAATTT ATTGAAAAGA AACGTTTGAA GTGGGGAAGC TTATTATTTC 10101

ATTTAGGTAT TATTCCTGTT GCAGCAGGTC ACTTTGTCGG ACTCGTCATT CCAGCTGCTT 10161

GGTTAGAAGG TATGGGTGTT AATAATCATA TTTATCATAT TGGTGCAGTT TATATCGGCA 10221

GCTTATTTGG TTTTATGGTC TTAATTGGTA TGATTTTATT AACATATCGC CGTGTATCTA 10281

TTAAAAACAT CAGACGTTTA AGTTCTGCTT CTGACATGGT AGTCAACTTC TTTTTACTTG 10341

TGATTATCTG TATGGGATTA TATTCTACAC TTGTAACCAA TGCGATATCA CCAGAATTCG 10401

ATTACAGACA AACCATTTCT ATTTGGTTCA GACACTTGTT TACATTTAAT CCGAATGCAA 10461

GTTTGATGAC AAATGTGCCT TGGTCATTTA AACTGCATAT ATTCTTAGGT TTTTCTATTT 10521

TTGCATTATG GCCATTCACA CGTTTAGTTC ACGTGTGGAG TGTTCCATTA TCTTATGCAA 10581

ACAGAAGTTA TATTATTTAT CGTAAAAATA AAGCAAGAAC ATAATAGTGA GGGGGAGAGA 10641

TGTTGAATAG TGTAATTGCT TCAGATTAC 10670

(2) INFORMATION FOR SEQ ID NO: 2:
(i) SEQUENCE CHARACTERISTICS:
(A) LENGTH: 801 amino acids
(B) TYPE: amino acid

(D) TOPOLOGY: linear
(ii) MOLECULE TYPE: protein
(xi) SEQUENCE DESCRIPTION: SEQ ID NO: 2:

Met Ser Lys Gln Arg Leu Val Met Ile Gly Asn Gly Met Ala Gly Val
1               5                   10                  15

Arg Thr Ile Glu Glu Ile Ile Asp Arg Asp Pro Asp Lys Phe Asp Ile
            20                  25                  30

Thr Ile Ile Gly Lys Glu Pro Tyr Pro Asn Tyr Asn Arg Ile Met Leu
        35                  40                  45

Ser Asn Ile Leu Gln Lys Lys Met Thr Val Glu Glu Thr Ile Met Asn
    50                  55                  60

Pro Tyr Ser Trp Tyr Glu Glu Asn Gly Ile Ala Leu Ile Thr Gly Glu
65                  70                  75                  80

Lys Ala Thr Arg Val Asn Arg Glu Lys His Ile Val Glu Thr Glu Lys
                85                  90                  95

Gly Arg Glu Ile Pro Tyr Asp Ile Cys Ile Phe Ala Thr Gly Ser Lys
            100                 105                 110

Ala Phe Val Leu Pro Ile Pro Gly His Glu Leu Pro Ser Val Ile Gly
        115                 120                 125

Trp Arg Thr Ile Glu Asp Thr Glu Arg Met Ile Glu Ile Ala Gln Asp
    130                 135                 140

Lys Lys Glu Ala Thr Val Ile Gly Gly Gly Leu Leu Gly Leu Glu Cys
145                 150                 155                 160

Ala Arg Gly Leu Leu Asp Gln Gly Met Asn Val Thr Val Val His Leu
                165                 170                 175

Ala Glu Trp Leu Met Glu Met Gln Leu Asp Arg Gln Ala Gly Asp Met
            180                 185                 190

Leu Arg Glu Asp Leu Glu Lys Gln Gly Met Lys Phe Glu Leu Gly Ala
        195                 200                 205

Asn Thr Lys Glu Ile Ile Gly Asn Gln Asp Val Glu Ala Ile Arg Leu
    210                 215                 220

Ala Asp Gly Arg Asp Leu Pro Ala Asp Leu Val Val Met Ala Val Gly
225                 230                 235                 240

Ile Lys Pro Phe Thr Gln Glu Ala Ile Asp Ser Gly Ile Asp Val Glu
                245                 250                 255

Arg Gly Ile Val Val Asp Asp Phe Met Arg Thr Ser Asp Pro Glu Ile
            260                 265                 270

Tyr Ala Val Gly Glu Cys Ala Gln His Asn Cys Lys Val Tyr Gly Leu
        275                 280                 285

Val Ala Pro Leu Tyr Glu Gln Gly Lys Val Leu Ala Asp His Leu Thr
    290                 295                 300

Gly Arg Pro Thr Glu Gly Tyr Gln Gly Ser Thr Thr Phe Thr Ser Leu
305                 310                 315                 320

Lys Val Ser Gly Cys Asp Leu Tyr Ser Ala Gly Gln Ile Gln Glu Asp
                325                 330                 335

Asp Glu Val Glu Gly Val Glu Ile Tyr Asn Ser His Asp His Val Tyr
            340                 345                 350

Lys Lys Ile Phe Leu Lys Glu Glu Arg Val Val Gly Ala Val Leu Tyr
        355                 360                 365

Gly Asp Thr Asp Asp Gly Thr Arg Phe Phe Asn Met Met Lys Lys Arg
    370                 375                 380

Glu Pro Ile Glu Asp Tyr Thr Leu Val Ser Leu Leu Gln Lys Val Gly
385                 390                 395                 400

Glu Glu Ala Val Ser Thr Val Ala Asp Met Asp Asp Ser Glu Thr Ile
                405                 410                 415

Cys Gly Cys Asn Gly Val Ser Lys Gly Lys Ile Val Ser Ala Ile Val
            420                 425                 430

Glu Asp Gly Leu Thr Ser Val Ala Glu Val Thr Lys Ala Thr Lys Ala
        435                 440                 445

Gly Asn Ser Cys Gly Lys Cys Lys Pro Gln Ile Gly Glu Leu Leu Glu
    450                 455                 460

His Thr Leu Gly Gly Ala Phe Val Ala Ser Lys Pro Ala Gly Ile Cys
465                 470                 475                 480

Asp Cys Thr Glu Leu Thr Arg Asp Gln Ile Val Thr Gln Ile Arg Ala
                485                 490                 495

Lys Gly Leu Lys Thr Ser Lys Glu Val Arg His Val Leu Asp Trp Lys
            500                 505                 510

Asn Lys Gly Gly Cys Pro Lys Cys Arg Pro Ala Ile Asn Tyr Tyr Leu
        515                 520                 525

Asn Met Val Tyr Pro His Glu His Glu Asp Glu Lys Ala Ser Arg Phe
    530                 535                 540

Ala Asn Glu Arg Tyr His Ala Asn Ile Gln Asn Asp Gly Thr Phe Ser
545                 550                 555                 560

Val Ile Pro Gln Met Arg Gly Gly Val Thr Asn Pro Asp Gln Leu Ile
                565                 570                 575

Arg Leu Gly Glu Val Ala Lys Lys Tyr Asp Val Pro Leu Val Lys Val
            580                 585                 590

Thr Gly Ser Gln Arg Ile Gly Leu Tyr Gly Ile Ser Lys Glu Asp Leu
        595                 600                 605

Pro Lys Val Trp Lys Asp Leu Gly Met Arg Ser Ala Ser Ala Tyr Ala
    610                 615                 620

Lys Lys Thr Arg Ser Val Lys Ser Cys Val Gly Lys Glu Phe Cys Arg
625                 630                 635                 640

Phe Gly Thr Gln Tyr Thr Thr Arg Leu Gly Ile Arg Leu Glu Glu Thr
                645                 650                 655

Phe Glu Tyr Ile Asp Thr Pro His Lys Phe Lys Met Gly Val Ser Gly
            660                 665                 670

Cys Pro Arg Ser Cys Val Glu Ser Gly Val Lys Asp Phe Gly Val Ile
        675                 680                 685

Gly Val Glu Asn Gly Phe Gln Ile Tyr Ile Gly Gly Asn Gly Gly Thr
    690                 695                 700

Glu Val Thr Lys Gly Gln Phe Leu Thr Thr Val Glu Thr Glu Glu Asp
705                 710                 715                 720

Val Ile Gln Leu Cys Gly Ala Tyr Met Gln Tyr Tyr Arg Glu Thr Gly
                725                 730                 735

Ile Tyr Ala Glu Arg Thr Ala Pro Trp Leu Glu Arg Met Gly Phe Glu
            740                 745                 750

Asn Val Lys Glu Tyr Val Leu Asp Pro Glu Arg Gln Lys Ala Leu Tyr
        755                 760                 765

Asp Arg Ile Met Asp Ala Lys Arg Ala Val Ala Glu Glu Pro Trp Ser
    770                 775                 780

Glu Ile Val Asn Asp Gln Ala Asp Gln Lys Ile Phe Lys Val Glu Lys
785                 790                 795                 800

Val

(2) INFORMATION FOR SEQ ID NO: 3:
     (i) SEQUENCE CHARACTERISTICS:
          (A) LENGTH: 104 amino acids
          (B) TYPE: amino acid
          (D) TOPOLOGY: linear
    (ii) MOLECULE TYPE: protein
    (xi) SEQUENCE DESCRIPTION: SEQ ID NO: 3:

Met Arg Ala Ile Glu Lys Val Arg Val Ala Ala Leu Glu Asp Leu Thr
  1               5                  10                  15

Pro Leu Ile Gly Thr Lys Val Leu Val Arg Asp Lys Glu Ile Gly Leu
             20                  25                  30

Phe Leu Thr Glu Asp Gly Arg Ile Phe Ala Val Asn Asn Val Cys Pro
         35                  40                  45

His Lys Gln Gly Pro Leu Ser Glu Gly Thr Val Ser Glu His Ser Val
     50                  55                  60

Phe Cys Pro Leu His Asp Gln Gln Ile Asp Leu Glu Thr Gly Leu Val
 65                  70                  75                  80

Gln Glu Pro Asp Thr Gly Cys Val Glu Thr Tyr Glu Val Glu Ile Ile
                 85                  90                  95

Asp Gly Asp Val Tyr Leu Cys Leu
            100

(2) INFORMATION FOR SEQ ID NO: 4:

(i) SEQUENCE CHARACTERISTICS:
(A) LENGTH: 1224 amino acids
(B) TYPE: amino acid
(D) TOPOLOGY: linear
(ii) MOLECULE TYPE: protein
(xi) SEQUENCE DESCRIPTION: SEQ ID NO: 4:

Met Ala Lys Phe Gly Met Asn Phe Phe Lys Pro Thr Glu Lys Phe Asn
1               5                   10                  15

Gly Asn Trp Ser Val Leu Thr Asp Lys Ser Arg Glu Trp Glu Lys Met
            20                  25                  30

Tyr Arg Glu Arg Trp Ser His Asp Lys Val Val Arg Thr Thr His Gly
        35                  40                  45

Val Asn Cys Thr Gly Ser Cys Ser Trp Lys Val Phe Val Lys Asn Gly
    50                  55                  60

Val Ile Thr Trp Glu Asn Gln Gln Thr Asp Tyr Pro Ser Cys Gly Pro
65                  70                  75                  80

Asp Met Pro Glu Phe Glu Pro Arg Gly Cys Pro Arg Gly Ala Ser Phe
                85                  90                  95

Ser Trp Tyr Glu Tyr Ser Pro Leu Arg Val Arg Tyr Pro Tyr Met Arg
            100                 105                 110

Gly Lys Leu Trp Glu Leu Trp Thr Thr Ala Leu Glu Glu His Glu Gly
        115                 120                 125

Asn Asn Ile Ala Ala Trp Ala Ser Ile Val Glu Asp Pro Glu Lys Ala
    130                 135                 140

Lys Ile Tyr Lys Asn Ala Arg Gly Lys Gly Gly His Met Arg Val Ser
145                 150                 155                 160

Trp Lys Asp Val Leu Gln Leu Ile Ala Ala Gln Leu Leu Tyr Thr Ile
                165                 170                 175

Lys Lys Asp Gly Pro Asp Arg Ile Ala Gly Phe Thr Pro Ile Pro Ala
            180                 185                 190

Met Ser Met Leu Ser Tyr Ala Ser Gly Ala Arg Phe Ile Asn Leu Met
        195                 200                 205

Gly Gly Glu Met Leu Ser Phe Tyr Asp Trp Tyr Ala Asp Leu Pro Pro
    210                 215                 220

Ala Ser Pro Gln Ile Trp Gly Glu Gln Thr Asp Val Pro Glu Ser Ser
225                 230                 235                 240

Asp Trp Tyr Asn Ala Asn Tyr Ile Ile Met Trp Gly Ser Asn Val Pro
                245                 250                 255

Leu Thr Arg Thr Pro Asp Ala His Phe Met Thr Glu Val Arg Tyr Lys
            260                 265                 270

Gly Thr Lys Val Val Ser Val Ala Pro Asp Tyr Ala Glu Asn Val Lys
        275                 280                 285

Phe Ala Asp Asn Trp Leu Ala Pro Asn Pro Gly Thr Asp Ala Ala Val
    290                 295                 300

Ala Gln Ala Met Thr His Val Ile Leu Gln Lys Tyr Tyr Val Asp Glu
305                 310                 315                 320

Pro Ser Glu Met Phe Val Asn Tyr Ala Lys Gln Tyr Thr Asp Met Pro
                325                 330                 335
Phe Leu Leu Met Phe Asp Glu His Glu Gly Gly Tyr Lys Ala Gly Arg
            340                 345                 350

Phe Leu Arg Ala Ser Asp Leu Gly Met Glu Ser Glu Asn Asn Glu Trp
        355                 360                 365

Lys Pro Val Ile Val Asp Asn Glu Ser Gly Glu Ile Ile Val Pro Asn
    370                 375                 380

Gly Thr Met Gly Gln Arg Trp Glu Glu Gly Lys Gln Trp Asn Leu Lys
385                 390                 395                 400

Leu Glu Asn Glu Asp Gly Ser Lys Val Asp Pro Ala Leu Ser Val Ala
                405                 410                 415

Glu Gly Glu His Asp Ile Asp Ile Met Lys Phe Pro Tyr Phe Asp Asn
            420                 425                 430

Ala Gly Asn Gly Ile Phe Glu Arg Pro Ile Ala Ser Arg Glu Val Thr
        435                 440                 445

Leu Ala Asp Gly Lys Lys Val Lys Val Ala Thr Val Tyr Asp Leu Met
    450                 455                 460

Thr Ser Gln Tyr Gly Ile Arg Arg Phe Asn His Glu Leu Glu Ala Lys
465                 470                 475                 480

Gly Phe Asp Asp Ala Glu Thr Pro Tyr Thr Pro Ala Trp Gln Glu Ala
                485                 490                 495

Val Ala Gly Val Lys Gln Asp Val Ile Thr Gln Val Ala Thr Glu Phe
            500                 505                 510

Ala Gln Asn Ala Ile Asp Thr Gly Gly Arg Ser Met Ile Ile Met Gly
        515                 520                 525

Ala Gly Ile Asn His Trp Ser Asn Ser Asp Thr Ile Tyr Arg Ala Val
    530                 535                 540

Leu Asn Leu Val Met Leu Cys Gly Cys Gln Gly Val Asn Gly Gly Gly
545                 550                 555                 560

Trp Ala His Tyr Val Gly Gln Glu Lys Cys Arg Pro Ile Glu Gly Trp
                565                 570                 575

Ser Thr Ile Ala Phe Ala Lys Asp Trp Gln Gly Pro Pro Arg Leu Gln
            580                 585                 590

Asn Gly Thr Ser Trp Phe Tyr Phe Thr Thr Asp Gln Trp Lys Tyr Glu
        595                 600                 605

Glu Ser Gly Val Asp Lys Leu Ala Ser Pro Leu Ala Gly Glu Leu Lys
    610                 615                 620

His Lys His Pro Ala Asp Tyr Asn Val Thr Ala Ala Arg Leu Gly Trp
625                 630                 635                 640

Leu Pro Ser Tyr Pro Gln Phe Asp Thr Asn Ser Leu Arg Phe Gly Glu
                645                 650                 655

Asp Ala Lys Glu Ala Gly Glu Phe Thr Asn Glu Glu Val Leu Lys Arg
            660                 665                 670

Ala Val Glu Ser Val Lys Ser Arg Glu Thr Lys Phe Ala Val Glu Asp
        675                 680                 685

Pro Asp Leu Arg Thr Asn His Pro Lys Ser Leu Phe Ile Trp Arg Ser
    690                 695                 700

Asn Leu Leu Ser Ser Ser Ala Lys Gly Gln Glu Tyr Phe Met Lys His
705                 710                 715                 720

Met Leu Gly Thr Ser Ser Gly Leu Leu Ala Glu Pro Asn Glu Glu Asp
                725                 730                 735

Lys Pro Glu Glu Met Ile Trp Arg Asp Asp Val Gln Gly Lys Leu Asp
            740                 745                 750

Leu Ile Val Ala Leu Asp Phe Arg Met Thr Ser Thr Pro Leu Lys Ala
        755                 760                 765

Asp Ile Val Leu Pro Ala Ala Thr Trp Tyr Glu Lys His Asp Leu Ser
    770                 775                 780

Ser Thr Asp Met His Pro Phe Val His Pro Phe Asn Pro Ala Ile Asp
785                 790                 795                 800

Pro Leu Trp Glu Ser Arg Ser Asp Trp Asp Ile Phe Lys Ser Leu Ser
                805                 810                 815

Gln Thr Val Ser Glu Met Ala Lys Val Tyr Met Pro Gly Thr Tyr Lys
            820                 825                 830

Asp Val Val Thr Ser Pro Leu Ala His Asp Ser Lys Gln Glu Ile Ala
        835                 840                 845

Thr Pro Tyr Gly Lys Val Lys Asp Trp Thr Lys Gly Glu Val Glu Ala
    850                 855                 860

Ile Pro Gly Lys Thr Met Pro Gly Phe Ala Val Val Asp Arg Asp Tyr
865                 870                 875                 880

Thr Leu Ile Tyr Asp Lys Tyr Val Thr Ala Gly Pro Leu Leu Glu Thr
                885                 890                 895

Ala Lys Leu Gly Ala His Gly Val Ser Phe Pro Val Lys Lys Gln Tyr
            900                 905                 910

Glu Glu Ala Lys Asp Ile Val Gly Thr Trp Ser Asp Gly Thr Ile Lys
        915                 920                 925

Asp Gly Arg Pro Arg Ile Asp Thr Ala Arg Arg Ala Ala Asp Val Val
    930                 935                 940

Leu Asn Ile Ser Ser Ala Thr Asn Gly Glu Leu Ser Gln Ala Ser Tyr
945                 950                 955                 960

Lys Asp Leu Glu Glu Lys Thr Gly Met Pro Leu Thr Asp Ile Ser Ser
                965                 970                 975

Asp Arg Ser Ala Glu Lys Ile Thr Phe Met Asn Ile Thr Ser Gln Pro
            980                 985                 990

Arg Glu Val Ile Pro Thr Ala Val Phe Ser Gly Ser Asn Lys Gly Asn
        995                 1000                1005

Lys Arg Tyr Ser Pro Phe Thr Thr Asn Ile Glu Arg Leu Val Pro Phe
    1010                1015                1020

Arg Thr Leu Thr Gly Arg Gln Ser Tyr Tyr Leu Asp His Glu Ile Phe
1025                1030                1035                1040

Gln Gln Phe Gly Glu Ser Leu Pro Val Tyr Lys Pro Thr Leu Pro Pro
                1045                1050                1055

Met Val Phe Gly Thr Lys Asp Lys Gln Val Ile Gly Gly Gln Asp Asn
            1060                1065                1070

Leu Val Leu Arg Tyr Leu Thr Pro His Gly Lys Trp Asn Ile His Ser
        1075                1080                1085

Thr Tyr Gln Asp Asn Gln His Met Leu Thr Leu Phe Arg Gly Gly Pro
    1090                1095                1100

Thr Val Trp Ile Ala Lys Glu Asp Ala Glu Ala His Asp Ile His Asp
1105                1110                1115                1120

Asn Asp Trp Leu Glu Val Tyr Asn Arg Asn Gly Val Val Thr Ala Arg
                1125                1130                1135

Ala Val Thr Ser His Arg Met Pro Lys Gly Thr Met Phe Met Tyr His
            1140                1145                1150

Ala Gln Asp Lys His Ile Gln Thr Pro Gly Ser Glu Ile Thr Gly Leu
        1155                1160                1165

Arg Gly Gly Ser His Asn Ala Pro Thr Arg Ile His Leu Lys Pro Thr
    1170                1175                1180

Gln Leu Val Gly Gly Tyr Ala Gln Ile Ser Tyr Ser Phe Asn Tyr Tyr
1185                1190                1195                1200

Gly Pro Ile Gly Asn Gln Arg Asp Val Tyr Val Ala Val Arg Lys Met
                1205                1210                1215

Lys Glu Val Asp Trp Leu Glu Asp
            1220

(2) INFORMATION FOR SEQ ID NO: 5:
    (i) SEQUENCE CHARACTERISTICS:
        (A) LENGTH: 297 amino acids
        (B) TYPE: amino acid
        (C) STRANDEDNESS: single
        (D) TOPOLOGY: linear
    (ii) MOLECULE TYPE: protein
    (ix) FEATURE:
        (A) NAME/KEY: Protein
        (B) LOCATION:1..297
        (D) OTHER INFORMATION:/note= "CysG protein encoded by
            nucleotide 2894-3844 of SEQ ID NO:1"
    (xi) SEQUENCE DESCRIPTION: SEQ ID NO: 5:

Met Ser Val Ser Pro Thr Gly His Val Tyr Leu Ile Gly Ala Gly Pro
1               5                   10                  15

Gly Asn Pro Asn Leu Leu Thr Arg Lys Ala Glu His Leu Ile Arg Thr
            20                  25                  30

Ala Asp Ile Ile Leu Tyr Asp Arg Leu Val Asn Pro Phe Ile Leu Gln
        35                  40                  45

Leu Ala Pro Pro Glu Ile Glu Ile Ile Asp Val Gly Lys Lys Pro Tyr
    50                  55                  60

Ser Arg His Ile Gln Gln Ser Glu Ile Asn Glu Val Leu Leu Asp Ala
65                  70                  75                  80

Ala Tyr Lys His Arg Asn Val Val Arg Leu Lys Gly Gly Asp Pro Ala
                85                  90                  95

Ile Phe Gly Arg Val His Glu Glu Val Asp Ile Leu Lys Glu His Asn
            100                 105                 110

Ile Pro Cys Glu Ile Val Pro Gly Ile Thr Thr Ala Ser Ala Ala Ala
        115                 120                 125

Ala Thr Leu Gly Val Gly Leu Thr Gln Glu Ile Asp Ile Ala Ser Leu
    130                 135                 140

Ile Asp Gly Gly Thr Leu Ala Ile Tyr Met Gly Ile Lys Arg Leu His
145                 150                 155                 160

Thr Ile Ile Thr Gln Ile Tyr Lys Gln Val Gly Lys Asp Tyr Pro Ile
                165                 170                 175

Cys Ala Val Phe Asp Val Ser Tyr Ala Thr Gln Lys Val Ile Thr Gly
            180                 185                 190

His Leu Ser Asp Ile Glu Lys Lys Val Gln Ala Asn Ala Pro Glu Asp
        195                 200                 205

Ala Pro Gly Ile Leu Leu Ile Gly Asp Ala Leu Glu Tyr Ala Asp Glu
    210                 215                 220

Asn Thr His Ile Gln Leu Glu Glu Thr Ser Lys Tyr Phe Gly Lys Glu
225                 230                 235                 240

Cys Ile Val Thr Gly Pro Arg Glu Ala Ala Ile Glu Lys Ala Phe Glu
                245                 250                 255

Ile Tyr Asp Ala Gly Gly Trp Cys Met Ile Asp Pro Asn Ser Asp Asp
            260                 265                 270

His Tyr His Gln Ser Gln Leu Asp Leu Ile Tyr Arg Tyr Met Gln Val
        275                 280                 285

Asn Asp Asn Thr Ile Ile Val Lys Ala
    290                 295

(2) INFORMATION FOR SEQ ID NO: 6:
    (i) SEQUENCE CHARACTERISTICS:
        (A) LENGTH: 525 amino acids
        (B) TYPE: amino acid
        (C) STRANDEDNESS: single
        (D) TOPOLOGY: linear

(ii) MOLECULE TYPE: protein
(ix) FEATURE:
(A) NAME/KEY: Protein
(B) LOCATION:1..525
(D) OTHER INFORMATION:/note= "NarH protein encoded by
nucleotide 7804-9378 of SEQ ID NO:1"
(xi) SEQUENCE DESCRIPTION: SEQ ID NO: 6:

Met Lys Ile Lys Ala Gln Val Ala Met Val Leu Asn Leu Asp Lys Cys
1               5                   10                  15

Ile Gly Cys His Thr Cys Ser Val Thr Cys Lys Asn Thr Trp Thr Asn
            20                  25                  30

Arg Pro Gly Ala Glu Tyr Ile Trp Phe Asn Asn Val Glu Thr Lys Pro
        35                  40                  45

Gly Val Gly Tyr Pro Lys Arg Trp Glu Asp Gln Glu His Tyr Lys Gly
    50                  55                  60

Gly Trp Thr Leu Asn Lys Lys Gly Lys Leu Asp Leu Lys Ala Gly Ser
65                  70                  75                  80

Arg Trp Ser Lys Ile Ala Phe Gly Lys Ile Phe Tyr Asn Pro Asp Met
                85                  90                  95

Pro Val Ile Lys Asp Tyr Tyr Glu Pro Trp Thr Tyr Asn Tyr Glu His
            100                 105                 110

Leu Thr Thr Ala Lys Ala Ser Lys His Ser Pro Val Ala Thr Ala His
        115                 120                 125

Ser Val Ile Ser Gly Asp Lys Ile Asp Leu His Trp Gly Pro Asn Trp
    130                 135                 140

Glu Asp Asp Leu Ala Gly Gly His Val Thr Gly Pro Glu Asp Pro Asn
145                 150                 155                 160

Ile Gln Arg Ile Glu Glu Lys Ile Gln Phe Asp Phe Asp Gln Thr Phe
                165                 170                 175

Met Met Tyr Leu Pro Arg Leu Cys Glu His Cys Leu Asn Pro Ser Cys
            180                 185                 190

Val Ala Ser Cys Pro Ser Gly Ala Met Tyr Lys Arg Asp Glu Asp Gly
        195                 200                 205

Ile Val Leu Val Asp Gln Asp Ala Cys Arg Gly Trp Arg Tyr Cys Met
    210                 215                 220

Thr Gly Cys Pro Tyr Lys Lys Val Tyr Phe Asn Trp Lys Thr Asn Lys
225                 230                 235                 240

Ala Glu Lys Cys Thr Phe Cys Phe Pro Arg Val Glu Ala Gly Met Pro
                245                 250                 255

Thr Val Cys Ser Glu Thr Cys Thr Gly Arg Met Arg Tyr Leu Gly Val
            260                 265                 270

Leu Leu Tyr Asp Ala Asp Arg Val His Glu Val Ala Ser Cys Glu Asn
        275                 280                 285

Glu Gln Asp Leu Tyr Glu Lys Gln Cys Glu Leu Phe Leu Asn Pro Tyr
    290                 295                 300

Asp Glu Asp Val Ile Glu Gln Ala Lys Lys Asp Gly Ile Thr Met Asp
305                 310                 315                 320

Trp Ile Glu Ala Ala Gln Asn Ser Pro Val Tyr Lys Leu Ala Ile Glu
                325                 330                 335

Tyr Lys Leu Ala Phe Pro Leu His Pro Glu Tyr Arg Thr Leu Pro Met
            340                 345                 350

Val Trp Tyr Cys Pro Pro Leu Ser Pro Ile Met Asn Tyr Phe Glu Gly
        355                 360                 365

Lys Asp Ser Ile Lys Asn Pro Asp Ala Ile Phe Pro Ala Ile Glu Glu
    370                 375                 380

Met Arg Leu Pro Ile Glu Tyr Leu Ala Ser Leu Leu Thr Ala Gly Asp
385                 390                 395                 400

Thr Lys Ser Val Lys Glu Ala Leu Gln Arg Met Ala Met Met Arg Ser
                405                 410                 415

Tyr Met Arg Ala Gln Val Thr Gly Lys Glu Phe Asp Thr Asp Arg Leu
            420                 425                 430

Ala Arg Leu Gly Leu Thr Glu Arg Gln Thr Lys Asp Met Tyr Arg Leu
        435                 440                 445

Leu Ala Ile Ala Lys Tyr Glu Asp Arg Phe Val Ile Pro Thr Ser His
    450                 455                 460

Lys Glu Glu Tyr Met Asp Thr Tyr Ala Ala Gln Gly Ser Gln Gly Tyr
465                 470                 475                 480

Thr Phe Gly Asp Gln Met Asn Gly Thr Gly Ser Gly Ser Gly Cys Glu
                485                 490                 495

Gly Cys Gly Ala Pro Val Ala Met Lys Pro Gly Gln Thr Gly Gln Glu
            500                 505                 510

Ala Tyr Asn Glu Ser Phe Tyr Gly Gly Ile Phe Arg Asp
        515                 520                 525

(2) INFORMATION FOR SEQ ID NO: 7:
    (i) SEQUENCE CHARACTERISTICS:
        (A) LENGTH: 191 amino acids
        (B) TYPE: amino acid
        (C) STRANDEDNESS: single
        (D) TOPOLOGY: linear
    (ii) MOLECULE TYPE: protein
    (ix) FEATURE:
        (A) NAME/KEY: Protein
        (B) LOCATION:1..191
        (D) OTHER INFORMATION:/note= "NarJ protein encoded by
              nucleotide 9374-9946 of SEQ ID NO:1"
    (xi) SEQUENCE DESCRIPTION: SEQ ID NO: 7:

Met Ile Asn Leu Asp Asn Phe Tyr Lys Tyr Gln Glu Ser Ile Gly Tyr
1               5                   10                  15

Phe Ala Gln Gln Leu Asn Phe Pro Glu Lys Leu Thr Phe His Pro Lys
            20                  25                  30

Thr Phe Asn Asp Ile Phe Asp Thr Asn His Pro Ala Tyr Ala Glu Val
        35                  40                  45

Val Ala Tyr Arg Asp Gln Ile Gln Ser Tyr Thr Leu Thr Glu Ile Lys
    50                  55                  60

Gln Leu Tyr Thr Asp Thr Phe Asp Phe Asn Lys Gln Ala Pro Leu Tyr
65                  70                  75                  80

Met Thr Tyr Asn Lys Phe Asp Thr Gln Lys Glu Arg Gly Gln Met Leu
                85                  90                  95

Ala Lys Leu Lys Val Leu Tyr Glu Met Phe Gly Leu Glu Met Val Asp
            100                 105                 110

Asn Glu Leu Ser Asp Tyr Leu Pro Leu Met Leu Glu Phe Leu Tyr Ala
        115                 120                 125

Ala Gln Trp Gln Asn Asp Asp Arg Ala Glu Gly Asn Ile Glu Phe Ile
    130                 135                 140

Ile Met Val Ile Glu Asp Gly Ser Tyr Ala Met Leu Gln Gln Leu Arg
145                 150                 155                 160

Glu Ile Asp Ser Pro Tyr Ala Pro Leu Ile Lys Gly Leu Arg Glu Thr
                165                 170                 175

Leu Lys Leu Cys Leu Gln Pro Ser Glu Glu Val Ser His His Ala
            180                 185                 190

(2) INFORMATION FOR SEQ ID NO: 8:
    (i) SEQUENCE CHARACTERISTICS:
        (A) LENGTH: 227 amino acids
        (B) TYPE: amino acid
        (C) STRANDEDNESS: single
        (D) TOPOLOGY: linear
    (ii) MOLECULE TYPE: protein
    (ix) FEATURE:
        (A) NAME/KEY: Protein
        (B) LOCATION:1..227
        (D) OTHER INFORMATION:/note= "NarI protein encoded by
            nucleotide 9942-10622 of SEQ ID NO:1"
    (xi) SEQUENCE DESCRIPTION: SEQ ID NO: 8:

Met Leu Asn Gln Phe Leu Trp Val Ile Tyr Pro Tyr Leu Cys Val Ala
1               5                   10                  15

Ile Phe Ile Ile Gly His Ile Ala Arg Tyr Arg Tyr Asp Gln Phe Ser
            20                  25                  30

Trp Thr Ala Lys Ser Ser Glu Phe Ile Glu Lys Lys Arg Leu Lys Trp
        35                  40                  45

Gly Ser Leu Leu Phe His Leu Gly Ile Ile Pro Val Ala Ala Gly His
    50                  55                  60

Phe Val Gly Leu Val Ile Pro Ala Ala Trp Leu Glu Gly Met Gly Val
65                  70                  75                  80

Asn Asn His Ile Tyr His Ile Gly Ala Val Tyr Ile Gly Ser Leu Phe
                85                  90                  95

Gly Phe Met Val Leu Ile Gly Met Ile Leu Leu Thr Tyr Arg Arg Val
            100                 105                 110

Ser Ile Lys Asn Ile Arg Arg Leu Ser Ser Ala Ser Asp Met Val Val
        115                 120                 125

Asn Phe Phe Leu Leu Val Ile Ile Cys Met Gly Leu Tyr Ser Thr Leu
    130                 135                 140

Val Thr Asn Ala Ile Ser Pro Glu Phe Asp Tyr Arg Gln Thr Ile Ser
145                 150                 155                 160

Ile Trp Phe Arg His Leu Phe Thr Phe Asn Pro Asn Ala Ser Leu Met
                165                 170                 175

Thr Asn Val Pro Trp Ser Phe Lys Leu His Ile Phe Leu Gly Phe Ser
            180                 185                 190

Ile Phe Ala Leu Trp Pro Phe Thr Arg Leu Val His Val Trp Ser Val
        195                 200                 205

Pro Leu Ser Tyr Ala Asn Arg Ser Tyr Ile Ile Tyr Arg Lys Asn Lys
    210                 215                 220

Ala Arg Thr
225

(2) INFORMATION FOR SEQ ID NO: 9:
    (i) SEQUENCE CHARACTERISTICS:
        (A) LENGTH: 2018 base pairs
        (B) TYPE: nucleic acid
        (C) STRANDEDNESS: double
        (D) TOPOLOGY: linear
    (ii) MOLECULE TYPE: DNA (genomic)
    (ix) FEATURE:
        (A) NAME/KEY: CDS
        (B) LOCATION:538..1701
        (D) OTHER INFORMATION:/function= "NITRATE TRANSPORT"
              /product= "NART GENE"
    (ix) FEATURE:
        (A) NAME/KEY: RBS
        (B) LOCATION:524..530
    (xi) SEQUENCE DESCRIPTION: SEQ ID NO: 9:

GAATTCGTCA ATAATACAGG TCAAGATGCT AATAGTACGA ATGATCCATT CCGTATTTTG      60

TCTAAAAGAG AATTAGAGAT TCTCCCGTTG ATTGCAAAAG GCTATGGAAA TAAAGAAATT     120

GCCGAAAAAC TGTTTGTGTC AGTTAAAACA GTTGAAGCGC ATAAAACACA TATTATGCAA     180

AAATTGAATT TAAAATCAAA ACCTGAATTA GTAGAGTATG CTTTGAAGAA AAAGTTACTT     240

GATTTTTGAT GAATTTTTGG AATCTGTCGA TAAAGACAGG TTCTTTTTTT ATTGTTGAAT     300

TGTGGCAAAA ATGTGACTGT AAACGCTGGT ATGACAAGGT TTAGACCTAT TAAACACCTA     360

TTAGGGAAAA GCCGAATCTT TTAAGGGAAA GCCCTTATGT TCAATCTTTC ACACGACCTA 420

TAAAATATAA ACTATAAGCA GTATCTTAGA ATATTAAAAA TAACTTCGAC TCAAAGTGAA 480

ATAATGCACT GAGGTACTGC TACTCTTTTT TGATATGAAA ATGTTAGGGT GAAAAAA 537

ATG AAC AAA TCA AAA GGC GGT TTA CAA TTA ACA GTC CAA ACT TTA AGT 585
Met Asn Lys Ser Lys Gly Gly Leu Gln Leu Thr Val Gln Thr Leu Ser
1225 1230 1235 1240

TTG GTA GCC GGC TTT ATG GTG TGG AGC ATC ATT GCA CCA TTG ATG CCG 633
Leu Val Ala Gly Phe Met Val Trp Ser Ile Ile Ala Pro Leu Met Pro
1245 1250 1255

ATG ATT TCA CAG GAC ATT AAA ATT ACA AGT TCT CAA ATT TCA ATT GTA 681
Met Ile Ser Gln Asp Ile Lys Ile Thr Ser Ser Gln Ile Ser Ile Val
1260 1265 1270

CTT GCA ATT CCT GTT ATT TTA GGA TCG GTT TTA CGT ATT CCA TTT GGT 729
Leu Ala Ile Pro Val Ile Leu Gly Ser Val Leu Arg Ile Pro Phe Gly
1275 1280 1285

TAT TTA ACT AAT ATA ATT GGC GCT AAA TGG GTT TTC TTT TCT AGT TTT 777
Tyr Leu Thr Asn Ile Ile Gly Ala Lys Trp Val Phe Phe Ser Ser Phe
1290 1295 1300

ATC ATA TTA TTA TTC CCG ATT TTC TTG CTT AGT CAA GCG CAA TCA GTG 825
Ile Ile Leu Leu Phe Pro Ile Phe Leu Leu Ser Gln Ala Gln Ser Val
1305 1310 1315 1320

AAC ATG CTG ATG TTA GCA GGA TTT TTC TTA GGT GTT GGC GGC GCA GTC 873
Asn Met Leu Met Leu Ala Gly Phe Phe Leu Gly Val Gly Gly Ala Val
1325 1330 1335

TTT TCA GTA GGT GTA ACT TCA ATT CCT AAG TAT TTC CCT AAA GAT AAA 921
Phe Ser Val Gly Val Thr Ser Ile Pro Lys Tyr Phe Pro Lys Asp Lys
1340 1345 1350

GTA GGA TTA GCA AAC GGT ATT TAT GGA ATG GGG AAC TTA GGT ACA GCG 969
Val Gly Leu Ala Asn Gly Ile Tyr Gly Met Gly Asn Leu Gly Thr Ala
1355 1360 1365

GTA TCT TCA TTC TTA GCA CCT CCG ATT GCT GGG GCA ATC GGC TGG CAA 1017
Val Ser Ser Phe Leu Ala Pro Pro Ile Ala Gly Ala Ile Gly Trp Gln
1370 1375 1380

TCA ACT GTC AGA TTA TAC TTA ATT GTG ATG GCA GTA TTT GCG ATT GTC 1065
Ser Thr Val Arg Leu Tyr Leu Ile Val Met Ala Val Phe Ala Ile Val
1385 1390 1395 1400

ATG TTC TTC TTA GGT GAT GCT AAA GAG CCT AAA GTT AAA ATT CCT TTA 1113
Met Phe Phe Leu Gly Asp Ala Lys Glu Pro Lys Val Lys Ile Pro Leu
1405 1410 1415

GTA GCA CAA ACA AAA GAT TTA CTT AAA GAT TTA AGA ACA TAC TAT TTA 1161
Val Ala Gln Thr Lys Asp Leu Leu Lys Asp Leu Arg Thr Tyr Tyr Leu
1420 1425 1430

AGT TTT TGG TAC TTT ATT ACC TTT GGT TCA TTT GTA GCA TTC GGT ATC 1209
Ser Phe Trp Tyr Phe Ile Thr Phe Gly Ser Phe Val Ala Phe Gly Ile
1435 1440 1445

TTC TTA CCG AAA TAC TTA GTC GAT CAT TAT GAA TTG ACT ACA GTA GAT 1257

Phe Leu Pro Lys Tyr Leu Val Asp His Tyr Glu Leu Thr Thr Val Asp
    1450                1455                1460

GCA GGT ATA CGT GCA GGT ATC TTT ATT GCG ATT GCT ACA TTC TTA AGA      1305
Ala Gly Ile Arg Ala Gly Ile Phe Ile Ala Ile Ala Thr Phe Leu Arg
1465                1470                1475                1480

CCT CTT GGC GGT ATT ATC GGT GAT AAA ATT GAT GCT GTA AAA GCA CTG      1353
Pro Leu Gly Gly Ile Ile Gly Asp Lys Ile Asp Ala Val Lys Ala Leu
                1485                1490                1495

AAA GTA GAC TTT TTA TTT ATG ATT ATC GGT GCA ATT ATT TTA GGT ATT      1401
Lys Val Asp Phe Leu Phe Met Ile Ile Gly Ala Ile Ile Leu Gly Ile
            1500                1505                1510

GCC AAT GAT ATG ATT TTA TTC ACA GTA GGA TGT TTA ACA GTC AGT GTG      1449
Ala Asn Asp Met Ile Leu Phe Thr Val Gly Cys Leu Thr Val Ser Val
        1515                1520                1525

TGT GCA GGT ATT GGA AAT GGA TTG GTA TTC AAA CTT GTA CCT CAG TAC      1497
Cys Ala Gly Ile Gly Asn Gly Leu Val Phe Lys Leu Val Pro Gln Tyr
    1530                1535                1540

TTC CAA AAA GAA GCG GGT GTC GCA AAC GGT ATT GTT TCA ATG ATG GGA      1545
Phe Gln Lys Glu Ala Gly Val Ala Asn Gly Ile Val Ser Met Met Gly
1545                1550                1555                1560

GGA CTT GGA GGT TTC TTC CCA CCA CTT GTT ATA ACA TAT GTA ACA AGT      1593
Gly Leu Gly Gly Phe Phe Pro Pro Leu Val Ile Thr Tyr Val Thr Ser
                1565                1570                1575

ATC ACT GGA ACA AGC CAC TTA GCT TTT ATC TTC TTA GCA TTG TTT GGT      1641
Ile Thr Gly Thr Ser His Leu Ala Phe Ile Phe Leu Ala Leu Phe Gly
            1580                1585                1590

GTC TTA GCC CTT GTA ACG ATG TGG CAT TTA AGC AAG AAA AAT CGT TCG      1689
Val Leu Ala Leu Val Thr Met Trp His Leu Ser Lys Lys Asn Arg Ser
        1595                1600                1605

CTT GCA TAT AAA TAAGAACGAT ATGGATGAAT GAAAATGTAA ATATTGGAAA      1741
Leu Ala Tyr Lys
    1610

CGTATGGGCC ATCAATCAGA AATGTTGATG GCCCATAGTT ATACTCTAAG CAGAACGTTG      1801

TGAGATAGTT GAAACTCGTG ATTGTTCTGC TTTTGTCGTG TTATTTAATG GTATGTATTT      1861

TTTGATTTGA TGCACGATCG CGATTGATTT TACGTGTCGT CATAATTAGA ACCGCAAAAG      1921

CAATACTGAC ACTCAACATT GATGAACCAC CATAACTCAG CAGTGGTAAT GTAACACCTG      1981

TTAAAGGAAT TGTACCAGAC ACACCGCCGA TGTTAAC      2018

(2) INFORMATION FOR SEQ ID NO: 10:
    (i) SEQUENCE CHARACTERISTICS:
        (A) LENGTH: 388 amino acids
        (B) TYPE: amino acid
        (D) TOPOLOGY: linear
    (ii) MOLECULE TYPE: protein

(xi) SEQUENCE DESCRIPTION: SEQ ID NO: 10:

Met Asn Lys Ser Lys Gly Gly Leu Gln Leu Thr Val Gln Thr Leu Ser
1               5                   10                  15

Leu Val Ala Gly Phe Met Val Trp Ser Ile Ile Ala Pro Leu Met Pro
            20                  25                  30

Met Ile Ser Gln Asp Ile Lys Ile Thr Ser Ser Gln Ile Ser Ile Val
        35                  40                  45

Leu Ala Ile Pro Val Ile Leu Gly Ser Val Leu Arg Ile Pro Phe Gly
    50                  55                  60

Tyr Leu Thr Asn Ile Ile Gly Ala Lys Trp Val Phe Phe Ser Ser Phe
65                  70                  75                  80

Ile Ile Leu Leu Phe Pro Ile Phe Leu Leu Ser Gln Ala Gln Ser Val
                85                  90                  95

Asn Met Leu Met Leu Ala Gly Phe Phe Leu Gly Val Gly Gly Ala Val
            100                 105                 110

Phe Ser Val Gly Val Thr Ser Ile Pro Lys Tyr Phe Pro Lys Asp Lys
        115                 120                 125

Val Gly Leu Ala Asn Gly Ile Tyr Gly Met Gly Asn Leu Gly Thr Ala
    130                 135                 140

Val Ser Ser Phe Leu Ala Pro Pro Ile Ala Gly Ala Ile Gly Trp Gln
145                 150                 155                 160

Ser Thr Val Arg Leu Tyr Leu Ile Val Met Ala Val Phe Ala Ile Val
                165                 170                 175

Met Phe Phe Leu Gly Asp Ala Lys Glu Pro Lys Val Lys Ile Pro Leu
            180                 185                 190

Val Ala Gln Thr Lys Asp Leu Leu Lys Asp Leu Arg Thr Tyr Tyr Leu
        195                 200                 205

Ser Phe Trp Tyr Phe Ile Thr Phe Gly Ser Phe Val Ala Phe Gly Ile
    210                 215                 220

Phe Leu Pro Lys Tyr Leu Val Asp His Tyr Glu Leu Thr Thr Val Asp
225                 230                 235                 240

Ala Gly Ile Arg Ala Gly Ile Phe Ile Ala Ile Ala Thr Phe Leu Arg
                245                 250                 255

Pro Leu Gly Gly Ile Ile Gly Asp Lys Ile Asp Ala Val Lys Ala Leu
            260                 265                 270

Lys Val Asp Phe Leu Phe Met Ile Ile Gly Ala Ile Ile Leu Gly Ile
        275                 280                 285

Ala Asn Asp Met Ile Leu Phe Thr Val Gly Cys Leu Thr Val Ser Val
    290                 295                 300

Cys Ala Gly Ile Gly Asn Gly Leu Val Phe Lys Leu Val Pro Gln Tyr
305                 310                 315                 320

Phe Gln Lys Glu Ala Gly Val Ala Asn Gly Ile Val Ser Met Met Gly
                325                 330                 335

Gly Leu Gly Gly Phe Phe Pro Pro Leu Val Ile Thr Tyr Val Thr Ser
          340                 345                 350

Ile Thr Gly Thr Ser His Leu Ala Phe Ile Phe Leu Ala Leu Phe Gly
        355                 360                 365

Val Leu Ala Leu Val Thr Met Trp His Leu Ser Lys Lys Asn Arg Ser
    370                 375                 380

Leu Ala Tyr Lys
385

(2) INFORMATION FOR SEQ ID NO: 11:
     (i) SEQUENCE CHARACTERISTICS:
          (A) LENGTH: 6582 base pairs
          (B) TYPE: nucleic acid
          (C) STRANDEDNESS: double
          (D) TOPOLOGY: linear
    (ii) MOLECULE TYPE: DNA (genomic)
    (ix) FEATURE:
          (A) NAME/KEY: -35_signal
          (B) LOCATION:55..60
    (ix) FEATURE:
          (A) NAME/KEY: -10_signal
          (B) LOCATION:78..83
    (ix) FEATURE:
          (A) NAME/KEY: RBS
          (B) LOCATION:101..115
    (ix) FEATURE:
          (A) NAME/KEY: CDS
          (B) LOCATION:117..1115
          (D) OTHER INFORMATION:/function= "moeB gene"
    (ix) FEATURE:
          (A) NAME/KEY: CDS
          (B) LOCATION:1150..1659
          (D) OTHER INFORMATION:/function= "moaB gene"
    (ix) FEATURE:
          (A) NAME/KEY: CDS
          (B) LOCATION:2396..3652
          (D) OTHER INFORMATION:/function= "moeA gene"
    (ix) FEATURE:
          (A) NAME/KEY: misc_feature
          (B) LOCATION:3652..4131
          (D) OTHER INFORMATION:/function= "mobB gene"
    (ix) FEATURE:
          (A) NAME/KEY: misc_feature
          (B) LOCATION:4131..4580
          (D) OTHER INFORMATION:/function= "moaE gene"
    (ix) FEATURE:
          (A) NAME/KEY: CDS
          (B) LOCATION:4583..4813
          (D) OTHER INFORMATION:/product= "moaD gene"
    (ix) FEATURE:
          (A) NAME/KEY: CDS
          (B) LOCATION:4821..5408
          (D) OTHER INFORMATION:/function= "mobA gene"
    (ix) FEATURE:
          (A) NAME/KEY: CDS
          (B) LOCATION:5470..6489
          (D) OTHER INFORMATION:/product= "moaA gene"

(ix) FEATURE:
(A) NAME/KEY: terminator
(B) LOCATION:6500..6543
(ix) FEATURE:
(A) NAME/KEY: CDS
(B) LOCATION:complement (1849..2331)
(D) OTHER INFORMATION:/function= "moaC gene"
(xi) SEQUENCE DESCRIPTION: SEQ ID NO: 11:

GAATTCAGTG CATGTCGGGT TTGTTGTATC TTTTAAAAAG TTTAGTTTGG AAAATTGTGT 60

CGTGCTCAGA ATCATCATAT AATAAAAGTA ATGTACTTTA GAAAGGATAA AAAAAT 116

ATG AAT GAA CGC TAT TCA CGA CAA ATA TTA TTT AAA AAT ATT GGA GTG 164
Met Asn Glu Arg Tyr Ser Arg Gln Ile Leu Phe Lys Asn Ile Gly Val
390 395 400

GAA GGT CAA GAA AAA ATA TCA CAA AAA CAT GTT TTG ATT ATC GGG ATG 212
Glu Gly Gln Glu Lys Ile Ser Gln Lys His Val Leu Ile Ile Gly Met
405 410 415 420

GGA GCT TTA GGT ACA CAT CTT GCT GAT GGC TTA GTA CGT GCT GGG GTA 260
Gly Ala Leu Gly Thr His Leu Ala Asp Gly Leu Val Arg Ala Gly Val
425 430 435

CGT AAA TTG ACT ATC GTG GAT CGT GAT TAT ATT GAA TTC AGC AAT CTG 308
Arg Lys Leu Thr Ile Val Asp Arg Asp Tyr Ile Glu Phe Ser Asn Leu
440 445 450

CAG CGT CAA ACA ATG TAT ACA GAA AAT GAT GCT AAA GAA GCA TTG CCT 356
Gln Arg Gln Thr Met Tyr Thr Glu Asn Asp Ala Lys Glu Ala Leu Pro
455 460 465

AAA GTA ATA GCT GCA AAA GAA AGA TTG ACA GAA ATT CGT AGT GAT ATT 404
Lys Val Ile Ala Ala Lys Glu Arg Leu Thr Glu Ile Arg Ser Asp Ile
470 475 480

GAA ATA GAT GCA TAT ATC GAT CAA GTT AAC CCG CCA TTT TTA GAA GAA 452
Glu Ile Asp Ala Tyr Ile Asp Gln Val Asn Pro Pro Phe Leu Glu Glu
485 490 495 500

CAT GCA CAA CAT GTG GAT TTA ATA TTA GAT GCA ACA GAT AAT TTT GAT 500
His Ala Gln His Val Asp Leu Ile Leu Asp Ala Thr Asp Asn Phe Asp
505 510 515

ACG CGT TTA TTG GTA AAT GAT TTT GCC TAT AAA TAT AAT ATA CCT TGG 548
Thr Arg Leu Leu Val Asn Asp Phe Ala Tyr Lys Tyr Asn Ile Pro Trp
520 525 530

ATA TAT GGG GGC GTT GTA CAA AGC ACG TAC ATT GAA GCA GCC TTT ATT 596
Ile Tyr Gly Gly Val Val Gln Ser Thr Tyr Ile Glu Ala Ala Phe Ile
535 540 545

CCA GGA CAA ACA CCT TGT TTT AAT TGT GTG ATG CCG CAA TTG CCA GTC 644
Pro Gly Gln Thr Pro Cys Phe Asn Cys Val Met Pro Gln Leu Pro Val
550 555 560

ATT AAT ATG ACT TGT GAT ACT GTA GGT GTA ATT CAA CCA GCT GTC ACA 692
Ile Asn Met Thr Cys Asp Thr Val Gly Val Ile Gln Pro Ala Val Thr
565 570 575 580

ATG ACA ACG AGT TTA CAG TTA AGA GAC GCG TTG AAA ATA CTG ACG GAC 740
Met Thr Thr Ser Leu Gln Leu Arg Asp Ala Leu Lys Ile Leu Thr Asp
585 590 595

ACA CCC TTT ACA CCT AAA TTA ACT TAT GGT GAC ATA TGG GAA GGT ACA 788
Thr Pro Phe Thr Pro Lys Leu Thr Tyr Gly Asp Ile Trp Glu Gly Thr
600 605 610

CAT TAT ACA TTT GGT TTC AGC CGA ATG TTT GAT TCT ACT TGT ACA ACT 836
His Tyr Thr Phe Gly Phe Ser Arg Met Phe Asp Ser Thr Cys Thr Thr
615 620 625

TGC GGA CCG AAC CCT ACG TAT CCT CAT TTA CAT CAA ACT GAT CAG CAA 884
Cys Gly Pro Asn Pro Thr Tyr Pro His Leu His Gln Thr Asp Gln Gln
630 635 640

TAT GCG ACA CTT TGC GGG AGA GAC ACC GTA CAA TAC AGT AAT GAA AAT 932
Tyr Ala Thr Leu Cys Gly Arg Asp Thr Val Gln Tyr Ser Asn Glu Asn
645 650 655 660

ATT ACC CAG GAA ATG TTG AAA CAA TAT TTA GAT GCG CAT CAT ATC CAT 980
Ile Thr Gln Glu Met Leu Lys Gln Tyr Leu Asp Ala His His Ile His
665 670 675

TAT CAT GCA AAT CCA TAT ATG CTT CGT TTT GAA TTT AAT GGG CAT CGT 1028
Tyr His Ala Asn Pro Tyr Met Leu Arg Phe Glu Phe Asn Gly His Arg
680 685 690

ATT GTG AGT TTC TCA GGT GGA AGA ATG TTA ATT CAT GGT ATG ACT AAG 1076
Ile Val Ser Phe Ser Gly Gly Arg Met Leu Ile His Gly Met Thr Lys
695 700 705

CCG ACT GAG GCT ATT AAA TTA ATG AAT CAA CTG TTA GGC TAGAAGTAGA 1125
Pro Thr Glu Ala Ile Lys Leu Met Asn Gln Leu Leu Gly
710 715 720

TTATAAATAA CGGAGGTCAA TCGA ATG ACA AAA AAT GAA CAT GTA AAC GTT 1176
Met Thr Lys Asn Glu His Val Asn Val
1 5

AAG CTA GAT AGA GAT ATT CAA TGT GCG GTG TTG ACA GTA TCA GAT ACG 1224
Lys Leu Asp Arg Asp Ile Gln Cys Ala Val Leu Thr Val Ser Asp Thr
10 15 20 25

AGA ACA CCG GAA ACA GAT AAA GGC GGC AAC TTA GCA AAA GAA TTA TTA 1272
Arg Thr Pro Glu Thr Asp Lys Gly Gly Asn Leu Ala Lys Glu Leu Leu
30 35 40

TCT GAA ATT AAT GTA GAA ATC AAA CCA GAA CAT TAT GCG ATT GTT AAA 1320
Ser Glu Ile Asn Val Glu Ile Lys Pro Glu His Tyr Ala Ile Val Lys
45 50 55

GAT GAT AAA CAA GCG ATT ACA GAG CAA TTA CAA CAA TGG TTG GCT GAA 1368
Asp Asp Lys Gln Ala Ile Thr Glu Gln Leu Gln Gln Trp Leu Ala Glu
60 65 70

GAT GTA GAT GTC ATT ATC ACA ACT GGT GGC ACA GGA ATT GCA CAA CGC 1416
Asp Val Asp Val Ile Ile Thr Thr Gly Gly Thr Gly Ile Ala Gln Arg
75 80 85

GAT GTT ACA ATA GAA GCT GTA ACA CCA TTG CTT TCA AAA GAA ATT GAA 1464
Asp Val Thr Ile Glu Ala Val Thr Pro Leu Leu Ser Lys Glu Ile Glu
90 95 100 105

GGT TTC GGA GAA TTG TTC AGA TAT TTA AGT TAT GCT GAA GAT GTT GGT 1512
Gly Phe Gly Glu Leu Phe Arg Tyr Leu Ser Tyr Ala Glu Asp Val Gly
110 115 120

ACA CGT GCT TTA CTT TCA CGT GCA GTA GCA GGC ACA GTT GGA GAA CAA 1560
Thr Arg Ala Leu Leu Ser Arg Ala Val Ala Gly Thr Val Gly Glu Gln
125 130 135

CTT ATT TTC TCA GTA CCA GGT TCA ACG GGT GCA GTG AAG CTT GCA ATG 1608
Leu Ile Phe Ser Val Pro Gly Ser Thr Gly Ala Val Lys Leu Ala Met
140 145 150

AAT AAA CTA ATT AAA CCA GAA TTA AAT CAT TTA ATT CAT GAA CTG ACA 1656
Asn Lys Leu Ile Lys Pro Glu Leu Asn His Leu Ile His Glu Leu Thr
155 160 165

AAA TAAATCATCC TTATAGACGA TTATCATATG ATGATTGAGG TTTATAATTA 1709
Lys
170

ATATATGATA CGAAAAAACG GAATCATTGA GCAGACATTT TATGTTAAAT AAAAAATGTT 1769

GTTGCTCAAC AATTCCGTAT TTTGTTCTGT TTAAATTAAA TAACTCAGAA CGGGTATATA 1829

ATTTAAATAA AGAATATTAT TTAGATTCTC GGCGATAGTC GCCAGATTTA CCACCAGATT 1889

TAGTTAGAAG ATACGTTTCT CCGATGACCA TTCCTTTATC TAAAGCTTTG GTCATATCAT 1949

AAACCGTTAA TGCAGTAGCA GAAGCTGCAG TTAAGGCTTC CATCTCTACT CCGGTTTTAC 2009

CAGACGTTGA AACAGTAGCT TCAATGTTTA GAGTATAAGA TGATGTTGTA TCCCATTCGA 2069

AAGCAATATC AATACCAGTA AGTGAGAGCG GATGGCACAT TGGAATAATT GTGGATGTGT 2129

TTTTGGCAGC CATAATTCCA GCAATCTGTG CAGTGTTCAG CACATTACCT TTCTTATTTG 2189

TATGATCGAC AATTTGCTGA TAAATATCTT GATTGACGGT AATACTTGAA TGAGCGGTAG 2249

CAGTACGTTT CGTGATTTCT TTTTCAGAAA CATCTATCAT CTTCGCATTA CCTTGTTCGT 2309

TGATATGAGT AAATTCAGAC ATGCTATTCC TCCTCGCTTA AATTATTATA GCACGAAATA 2369

AATTGAATGT TAGGAGCAGA TAAATT ATG CCA GTT GAA AAA AGA AAT CCA ATT 2422
Met Pro Val Glu Lys Arg Asn Pro Ile
1 5

CCT GTT AGA GAA GCT ATC AAA CGT GTT GTA GAA CAA GAT ATT GAA ACT 2470
Pro Val Arg Glu Ala Ile Lys Arg Val Val Glu Gln Asp Ile Glu Thr
10 15 20 25

GAA GTA CAA AAT GTA CCA TTA AAT AAA AGT TTA GGG TAC GTA CTT GCA 2518
Glu Val Gln Asn Val Pro Leu Asn Lys Ser Leu Gly Tyr Val Leu Ala
30 35 40

GAA GAT ATT GTA GCA ACA TAT GAA ATT CCT AGA TTT AAT AAA TCA CCT 2566
Glu Asp Ile Val Ala Thr Tyr Glu Ile Pro Arg Phe Asn Lys Ser Pro
45 50 55

TAT GAT GGT TTT GCT TTG AGA AGT GAA GAT ACA GTA GGT GCA AGC GGT 2614
Tyr Asp Gly Phe Ala Leu Arg Ser Glu Asp Thr Val Gly Ala Ser Gly
60 65 70

GAT AAC CGT ATT GAA TTC GAA GTG ATT GAT CAC ATC GGT GCA GGT TCA 2662
Asp Asn Arg Ile Glu Phe Glu Val Ile Asp His Ile Gly Ala Gly Ser
75 80 85

GTT TCA GAT AAA ACT GTA GGT CCT AAT CAA GCT GTT CGA ATC ATG ACA 2710
Val Ser Asp Lys Thr Val Gly Pro Asn Gln Ala Val Arg Ile Met Thr
90 95 100 105

GGT GCA GAA GTT CCA GCA GGT GCA GAT GCT GTG GTT ATG CTA GAA CAA 2758
Gly Ala Glu Val Pro Ala Gly Ala Asp Ala Val Val Met Leu Glu Gln
110 115 120

ACA AAA GAA GGC GAC CAT ACA TTT ACG ATT CGT AAA ACA TTC TCT CAC 2806
Thr Lys Glu Gly Asp His Thr Phe Thr Ile Arg Lys Thr Phe Ser His
125 130 135

AAT GAA AAT GTT TCA TTA AAA GGC GAA GAA ACA GAA GTG GGC GAT GTT 2854
Asn Glu Asn Val Ser Leu Lys Gly Glu Glu Thr Glu Val Gly Asp Val
140 145 150

GTA TTG AAA AAA GGA CAA CGT ATT AAT GCC GGC GGT ATT GCA GTG CTT 2902
Val Leu Lys Lys Gly Gln Arg Ile Asn Ala Gly Gly Ile Ala Val Leu
155 160 165

GCA ACA TTC GGC TAC ACA GAA GTT CCA GTT CGT AAA TTA CCA TCT GCT 2950
Ala Thr Phe Gly Tyr Thr Glu Val Pro Val Arg Lys Leu Pro Ser Ala
170 175 180 185

GCA ATT ATC GCA ACA GGC AGT GAA TTA TTA GAT GTC GGT GAT GAA TTA 2998
Ala Ile Ile Ala Thr Gly Ser Glu Leu Leu Asp Val Gly Asp Glu Leu
190 195 200

GAA CCA GGT AAA ATT CGT AAT TCT AAC GGA CCT ATG ATT GAA GGG TTA 3046
Glu Pro Gly Lys Ile Arg Asn Ser Asn Gly Pro Met Ile Glu Gly Leu
205 210 215

TTA AAT CAA TTT GGT TTA GAT GGA GAA GTT TAT AAA ATT CAA GAA GAT 3094
Leu Asn Gln Phe Gly Leu Asp Gly Glu Val Tyr Lys Ile Gln Glu Asp
220 225 230

GAC TTA GAA AGT AGT ATT GCC GTT GTA AAA CAA GCG ATG GAA ACA CAT 3142
Asp Leu Glu Ser Ser Ile Ala Val Val Lys Gln Ala Met Glu Thr His
235 240 245

GAC ATG GTC ATT ACT ACA GGC GGC GTT TCA GTA GGT GAT TTT GAT TAT 3190
Asp Met Val Ile Thr Thr Gly Gly Val Ser Val Gly Asp Phe Asp Tyr
250 255 260 265

CTG CCT GAA ATC TAT AAA GCT TTA GAT GCT GAA GTA TTA TTT AAT AAA 3238
Leu Pro Glu Ile Tyr Lys Ala Leu Asp Ala Glu Val Leu Phe Asn Lys
270 275 280

GTA CAG ATG AGA CCA GGC AGT GTT ACA ACT GTA GCG GTT GCA CAT GGT 3286
Val Gln Met Arg Pro Gly Ser Val Thr Thr Val Ala Val Ala His Gly
285 290 295

AAA TAT TTA TTC GGT TTA TCC GGA AAT CCA TCA GCT TGT TAT ACA GGT 3334
Lys Tyr Leu Phe Gly Leu Ser Gly Asn Pro Ser Ala Cys Tyr Thr Gly
300 305 310

TTC CAA TTA TTT GTT AAA ACA GGC GTA TTG AAT ATG ATG GGT GCT AAT 3382
Phe Gln Leu Phe Val Lys Thr Gly Val Leu Asn Met Met Gly Ala Asn
315 320 325

GAA AAA TTC CCT CAA GTT GTG AAA GCA ACT TTA ATG GAA GAT TTC AAA 3430
Glu Lys Phe Pro Gln Val Val Lys Ala Thr Leu Met Glu Asp Phe Lys
330 335 340 345

AAA GCC AAT CCA TTC ACA CGT TTT ATT AGA TCA AAA GCT ACT TTA AAT 3478
Lys Ala Asn Pro Phe Thr Arg Phe Ile Arg Ser Lys Ala Thr Leu Asn
350 355 360

GGA ATG GAA GCA ACA GTT GTA CCT TCA GGA TTT AAT AAA TCA GGT GCC 3526
Gly Met Glu Ala Thr Val Val Pro Ser Gly Phe Asn Lys Ser Gly Ala
365 370 375

GTT GTA GCA ATT GCA CAT AGT AAT GCA ATG GTG ATG CTG CCA AGT GGT 3574
Val Val Ala Ile Ala His Ser Asn Ala Met Val Met Leu Pro Ser Gly
380 385 390

ACA AGA GGA TTC CAT GCT GGT AAT ACA GTA GAT GTT ATC TTA ACA GAA 3622
Thr Arg Gly Phe His Ala Gly Asn Thr Val Asp Val Ile Leu Thr Glu
395 400 405

ACA GAT GTA TTT GAA GAG GAA CTT TTA CTA TGATTTTGCA AATCGTGGGA 3672
Thr Asp Val Phe Glu Glu Glu Leu Leu Leu
410 415

TATAAAGATT CAGGCAAAAC TACATTGTTA GCTGAAACGG TTAAGTTTTT AAAAAATAAA 3732

GGTTATCGTG TAGTCACAAT CAAACATCAT GGTCATGGTG CAGAGGATAT TACATTGCAG 3792

GAGAACACTG TTGACCATAT GAAACATTTT GAAGCGGGAG CAGATCAAAG TATCGTACAA 3852

GGACACGAAC TCAGAGAAAC GATAACCCGC ACGTCTGAAG CGTCTTTATC CCAAATTATT 3912

GATTCAGCTG TTACAATTGA ATATGACATC ATTTTAGCTG AAGGGTTTAA AAATGCCGAC 3972

TATGATAAAG TTGTCATTTA TAAAAATCCT GAAGAATTAT CTTCTTTATC AGCGCTCAGT 4032

CATGTACAAT ATAAGTTGCC GTTTCAAAAA TTAAGTGATT TAGAGCCTTT TAAAAATTGG 4092

CTTGAATCGT GGATTGATAT AAAAAAGGAT GAGTCAGAAT GAAACAGTTT GAGATTGTAA 4152

CAGAACCGAT ACAAACAGAA CAGCATCGAG ACTTTACACT TAATCCGCAC CAAGGAGCAG 4212

TGGTTGTATT TACAGGACAT GTCCGAGAAT GGACTAAAGG GATACGCACT GAACATTTAG 4272

AATATGAAGC TTATATTCCA ATGGCTGAGA AGAAACTCGC ACAAATCGGT GATGAAATTA 4332

ATGAACAGTG GCCAGGTACA ATTGTAAGTA TTGTACATCG AATCGGGCCA TTAAAAATTT 4392

CAGATATAGC TGTGTTGATT GCGGTATCTT CTCCGCATAG AAAAGATGCA TACGCTGCTA 4452

ATGAGTATGC AATAGATCGT ATTAAAGAAG TTGTTCCCAT TTGGAAAAAA GAAATTTGGG 4512

AAGATGGAGC TGAGTGGATT GGCCATCAAC GCGGCTACCA CGATGATGCA GTTGAAAGGG 4572

GGCAAAATTA ATG AAA GTC CTT TAT TTT GCT GAA ATC AAA GAG ATT TTG 4621
Met Lys Val Leu Tyr Phe Ala Glu Ile Lys Glu Ile Leu
1 5 10

CAA AAA GAT ACT GAT CAA TTT CAG ATT GAT GAT GAA ATG ACT GTG GAA 4669
Gln Lys Asp Thr Asp Gln Phe Gln Ile Asp Asp Glu Met Thr Val Glu
15 20 25

GCA TTT AAA CAA TAT TTA TTT GAA AAA TAT CCA GAG ATA GAT GGA AAG 4717
Ala Phe Lys Gln Tyr Leu Phe Glu Lys Tyr Pro Glu Ile Asp Gly Lys
30 35 40 45

AAA TTT CAA ATT GCT TTA AAC GAA GAA TTT GTA CAA CCT CAC GAA AAA 4765
Lys Phe Gln Ile Ala Leu Asn Glu Glu Phe Val Gln Pro His Glu Lys
50 55 60

ATC AAC CAA TCA GAT GTT GTT GCT TTG ATT CCA CCA GTA AGT GGA GGT 4813
Ile Asn Gln Ser Asp Val Val Ala Leu Ile Pro Pro Val Ser Gly Gly
65 70 75

TAATGAA GTG AAA GCC ATT ATA TTA GCA GGA GGG CAA TCA GAA CGT TTT 4862
Val Lys Ala Ile Ile Leu Ala Gly Gly Gln Ser Glu Arg Phe
1 5 10

GGG GCG CCT AAA GCA TTT GCA GAA ATA GAT GGG AAA ATG TTC TAT GAA 4910
Gly Ala Pro Lys Ala Phe Ala Glu Ile Asp Gly Lys Met Phe Tyr Glu
15 20 25 30

CAA ATA ATT ACT GTA TTA GAT AGC ATG AAT ATG TTC AAT GAA ATT ATT 4958
Gln Ile Ile Thr Val Leu Asp Ser Met Asn Met Phe Asn Glu Ile Ile
35 40 45

ATA AGT TCA AAC GAA ACA CTT GCA TCT GAA TTT AAA GGT GCA CGT GTT 5006
Ile Ser Ser Asn Glu Thr Leu Ala Ser Glu Phe Lys Gly Ala Arg Val
50 55 60

ATT GTG GAC GAT TCA GAA CAC AAA AAT AAA GGA CCT TTA TCT GGG ATT 5054
Ile Val Asp Asp Ser Glu His Lys Asn Lys Gly Pro Leu Ser Gly Ile
65 70 75

TAT TCT GTT ATG AAA CAA GAT TTG AAT CAG AAT TAT TTT TGT GAT TTC 5102
Tyr Ser Val Met Lys Gln Asp Leu Asn Gln Asn Tyr Phe Cys Asp Phe
80 85 90

TGT GGA TAC GCC GCT TAT TAC CGT AAA GCA ATC AGC CAA TTG TAT CAA 5150
Cys Gly Tyr Ala Ala Tyr Tyr Arg Lys Ala Ile Ser Gln Leu Tyr Gln
95 100 105 110

TTC ATG GTA GAA CAT GTG ATT GAA GAT CAA TTA GAC ATT GCT GGT TTT 5198
Phe Met Val Glu His Val Ile Glu Asp Gln Leu Asp Ile Ala Gly Phe
115 120 125

AAA GAA GGT AAC CAT CCT ATT CCG ACA ATT GCC TTT TAC AGC CCT AAT 5246
Lys Glu Gly Asn His Pro Ile Pro Thr Ile Ala Phe Tyr Ser Pro Asn
130 135 140

TGT TTG CCG ATT ATT GCA CGT GCT TTA GAG TCA GAT GAC TAT AGT ATG 5294
Cys Leu Pro Ile Ile Ala Arg Ala Leu Glu Ser Asp Asp Tyr Ser Met
145 150 155

CGT CAT GTT TAT CAA CAA ACC GCA TCT GAT TGG ATA GAT GTA AGT AGT 5342
Arg His Val Tyr Gln Gln Thr Ala Ser Asp Trp Ile Asp Val Ser Ser
160 165 170

GTA GAC GAT GAT ACA GAA TGG TAT AAG AAT ATT AAT TAT CCC CAA GAT 5390
Val Asp Asp Asp Thr Glu Trp Tyr Lys Asn Ile Asn Tyr Pro Gln Asp
175 180 185 190

TTA GAG AGC ATA AAG AAA TAATAATCAA CGTTTAACGA GATGTGTTAA 5438
Leu Glu Ser Ile Lys Lys
195

GCGATTAATA TAGATAGAAG GGGGTCAAAA G ATG GTA AAA CAG ATA ACG GAT 5490
Met Val Lys Gln Ile Thr Asp
1 5

AAA CTT GGA CGT CCA ATT CGT GAT TTA AGA TTA TCT GTG ACA GAC CGT 5538
Lys Leu Gly Arg Pro Ile Arg Asp Leu Arg Leu Ser Val Thr Asp Arg
10 15 20

TGC AAT TTC CGA TGT GAT TAT TGC ATG CCG AAA GAA ATA TTC GGT GAC 5586
Cys Asn Phe Arg Cys Asp Tyr Cys Met Pro Lys Glu Ile Phe Gly Asp
25 30 35

GAT TTT GTC TTT TTA CCC AAA GAT GAA TTA CTG AGT TTC AGC GAA ATG 5634
Asp Phe Val Phe Leu Pro Lys Asp Glu Leu Leu Ser Phe Ser Glu Met
40 45 50 55

GAA CGA ATT GCT CGC GTA TAT ACA CAC TTA GGT GTG AAA AAA ATC CGT 5682
Glu Arg Ile Ala Arg Val Tyr Thr His Leu Gly Val Lys Lys Ile Arg
60 65 70

ATT ACA GGC GGA GAA CCA TTA ATG CGC AGA GAT TTA TAT AAA TTA ATT 5730
Ile Thr Gly Gly Glu Pro Leu Met Arg Arg Asp Leu Tyr Lys Leu Ile
75 80 85

GCA GCC TTG AAT GAG ATT GAA GGT GTA GAA GAT ATT GGT TTA ACA ACC 5778
Ala Ala Leu Asn Glu Ile Glu Gly Val Glu Asp Ile Gly Leu Thr Thr
90 95 100

AAT GGT TTA TTA CTG AAA AAA CAT GGA CAG AAA TTA TAT GAT GCA GGT 5826
Asn Gly Leu Leu Leu Lys Lys His Gly Gln Lys Leu Tyr Asp Ala Gly
105 110 115

TTA AGA CGT ATT AAT GTC AGT CTA GAT GCT ATT GAT AAT GAA CTG TTC 5874
Leu Arg Arg Ile Asn Val Ser Leu Asp Ala Ile Asp Asn Glu Leu Phe
120 125 130 135

CAA TCT ATT AAC AAC CGT AAT ATC AAA GCA GAT ACA ATT TTA GAA CAA 5922
Gln Ser Ile Asn Asn Arg Asn Ile Lys Ala Asp Thr Ile Leu Glu Gln
140 145 150

ATT GAT TAT GCT GTT TCT ATT GGT TTT AAA GTG AAA ATC AAT GTT GTT 5970
Ile Asp Tyr Ala Val Ser Ile Gly Phe Lys Val Lys Ile Asn Val Val
155 160 165

GTT CAA AAA GGT GTG AAC GAT GAC CAA ATT ATT CCT ATG GTT CAA TAT 6018
Val Gln Lys Gly Val Asn Asp Asp Gln Ile Ile Pro Met Val Gln Tyr
170 175 180

TTC AAG GAT AAG AAT ATT CAA GTC AGA TTT ATT GAA TTC ATG GAT GTT 6066
Phe Lys Asp Lys Asn Ile Gln Val Arg Phe Ile Glu Phe Met Asp Val
185 190 195

GGT AAT GAT AAC GGT TGG GAT TTT AGT AAA GTT GTA TCG AAA GAT GAA 6114
Gly Asn Asp Asn Gly Trp Asp Phe Ser Lys Val Val Ser Lys Asp Glu
200 205 210 215

ATG CTT TCA ATG ATT CAA GAA GAA TTT GAT ATT GAA GCG GTA GAA CCT 6162
Met Leu Ser Met Ile Gln Glu Glu Phe Asp Ile Glu Ala Val Glu Pro
220 225 230

AAA TAT TAT GGG GAA GTT GCG AAA TAT TAC CGC CAT AAA GAT AAT GGC 6210
Lys Tyr Tyr Gly Glu Val Ala Lys Tyr Tyr Arg His Lys Asp Asn Gly
235 240 245

GCA CAA TTT GGA TTG ATT ACA AGT GTG TCA CAA TCA TTC TGC TCT ACT 6258
Ala Gln Phe Gly Leu Ile Thr Ser Val Ser Gln Ser Phe Cys Ser Thr
250 255 260

TGT ACA AGA GCG AGA CTA TCA TCT GAC GGT AAG TTT TAT GGC TGC TTG 6306
Cys Thr Arg Ala Arg Leu Ser Ser Asp Gly Lys Phe Tyr Gly Cys Leu
265 270 275

TTC AGT ACC GTT GAT GGT TTT AAT GTT AAA GAA TTT ATG CGT TCA GGT 6354
Phe Ser Thr Val Asp Gly Phe Asn Val Lys Glu Phe Met Arg Ser Gly
280 285 290 295

GTT TCA GAT GAT GAA CTT CAA GCG AAG TTC GAA GAA TTA TGG AAT ATT 6402
Val Ser Asp Asp Glu Leu Gln Ala Lys Phe Glu Glu Leu Trp Asn Ile
300 305 310

CGA GAT GAT CGT TAT TCA GAT GAA CGT ACA GAA CAA ACT GTC GCA ATT 6450
Arg Asp Asp Arg Tyr Ser Asp Glu Arg Thr Glu Gln Thr Val Ala Ile
315 320 325

CGA AAA CGT AAA AAA ATT AAT ATG AAC TAT ATT GGC GGT TAAAGATACG 6499
Arg Lys Arg Lys Lys Ile Asn Met Asn Tyr Ile Gly Gly
330 335 340

AAGACCCGAA TCTCTTTTCT AAATAGGAAT GAGATTCGGG TCTTTTTTAT GCCGCTGTAT 6559

TAAAATCAAT TTCTTTAACA ATT 6582

(2) INFORMATION FOR SEQ ID NO: 12:
(i) SEQUENCE CHARACTERISTICS:
(A) LENGTH: 333 amino acids
(B) TYPE: amino acid
(D) TOPOLOGY: linear
(ii) MOLECULE TYPE: protein
(xi) SEQUENCE DESCRIPTION: SEQ ID NO: 12:

Met Asn Glu Arg Tyr Ser Arg Gln Ile Leu Phe Lys Asn Ile Gly Val
1               5                   10                  15

Glu Gly Gln Glu Lys Ile Ser Gln Lys His Val Leu Ile Ile Gly Met
            20                  25                  30

Gly Ala Leu Gly Thr His Leu Ala Asp Gly Leu Val Arg Ala Gly Val
        35                  40                  45

Arg Lys Leu Thr Ile Val Asp Arg Asp Tyr Ile Glu Phe Ser Asn Leu
    50                  55                  60

Gln Arg Gln Thr Met Tyr Thr Glu Asn Asp Ala Lys Glu Ala Leu Pro
65                  70                  75                  80

Lys Val Ile Ala Ala Lys Glu Arg Leu Thr Glu Ile Arg Ser Asp Ile
                85                  90                  95

Glu Ile Asp Ala Tyr Ile Asp Gln Val Asn Pro Pro Phe Leu Glu Glu
            100                 105                 110

His Ala Gln His Val Asp Leu Ile Leu Asp Ala Thr Asp Asn Phe Asp
        115                 120                 125

Thr Arg Leu Leu Val Asn Asp Phe Ala Tyr Lys Tyr Asn Ile Pro Trp
    130                 135                 140

Ile Tyr Gly Gly Val Val Gln Ser Thr Tyr Ile Glu Ala Ala Phe Ile
145                 150                 155                 160

Pro Gly Gln Thr Pro Cys Phe Asn Cys Val Met Pro Gln Leu Pro Val
                165                 170                 175

Ile Asn Met Thr Cys Asp Thr Val Gly Val Ile Gln Pro Ala Val Thr
            180                 185                 190

Met Thr Thr Ser Leu Gln Leu Arg Asp Ala Leu Lys Ile Leu Thr Asp
        195                 200                 205

Thr Pro Phe Thr Pro Lys Leu Thr Tyr Gly Asp Ile Trp Glu Gly Thr
    210                 215                 220

His Tyr Thr Phe Gly Phe Ser Arg Met Phe Asp Ser Thr Cys Thr Thr
225                 230                 235                 240

Cys Gly Pro Asn Pro Thr Tyr Pro His Leu His Gln Thr Asp Gln Gln
                245                 250                 255

Tyr Ala Thr Leu Cys Gly Arg Asp Thr Val Gln Tyr Ser Asn Glu Asn
            260                 265                 270

Ile Thr Gln Glu Met Leu Lys Gln Tyr Leu Asp Ala His His Ile His
        275                 280                 285

Tyr His Ala Asn Pro Tyr Met Leu Arg Phe Glu Phe Asn Gly His Arg
    290                 295                 300

Ile Val Ser Phe Ser Gly Gly Arg Met Leu Ile His Gly Met Thr Lys
305                 310                 315                 320

Pro Thr Glu Ala Ile Lys Leu Met Asn Gln Leu Leu Gly
                325                 330

(2) INFORMATION FOR SEQ ID NO: 13:
        (i) SEQUENCE CHARACTERISTICS:
            (A) LENGTH: 170 amino acids
            (B) TYPE: amino acid
            (D) TOPOLOGY: linear
       (ii) MOLECULE TYPE: protein
       (xi) SEQUENCE DESCRIPTION: SEQ ID NO: 13:

Met Thr Lys Asn Glu His Val Asn Val Lys Leu Asp Arg Asp Ile Gln
  1               5                  10                  15

Cys Ala Val Leu Thr Val Ser Asp Thr Arg Thr Pro Glu Thr Asp Lys
             20                  25                  30

Gly Gly Asn Leu Ala Lys Glu Leu Leu Ser Glu Ile Asn Val Glu Ile
         35                  40                  45

Lys Pro Glu His Tyr Ala Ile Val Lys Asp Asp Lys Gln Ala Ile Thr
     50                  55                  60

Glu Gln Leu Gln Gln Trp Leu Ala Glu Asp Val Asp Val Ile Ile Thr
 65                  70                  75                  80

Thr Gly Gly Thr Gly Ile Ala Gln Arg Asp Val Thr Ile Glu Ala Val
                85                  90                  95

Thr Pro Leu Leu Ser Lys Glu Ile Glu Gly Phe Gly Glu Leu Phe Arg
            100                 105                 110

Tyr Leu Ser Tyr Ala Glu Asp Val Gly Thr Arg Ala Leu Leu Ser Arg
        115                 120                 125

Ala Val Ala Gly Thr Val Gly Glu Gln Leu Ile Phe Ser Val Pro Gly
    130                 135                 140

Ser Thr Gly Ala Val Lys Leu Ala Met Asn Lys Leu Ile Lys Pro Glu
145                 150                 155                 160

Leu Asn His Leu Ile His Glu Leu Thr Lys
                165                 170

(2) INFORMATION FOR SEQ ID NO: 14:
      (i) SEQUENCE CHARACTERISTICS:
         (A) LENGTH: 161 amino acids
         (B) TYPE: amino acid
         (D) TOPOLOGY: linear
     (ii) MOLECULE TYPE: protein
     (xi) SEQUENCE DESCRIPTION: SEQ ID NO: 14:

Met Ser Glu Phe Thr His Ile Asn Glu Gln Gly Asn Ala Lys Met Ile
  1               5                  10                  15

Asp Val Ser Glu Lys Glu Ile Thr Lys Arg Thr Ala Thr Ala His Ser
             20                  25                  30

Ser Ile Thr Val Asn Gln Asp Ile Tyr Gln Gln Ile Val Asp His Thr
         35                  40                  45

Asn Lys Lys Gly Asn Val Leu Asn Thr Ala Gln Ile Ala Gly Ile Met
     50                  55                  60

Ala Ala Lys Asn Thr Ser Thr Ile Ile Pro Met Cys His Pro Leu Ser
 65                  70                  75                  80

Leu Thr Gly Ile Asp Ile Ala Phe Glu Trp Asp Thr Thr Ser Ser Tyr
                 85                  90                  95

Thr Leu Asn Ile Glu Ala Thr Val Ser Thr Ser Gly Lys Thr Gly Val
            100                 105                 110

Glu Met Glu Ala Leu Thr Ala Ala Ser Ala Thr Ala Leu Thr Val Tyr
        115                 120                 125

Asp Met Thr Lys Ala Leu Asp Lys Gly Met Val Ile Gly Glu Thr Tyr
    130                 135                 140

Leu Leu Thr Lys Ser Gly Gly Lys Ser Gly Asp Tyr Arg Arg Glu Ser
145                 150                 155                 160

Lys

(2) INFORMATION FOR SEQ ID NO: 15:
(i) SEQUENCE CHARACTERISTICS:
(A) LENGTH: 419 amino acids
(B) TYPE: amino acid
(D) TOPOLOGY: linear
(ii) MOLECULE TYPE: protein
(xi) SEQUENCE DESCRIPTION: SEQ ID NO: 15:

Met Pro Val Glu Lys Arg Asn Pro Ile Pro Val Arg Glu Ala Ile Lys
1               5                   10                  15

Arg Val Val Glu Gln Asp Ile Glu Thr Glu Val Gln Asn Val Pro Leu
            20                  25                  30

Asn Lys Ser Leu Gly Tyr Val Leu Ala Glu Asp Ile Val Ala Thr Tyr
        35                  40                  45

Glu Ile Pro Arg Phe Asn Lys Ser Pro Tyr Asp Gly Phe Ala Leu Arg
    50                  55                  60

Ser Glu Asp Thr Val Gly Ala Ser Gly Asp Asn Arg Ile Glu Phe Glu
65                  70                  75                  80

Val Ile Asp His Ile Gly Ala Gly Ser Val Ser Asp Lys Thr Val Gly
                85                  90                  95

Pro Asn Gln Ala Val Arg Ile Met Thr Gly Ala Glu Val Pro Ala Gly
            100                 105                 110

Ala Asp Ala Val Val Met Leu Glu Gln Thr Lys Glu Gly Asp His Thr
        115                 120                 125

Phe Thr Ile Arg Lys Thr Phe Ser His Asn Glu Asn Val Ser Leu Lys
    130                 135                 140

Gly Glu Glu Thr Glu Val Gly Asp Val Val Leu Lys Lys Gly Gln Arg
145                 150                 155                 160

Ile Asn Ala Gly Gly Ile Ala Val Leu Ala Thr Phe Gly Tyr Thr Glu
                165                 170                 175

Val Pro Val Arg Lys Leu Pro Ser Ala Ala Ile Ile Ala Thr Gly Ser
            180                 185                 190

Glu Leu Leu Asp Val Gly Asp Glu Leu Glu Pro Gly Lys Ile Arg Asn
        195                 200                 205

Ser Asn Gly Pro Met Ile Glu Gly Leu Leu Asn Gln Phe Gly Leu Asp
    210                 215                 220

Gly Glu Val Tyr Lys Ile Gln Glu Asp Asp Leu Glu Ser Ser Ile Ala
225                 230                 235                 240

Val Val Lys Gln Ala Met Glu Thr His Asp Met Val Ile Thr Thr Gly
                245                 250                 255

Gly Val Ser Val Gly Asp Phe Asp Tyr Leu Pro Glu Ile Tyr Lys Ala
            260                 265                 270

Leu Asp Ala Glu Val Leu Phe Asn Lys Val Gln Met Arg Pro Gly Ser
        275                 280                 285

Val Thr Thr Val Ala Val Ala His Gly Lys Tyr Leu Phe Gly Leu Ser
    290                 295                 300

Gly Asn Pro Ser Ala Cys Tyr Thr Gly Phe Gln Leu Phe Val Lys Thr
305                 310                 315                 320

Gly Val Leu Asn Met Met Gly Ala Asn Glu Lys Phe Pro Gln Val Val
                325                 330                 335

Lys Ala Thr Leu Met Glu Asp Phe Lys Lys Ala Asn Pro Phe Thr Arg
            340                 345                 350

Phe Ile Arg Ser Lys Ala Thr Leu Asn Gly Met Glu Ala Thr Val Val
        355                 360                 365

Pro Ser Gly Phe Asn Lys Ser Gly Ala Val Val Ala Ile Ala His Ser
    370                 375                 380

Asn Ala Met Val Met Leu Pro Ser Gly Thr Arg Gly Phe His Ala Gly
385                 390                 395                 400

Asn Thr Val Asp Val Ile Leu Thr Glu Thr Asp Val Phe Glu Glu Glu
                405                 410                 415

Leu Leu Leu

(2) INFORMATION FOR SEQ ID NO: 16:
     (i) SEQUENCE CHARACTERISTICS:
          (A) LENGTH: 77 amino acids
          (B) TYPE: amino acid
          (D) TOPOLOGY: linear
    (ii) MOLECULE TYPE: protein
    (xi) SEQUENCE DESCRIPTION: SEQ ID NO: 16:

Met Lys Val Leu Tyr Phe Ala Glu Ile Lys Glu Ile Leu Gln Lys Asp
  1               5                  10                  15

Thr Asp Gln Phe Gln Ile Asp Asp Glu Met Thr Val Glu Ala Phe Lys
             20                  25                  30

Gln Tyr Leu Phe Glu Lys Tyr Pro Glu Ile Asp Gly Lys Lys Phe Gln
         35                  40                  45

Ile Ala Leu Asn Glu Glu Phe Val Gln Pro His Glu Lys Ile Asn Gln
     50                  55                  60

Ser Asp Val Val Ala Leu Ile Pro Pro Val Ser Gly Gly
 65                  70                  75

(2) INFORMATION FOR SEQ ID NO: 17:
     (i) SEQUENCE CHARACTERISTICS:
          (A) LENGTH: 196 amino acids
          (B) TYPE: amino acid
          (D) TOPOLOGY: linear
    (ii) MOLECULE TYPE: protein
    (xi) SEQUENCE DESCRIPTION: SEQ ID NO: 17:

Val Lys Ala Ile Ile Leu Ala Gly Gly Gln Ser Glu Arg Phe Gly Ala
  1               5                  10                  15

Pro Lys Ala Phe Ala Glu Ile Asp Gly Lys Met Phe Tyr Glu Gln Ile
            20                  25                  30

Ile Thr Val Leu Asp Ser Met Asn Met Phe Asn Glu Ile Ile Ile Ser
        35                  40                  45

Ser Asn Glu Thr Leu Ala Ser Glu Phe Lys Gly Ala Arg Val Ile Val
    50                  55                  60

Asp Asp Ser Glu His Lys Asn Lys Gly Pro Leu Ser Gly Ile Tyr Ser
65                  70                  75                  80

Val Met Lys Gln Asp Leu Asn Gln Asn Tyr Phe Cys Asp Phe Cys Gly
                85                  90                  95

Tyr Ala Ala Tyr Tyr Arg Lys Ala Ile Ser Gln Leu Tyr Gln Phe Met
            100                 105                 110

Val Glu His Val Ile Glu Asp Gln Leu Asp Ile Ala Gly Phe Lys Glu
        115                 120                 125

Gly Asn His Pro Ile Pro Thr Ile Ala Phe Tyr Ser Pro Asn Cys Leu
    130                 135                 140

Pro Ile Ile Ala Arg Ala Leu Glu Ser Asp Asp Tyr Ser Met Arg His
145                 150                 155                 160

Val Tyr Gln Gln Thr Ala Ser Asp Trp Ile Asp Val Ser Ser Val Asp
                165                 170                 175

Asp Asp Thr Glu Trp Tyr Lys Asn Ile Asn Tyr Pro Gln Asp Leu Glu
            180                 185                 190

Ser Ile Lys Lys
        195

(2) INFORMATION FOR SEQ ID NO: 18:
     (i) SEQUENCE CHARACTERISTICS:
          (A) LENGTH: 340 amino acids
          (B) TYPE: amino acid
          (D) TOPOLOGY: linear
     (ii) MOLECULE TYPE: protein
     (xi) SEQUENCE DESCRIPTION: SEQ ID NO: 18:

Met Val Lys Gln Ile Thr Asp Lys Leu Gly Arg Pro Ile Arg Asp Leu
1               5                   10                  15

Arg Leu Ser Val Thr Asp Arg Cys Asn Phe Arg Cys Asp Tyr Cys Met
            20                  25                  30

Pro Lys Glu Ile Phe Gly Asp Asp Phe Val Phe Leu Pro Lys Asp Glu
        35                  40                  45

Leu Leu Ser Phe Ser Glu Met Glu Arg Ile Ala Arg Val Tyr Thr His
    50                  55                  60

Leu Gly Val Lys Lys Ile Arg Ile Thr Gly Gly Glu Pro Leu Met Arg
65                  70                  75                  80

Arg Asp Leu Tyr Lys Leu Ile Ala Ala Leu Asn Glu Ile Glu Gly Val
                85                  90                  95

Glu Asp Ile Gly Leu Thr Thr Asn Gly Leu Leu Leu Lys Lys His Gly
            100                 105                 110

Gln Lys Leu Tyr Asp Ala Gly Leu Arg Arg Ile Asn Val Ser Leu Asp
        115                 120                 125

Ala Ile Asp Asn Glu Leu Phe Gln Ser Ile Asn Asn Arg Asn Ile Lys
    130                 135                 140

Ala Asp Thr Ile Leu Glu Gln Ile Asp Tyr Ala Val Ser Ile Gly Phe
145                 150                 155                 160

Lys Val Lys Ile Asn Val Val Val Gln Lys Gly Val Asn Asp Asp Gln
                165                 170                 175

Ile Ile Pro Met Val Gln Tyr Phe Lys Asp Lys Asn Ile Gln Val Arg
            180                 185                 190

Phe Ile Glu Phe Met Asp Val Gly Asn Asp Asn Gly Trp Asp Phe Ser
        195                 200                 205

Lys Val Val Ser Lys Asp Glu Met Leu Ser Met Ile Gln Glu Glu Phe
    210                 215                 220

Asp Ile Glu Ala Val Glu Pro Lys Tyr Tyr Gly Glu Val Ala Lys Tyr
225                 230                 235                 240

Tyr Arg His Lys Asp Asn Gly Ala Gln Phe Gly Leu Ile Thr Ser Val
                245                 250                 255

Ser Gln Ser Phe Cys Ser Thr Cys Thr Arg Ala Arg Leu Ser Ser Asp
            260                 265                 270

Gly Lys Phe Tyr Gly Cys Leu Phe Ser Thr Val Asp Gly Phe Asn Val
        275                 280                 285

Lys Glu Phe Met Arg Ser Gly Val Ser Asp Asp Glu Leu Gln Ala Lys
    290                 295                 300

Phe Glu Glu Leu Trp Asn Ile Arg Asp Asp Arg Tyr Ser Asp Glu Arg
305                 310                 315                 320

Thr Glu Gln Thr Val Ala Ile Arg Lys Arg Lys Lys Ile Asn Met Asn
                325                 330                 335

Tyr Ile Gly Gly
            340

(2) INFORMATION FOR SEQ ID NO: 19:
    (i) SEQUENCE CHARACTERISTICS:
        (A) LENGTH: 160 amino acids
        (B) TYPE: amino acid
        (C) STRANDEDNESS: single
        (D) TOPOLOGY: linear
    (ii) MOLECULE TYPE: protein
    (ix) FEATURE:
        (A) NAME/KEY: Protein
        (B) LOCATION:1..160
        (D) OTHER INFORMATION:/note= "MobB protein encoded by
            nucleotide 3652-4131 of SEQ ID NO:11"

(xi) SEQUENCE DESCRIPTION: SEQ ID NO: 19:

Met Ile Leu Gln Ile Val Gly Tyr Lys Asp Ser Gly Lys Thr Thr Leu
1               5                   10                  15

Leu Ala Glu Thr Val Lys Phe Leu Lys Asn Lys Gly Tyr Arg Val Val
            20                  25                  30

Thr Ile Lys His His Gly His Gly Ala Glu Asp Ile Thr Leu Gln Glu
        35                  40                  45

Asn Thr Val Asp His Met Lys His Phe Glu Ala Gly Ala Asp Gln Ser
    50                  55                  60

Ile Val Gln Gly His Glu Leu Arg Glu Thr Ile Thr Arg Thr Ser Glu
65                  70                  75                  80

Ala Ser Leu Ser Gln Ile Ile Asp Ser Ala Val Thr Ile Glu Tyr Asp
                85                  90                  95

Ile Ile Leu Ala Glu Gly Phe Lys Asn Ala Asp Tyr Asp Lys Val Val
            100                 105                 110

Ile Tyr Lys Asn Pro Glu Glu Leu Ser Ser Leu Ser Ala Leu Ser His
        115                 120                 125

Val Gln Tyr Lys Leu Pro Phe Gln Lys Leu Ser Asp Leu Glu Pro Phe
    130                 135                 140

Lys Asn Trp Leu Glu Ser Trp Ile Asp Ile Lys Lys Asp Glu Ser Glu
145                 150                 155                 160

(2) INFORMATION FOR SEQ ID NO: 20:
    (i) SEQUENCE CHARACTERISTICS:
        (A) LENGTH: 150 amino acids
        (B) TYPE: amino acid
        (C) STRANDEDNESS: single
        (D) TOPOLOGY: linear
    (ii) MOLECULE TYPE: protein
    (ix) FEATURE:
        (A) NAME/KEY: Protein
        (B) LOCATION:1..150
        (D) OTHER INFORMATION:/note= "MoaE protein encoded by
            nucleotide 4131-4580 of SEQ ID NO:11"
    (xi) SEQUENCE DESCRIPTION: SEQ ID NO: 20:

Met Lys Gln Phe Glu Ile Val Thr Glu Pro Ile Gln Thr Glu Gln His
1               5                   10                  15

Arg Asp Phe Thr Leu Asn Pro His Gln Gly Ala Val Val Val Phe Thr
            20                  25                  30

Gly His Val Arg Glu Trp Thr Lys Gly Ile Arg Thr Glu His Leu Glu
        35                  40                  45

Tyr Glu Ala Tyr Ile Pro Met Ala Glu Lys Lys Leu Ala Gln Ile Gly
    50                  55                  60

Asp Glu Ile Asn Glu Gln Trp Pro Gly Thr Ile Val Ser Ile Val His
65                  70                  75                  80

Arg Ile Gly Pro Leu Lys Ile Ser Asp Ile Ala Val Leu Ile Ala Val
                85                  90                  95

Ser Ser Pro His Arg Lys Asp Ala Tyr Ala Ala Asn Glu Tyr Ala Ile
            100                 105                 110

Asp Arg Ile Lys Glu Val Val Pro Ile Trp Lys Lys Glu Ile Trp Glu
        115                 120                 125

Asp Gly Ala Glu Trp Ile Gly His Gln Arg Gly Tyr His Asp Asp Ala
    130                 135                 140

Val Glu Arg Gly Gln Asn
145                 150